# EUROPEAN PATENT SPECIFICATION

(11) **EP 3 404 410 B1**
(45) Date of publication and mention of the grant of the patent: **14.07.2021**
(21) Application number: 18180720.7
(22) Date of filing: 31.03.2016
(51) Int. Cl.: G01N 33/554

(54) **BIOSENSOR SYSTEM FOR THE RAPID DETECTION OF ANALYTES**
BIOSENSORSYSTEM ZUR SCHNELLEN DETEKTION VON ANALYTEN
SYSTÈME DE BIOCAPTEUR POUR LA DÉTECTION RAPIDE D'ANALYTES

(30) Priority: 31.03.2015 US 201562140920 P
(43) Date of publication of application: 21.11.2018
(62) Divisional of application: 16774179.2
(73) Proprietor: Fundamental Solutions Corporation, Easton, PA 18042 (US)
(72) Inventor: ZENG, Lingchun, Upper Arlington, OH 43221 (US); ZUPANCIC, Thomas, J., Powell, OH 43065 (US); VEDAMOORTHY, Srikanth, New Albany, OH 43054 (US); LWANDE, Joel, S., Athens, OH 45701 (US); KITTLE, Joseph, D., The Plains, OH 45780 (US); MO, Min, Dublin, OH 43107 (US)
(74) Representative: Samson & Partner Patentanwälte mbB

(56) References cited:
- US-A1- 2012 225 423
- US-A1- 2014 273 020

## Description

### BACKGROUND OF THE INVENTION

The described invention relates in general to systems, devices, and methods for detecting various analytes in biological samples or other sample types, and more specifically to a biosensor-based system for detecting and identifying analytes of interest in real time based on the emission of a detectable signal when the biosensor reacts with an analyte of interest in a sample being tested.

In generic terms, a biosensor is a system or device for the detection of an analyte that combines a sensitive biological component with a physicochemical detector component. The components of a typical biosensor system include a biological element, a transducer or detector element, and associated electronics or signal processors that display test results in a meaningful and useful manner. The biological element typically includes biological material such as tissue, microorganisms, organelles, cell receptors, enzymes, antibodies, nucleic acids, and the like that may be created by known biological engineering processes. The transducer or detector element works in a physicochemical manner (e.g., optical, piezoelectric, and/or electrochemical) that transforms the signal resulting from the interaction of the analyte with the biological element into another signal that can be more easily measured and quantified. Biosensors originated from the integration of molecular biology and information technology (e.g., microcircuits, optical fibers, etc.) to qualify or quantify biomolecule-analyte interactions such as antibody-antigen interactions. Considering that there is great demand for rapid, sensitive, easy-to-handle, and cost-effective detection tools for detecting infectious agents, pathogens or/and toxins in food (see, for example, Mead et al., Food Related Illness and Death in the United States, Emerging Infectious Diseases; Vol. 5, No. 5, September-October 1999 (607-625),), there is an ongoing need for the utilization of biosensors in real-time, field-portable devices and instruments for the detection and identification of infectious agents, pathogenic microorganisms, toxins, and other contaminants in foods and many other items.

US 2014/273020 A1 discloses a system for detecting infectious agents in biological sample based on a biosensor hybridoma cell that naturally expresses an endogenous anti-target antigen specific IgM and a detectable reporter gene introduced into the cell.

US 2012/225423 (A1) discloses a method for the detection of soluble analytes, such as pathogens, soluble antigens, nucleic acids, toxins, chemicals, plant pathogens, blood borne pathogens, bacteria, viruses and the like. The method is based on a sensor cell which detects target analytes bound to an antibody via binding of the antibody to Fc receptor of the sensor cell.

### SUMMARY OF THE INVENTION

The following provides a summary of certain exemplary embodiments of the present invention. This summary is not an extensive overview.

In accordance with one aspect of the present invention, a first system for the rapid detection of analytes is provided. An exemplary embodiment of this first system includes a living, engineered biosensor cell that is derived from a cellular component of the mammalian immune system; a reporter protein that is engineered into and produced by the living, engineered biosensor cell and that emits a detectable signal in response to certain predetermined changes in the cytosol of the living, engineered biosensor cell; a signal transduction pathway engineered into or occurring naturally within the living, engineered biosensor cell that controls a biological process within the cytosol of the living, engineered biosensor cell, wherein the biological process, when it occurs, causes the reporter protein to emit a detectable signal; at least one type of detector molecule, wherein each detector molecule is adapted to bind to a specific analyte; at least one analyte that binds to the detector molecule and that is specific to that analyte; a plurality of transmembrane, non-antibody signal transducing elements expressed by the living, engineered biosensor cell, wherein each signal transducing element is adapted to receive a detector molecule; and wherein upon the binding of a sufficient number of analytes to a sufficient number of detector molecules that are themselves bound to transmembrane non-antibody signal transducing elements, an aggregation of signal transducing elements occurs on the biosensor cell surface, the signal transduction pathway is activated, the biological process occurs, and the detectable signal is emitted by the reporter protein.

In accordance with another aspect of the present invention, a second system for the rapid detection of analytes is provided. An exemplary embodiment of this second system includes a living, engineered biosensor cell, wherein the living, engineered biosensor cell is derived from a cellular component of the mammalian immune system, and wherein the living, engineered biosensor cell expresses a plurality of at least one predetermined type of receptor molecule on the surface thereof; a reporter protein, wherein the reporter protein is engineered into and expressed by the living, engineered biosensor cell, and wherein the reporter protein emits a detectable signal in response to certain predetermined changes in the cytosol of the living, engineered cell; a signal transduction pathway engineered into or occurring naturally within the living, engineered biosensor cell, wherein the signal transduction pathway controls a biological process within the cytosol of the living, engineered biosensor cell, and wherein the biological process, when it occurs, causes the reporter protein to emit a detectable signal; at least one type of detector molecule, wherein each detector molecule is adapted to bind to a specific analyte; at least one analyte, wherein the at least one analyte binds to the detector molecule that is specific to that analyte; a plurality of soluble non-antibody signal transducing elements, wherein each soluble signal transducing element is adapted to bind to a receptor molecule and to a detector molecule; and wherein upon the binding of a sufficient number of analytes to a sufficient number of detector molecules to a sufficient number of non-antibody signal transducing elements that are themselves bound to the cell surface receptor molecules, an aggregation of the receptor molecules occurs, the signal transduction pathway is activated, the biological process occurs, and the detectable signal is emitted by the reporter protein.

In accordance with still another aspect of the present invention, a biosensor for the rapid detection of analytes in a sample is provided. This biosensor includes a living, engineered cell, wherein the living, engineered cell is derived from a cellular component of the mammalian immune system (i.e., an immunocyte); a reporter protein, wherein the reporter protein is engineered into and expressed by the living, engineered cell, and wherein the reporter protein emits a detectable signal in response to certain predetermined changes in the cytosol of the living, engineered cell; a signal transduction pathway engineered into or occurring naturally within the living, engineered cell, wherein the signal transduction pathway controls a biological process within the cytosol of the living, engineered biosensor cell, and wherein the biological process, when it occurs, causes the reporter protein to emit a detectable signal; and a plurality of non-antibody signal transducing elements that directly or indirectly bind to an analyte in a sample to be analyzed, wherein the bound non-antibody signal transducing elements then cooperate with the biosensor cell to directly or indirectly activate the signal transduction pathway.

Additional features and aspects of the present invention will become apparent to those of ordinary skill in the art upon reading and understanding the following detailed description of the exemplary embodiments. As will be appreciated by the skilled artisan, further embodiments of the invention are possible. Accordingly, the drawings and associated descriptions are to be regarded as illustrative.

### BRIEF DESCRIPTION OF THE DRAWINGS

The accompanying drawings, which are incorporated into and form a part of the specification, schematically illustrate one or more exemplary embodiments of the invention and, together with the general description given above and detailed description given below, serve to explain the principles of the invention, and wherein:

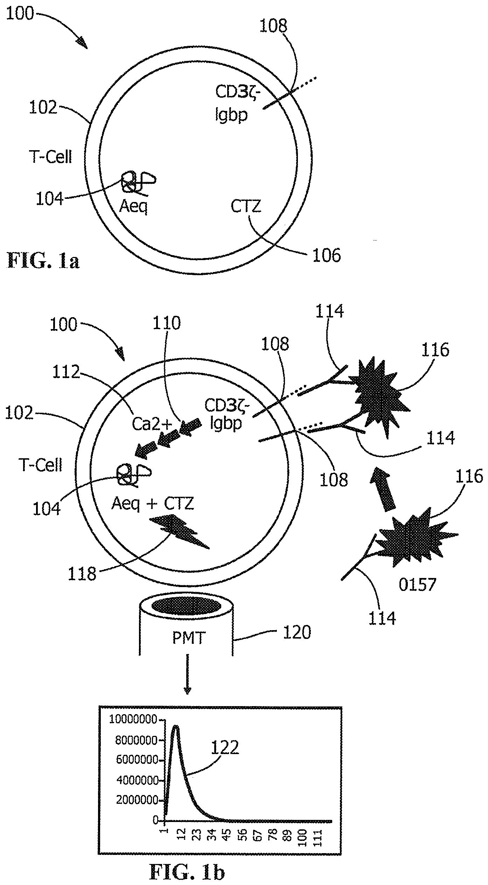

FIGS. 1a-b are illustrations of a first biosensor in accordance with an exemplary embodiment of the present invention, wherein Jurkat T cells have been engineered to produce aequorin and to express the transmembrane non-antibody signal transducing element IgGbp-CD3ζ;

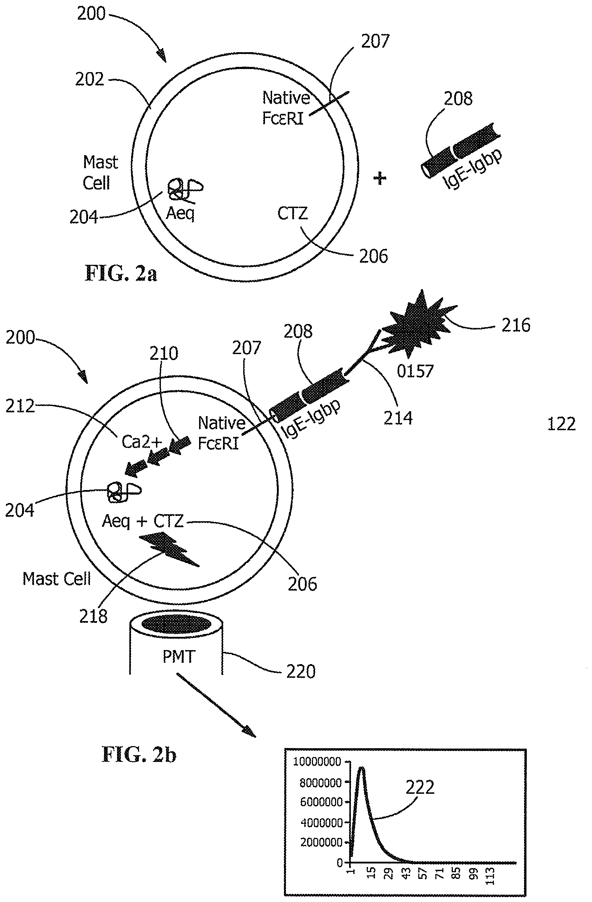

FIGS. 2a-b are illustrations of a second biosensor in accordance with an exemplary embodiment of the present invention, wherein MC/9 mast cells have been engineered to produce aequorin, and wherein the MC/9 cells express the native receptor FcεRI, which binds to the soluble non-antibody signal transducing element IgGbp-IgE;

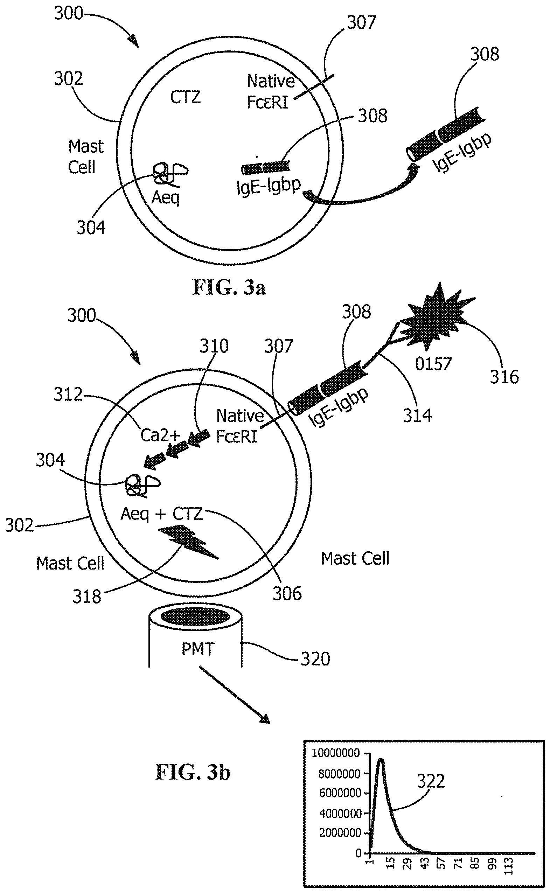

FIGS. 3a-b are illustrations of a third biosensor in accordance with an exemplary embodiment of the present invention, wherein MC/9 mast cells have been engineered to produce soluble non-antibody signal transducing element IgGbp-IgE, which has been excreted by the MC/9 mast cells;

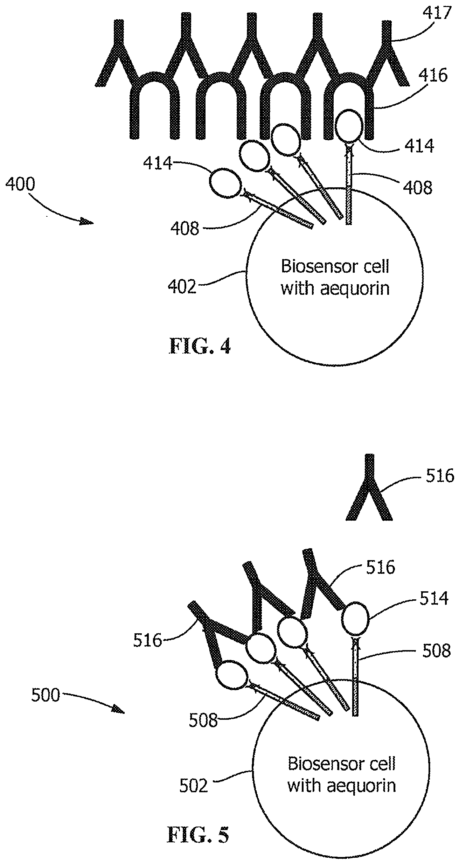

FIGS. 4 is an illustration of a fourth biosensor in accordance with an exemplary embodiment of the present invention, wherein biosensor cells have been engineered to produce aequorin and to express the transmembrane non-antibody signal transducing element mSA-CD3ζ, which binds to a biotinylated detector element; and
FIGS. 5 is an illustration of a fifth biosensor in accordance with an exemplary embodiment of the present invention, wherein biosensor cells have been engineered to produce aequorin and to express the transmembrane non-antibody signal transducing element mSA-OP3ζ, which binds to a biotinylated detector element.

### DETAILED DESCRIPTION OF THE INVENTION

Exemplary embodiments of the present invention are now described with reference to the Figures. Although the following detailed description contains many specifics for purposes of illustration, a person of ordinary skill in the art will appreciate that many variations and alterations to the following details are within the scope of the invention. Accordingly, the following embodiments of the invention are set forth without any loss of generality to, and without imposing limitations upon, the claimed invention.

The present invention relates in general to systems, devices, and methods for detecting various analytes in biological samples or other sample types, and more specifically to a biosensor-based system for detecting and identifying analytes of interest in real time based on the emission of a detectable signal when the biosensor reacts with an analyte of interest in a sample being tested. The engineered cells of the present invention are extremely sensitive and effective biosensors and because these biosensor cells have an intrinsic detection capacity, they provide a versatile system that can be readily adapted to detect a wide variety of different infectious agents or other targets by simply selecting alternative soluble detector (e.g., antibody) molecules with specificity for a particular pathogen or other target of interest. Furthermore, the system of this invention can be readily configured for multiplex detection of several infectious agents or other analytes in a single assay, providing for great flexibility and utility. The versatility of the present invention is derived from a unique combination of elements and in particular from the combination of a universal biosensor cell with a specific soluble detector (e.g., antibody). The universal biosensor cell has the capacity to respond to the presence of essentially any target molecule that can be recognized by the detector molecule. Because, in some embodiments, the detector or detector antibody is added to the system as a soluble factor, the system may be configured to detect an alternative target by simply selecting an appropriate alternate detector or detector antibody. The specificity of the disclosed system is determined by the detector molecule, which is selected based on its specificity and affinity for a target molecule that is characteristic of an infectious agent or other target analyte. The combination of this universal biosensor cell and soluble detector also enables the construction of multiplex assays by simply including a plurality of detector molecules (e.g., antibodies) within the test system, wherein the target molecules are selected based on their specificity for alternative infectious agents or other analytes.

Genetic manipulation and modification of the biosensor cell types used with this invention typically involve the use of appropriately selected gene delivery vehicles that contain genetic elements that function efficiently in the cell type of choice. For example, it is useful to employ a promoter element that directs high level expression of introduced transgenes in the specific biosensor cell of choice. In an exemplary embodiment of this invention, such a promoter element may be derived directly from the biosensor cell itself and then used to express a transgene of interest. In another embodiment of this invention, an appropriate element may be determined empirically by comparing the function of alternative promoter elements in the context of alternative gene delivery vehicles in order to identify effective promoter, transgene, vector combinations for the cell type of choice. Transgenes such as the gene encoding a luminescent reporter protein may be introduced into the biosensor cell using standard techniques such as electroporation or chemical transfection reagents such as, for example, lipofectamine. Other genetic engineering methods known to those of ordinary skill in the art are also compatible with the present invention.

In a generic embodiment, the present invention provides a system and method for rapid detection of analytes that includes the following components: (i) a living, engineered biosensor cell, wherein the living engineered biosensor cell is a component of the mammalian immune system; (ii) a reporter protein, wherein the reporter protein is expressed by the living, engineered biosensor cell, and wherein the reporter protein emits a detectable signal in response to certain predetermined changes in the cytosol of the living, engineered cell; (iii) a signal transduction pathway expressed by the living, engineered biosensor cell, wherein the signal transduction pathway controls a biological or biochemical process within the cytosol of the living, engineered biosensor cell, and wherein the biological or biochemical process, when it occurs, causes the reporter protein to emit a detectable signal; (iv) at least one type of detector molecule, wherein each detector molecule is adapted to bind to a specific analyte; (v) at least one analyte, wherein the at least one analyte binds to the detector molecule that is specific to that analyte; (vi) a plurality of non-antibody signal transducing elements that are either expressed by the living, engineered biosensor cell or that actively bind to a receptor or a receptor component expressed by the living, engineered biosensor cell, wherein each signal transducing element is adapted to receive a detector molecule. Upon the binding of a sufficient number of analytes to a sufficient number of detector molecules that are themselves bound to the non-antibody signal transducing elements, an aggregation of signal transducing elements occurs on the cell surface, the signal transduction pathway is activated, the biochemical process occurs, and the detectable signal is emitted by the reporter protein. This system may also include a device for mixing the living cells together with soluble components and a sample containing an analyte or infectious agent of interest while maintaining the viability and functionality of the living biosensor cell, and a detector for detecting the signal emitted by the biosensor cell.

### Living, Engineered Biosensor Cell

Exemplary embodiments of this invention include a living, engineered biosensor cell that is typically a component of the mammalian immune system, e.g., an immunocyte. In certain embodiments of this invention, the biosensor cell is a human or mouse B cell. B cells or B lymphocytes, are a type of white blood cell of the lymphocyte subtype that function in the humoral immunity component of the adaptive immune system by secreting antibodies. In other embodiments of this invention, the biosensor cell is a human or mouse T cell. T cells or T lymphocytes are another type of lymphocyte that play a central role in cell-mediated immunity as part of the adaptive immune system. T cells are distinguishable from other lymphocytes due to the presence of a T-cell receptor on the cell surface. In other embodiments of this invention, the biosensor cell is a mast cell. A mast cell is also a type of white blood cell known as a granulocyte that is derived from the myeloid stem cell that is a part of the immune and neuroimmune systems. Other types of cells are compatible with this invention, including basophils, which are another type of white blood cell, and which are similar in both appearance and function to mast cells.

### Reporter Protein

Exemplary embodiments of this invention include a reporter element, such as a reporter protein or enzyme that is produced or expressed by the living, engineered biosensor cell. The reporter protein emits a detectable signal in response to certain predetermined changes in the cytosol of the living, engineered biosensor cell. In certain embodiments of this invention, the reporter protein is a bioluminescent photoprotein such as aequorin, which is derived from the hydrozoan *Aequorea Victoria.* Aequorin has been previously used for engineering living biosensor cells to produce light signals in response to activation of a wide variety of signal transduction pathways; thus, various methods for manipulating the production of aequorin in living cells are well known to the skilled artisan. In particular, a skilled artisan may selected and employ any appropriate gene delivery vehicle such as, for example, bacterial plasmid vectors or viral vectors, for introducing the appropriate genetic material into the biosensor cells. Production of the reporter protein within the biosensor cell will then be controlled by expression of the introduced genetic material. One having ordinary skill in the art will also appreciate that other photoproteins or other types of reporter proteins, enzymes, and molecules may be incorporated into and utilized with various alternate embodiments of the present invention.

### Signal Transduction Pathway

Exemplary embodiments of this invention include a signal transduction pathway expressed by the living, engineered biosensor cell. The signal transduction pathway controls at least one biological process within the cytosol of the living, engineered cell, and the at least one biological process, when it occurs, causes the reporter protein to emit a detectable signal. In certain embodiments of this invention, the signal transduction pathway is any biochemical pathway in which an increase in intracellular Ca2+ concentration is induced in response to activation of a cell surface signal transducing molecule, such as a receptor protein. The biosensor cells used with this invention may be selected from a set of living cells that are capable of producing an increase in cytoplasmic Ca2+ in response to activation of a cell surface signal transduction molecule. For example, B cells, T cells, and mast cells have the capacity to induce an increase in Ca2+ concentration in response to activation of cell surface signal transducing molecules such as the B cell receptor, the T cell receptor, and the Fc epsilon receptor (mast cells), respectively.

Because mammalian cells growing in culture typically generate populations of cells in which specific individual cells may have differing capacities to induce an increase in Ca2+ concentration, it is useful to select for or screen for subpopulations of cells or clonal cell lines that have a robust ability to generate the Ca2+ signal. This may be accomplished by analyzing induction of an aequorin induced flash, for example. In particular, the transfectants created by the introduction of transgenes into a cell are a mixed population of cells derived from a large number of independent gene insertion events. Thus, when constructing a biosensor cell it is useful to screen or select specific subsets of cells or clonal cell lines that have efficient signal transduction capabilities together with useful levels of expression of introduced transgenes. It is particularly useful to use fluorescence-activated cell sorting (FACS) technology to select for subpopulations of high expressing cells or to generate clonal cell lines for this purpose.

As previously stated, aequorin has been used previously for engineering living biosensor cells to produce light signals in response to activation of a wide variety of signal transduction pathways, particularly wherein such signal transduction pathways lead to an increase in cytoplasmic Ca2+ ions within a living cell. In certain embodiments of this invention, biosensor cells that produce aequorin as the reporter protein are charged with coelenterazine (CTZ) prior to their use in a detection assay. This charging step covalently links the aequorin to a hydrophobic prosthetic group (e.g., CTZ) and upon calcium (Ca2+) binding, the CTZ undergoes an irreversible reaction that includes a conformation change, and emits blue light (at 469 nm).

### Detector Molecule

Exemplary embodiments of this invention include at least one type of detector element such as a detector molecule, wherein each detector molecule is adapted to bind to a specific target analyte. The detector molecule may a soluble antibody that is not in any way expressed by the biosensor cells. The particular detector molecule used with the present invention is selected based on its ability to unambiguously identify the target analyte of interest. In an exemplary embodiment, the detector molecule is a soluble antibody such as a commercially available IgG that is specific for a particular analyte, such as an infectious agent. In another exemplary embodiment, the detector molecule is a biotinylated molecule (or streptavidin-based molecule) that is specific for a predetermined analyte such as, for example, a biotinylated autoantigen molecule that is specific for an anti-autoantigen antibody. A detector or target molecule according to this invention may include an autoantigen or an autoantibody associated with an autoimmune disease. Representative autoimmune diseases or disorders include rheumatoid arthritis (RA), juvenile RA (JRA), diabetes mellitus type 1, systemic lupus erythematosus, Hashimoto's thyroiditis, Graves' disease, scleroderma, celiac disease, Crohn's disease, ulcerative colitis, Sjogren's syndrome, multiple sclerosis, Goodpasture's syndrome, Addison's disease, Wegener's granulomatosis, primary biliary cirrhosis, sclerosing cholangitis, autoimmune hepatitis, polymyalgia rheumatica, temporal arteritis/giant cell arteritis, and Guillain-Barre syndrome. Detector or target molecules may also comprise tumor-specific or tumor-associated antigens or antibodies to such antigens; or biologically active molecules, such as EGF, peptide hormones, including insulin and growth hormone, cytokines, interleukins, interferons, TNF, *etc.* or antibodies to such biologically active molecules.

### Analyte and Test Sample

An intended use of the present invention is the detection of various analytes that are or might be present within samples to be tested. In an exemplary embodiment of this invention, an analyte that is to be detected will bind to a detector molecule, such as a soluble antibody, that is specific to that analyte. A sample to be tested may be taken from a large number of food sources, including: (i) meats such as beef, pork, lamb, bison, poultry, and seafood; and (ii) plants and vegetables. A sample to be tested may also be taken from many other sources such as water, consumable fluids, preservative fluids, and bodily fluids such as blood. Analytes that may be detected include virtually anything that will bind with specificity to the detector or detector molecule such as chemicals, toxins, and infectious agents such as viruses, bacteria, and other biological materials or agents. In an exemplary embodiment of this invention, the specific infectious agent is *Escherichia coli,* although other infectious agents (such as Salmonella, Listeria, and Campylobacter) and contaminants may be detected with the present invention. *Escherichia coli O157 H7, 026, 045, 0103, O111, 0121,* and *0145,* in either separate assays or multiplexed assays, may all potentially be detected using this invention.

The present invention is capable of detecting many different analytes including meat pathogens, and those found on spinach, lettuce, and other vegetables and foods. An analyte may contain one or more epitopes of an antigen or allergen, including both linear or conformation epitopes; it may also contain one or more ligands or receptors recognized by reciprocal receptors or ligands. Exemplary analytes include a bacterium, such as Bacillus (e.g., B. anthracis), Enterobacteriaceae (e.g., Salmonella, Escherichia coli, Yersinia pestis, Klebsiella, and Shigella), Yersinia (e.g., Y. pestis or Y. enterocolitica), Staphylococcus (e.g., S. aureus), Streptococcus, Gonorrheae, Enterococcus (e.g., E. faecalis), Listeria (e.g., L. monocytogenes), Brucella (e.g., B. abortus, B. melitensis, or B. suis), Vibrio (e.g., V. cholerae), Corynebacterium diphtheria, Pseudomonas (e.g., P. pseudomallei or P. aeruginosa), Burkholderia (e.g., B. mallei or B. pseudomallei), Shigella (e.g., S. dysenteriae), Rickettsia (e.g., R. rickettsii, R. prowazekii, or R. typhi), Francisella tularensis, Chlamydia psittaci, Coxiella burnetii, Mycoplasma (e.g., M. mycoides), etc.; allergens, such as peanut dust, mycotoxins, mold spores, or bacterial spores such as Clostridium botulinum and C. perfringens; toxins, such as ricin, mycotoxin, tetrodotoxin, anthrax toxin, botulinum toxin, staphylococcal entertoxin B, or saxitoxin; a virus, such as Adenoviridae (e.g., adenovirus), Arenaviridae (e.g., Machupo virus), Bunyaviridae (e.g., Hantavirus or Rift Valley fever virus), Coronaviridae, Orthomyxoviridae (e.g., influenza viruses), Filoviridae (e.g., Ebola virus and Marburg virus), Flaviviridae (e.g., Japanese encephalitis virus and Yellow fever virus), Hepadnaviridae (e.g., hepatitis B virus), Herpesviridae (e.g., herpes simplex viruses), Papovaviridae (e.g., papilloma viruses), Paramyxoviridae (e.g., respiratory syncytial virus, measles virus, mumps virus, or parainfluenza virus), Parvoviridae, Picornaviridae (e.g., polioviruses), Poxviridae (e.g., variola viruses), Reoviridae (e.g., rotaviruses), Retroviridae (e.g., human T cell lymphotropic viruses (HTLV) and human immunodeficiency viruses (HIV)), Rhabdoviridae (e.g., rabies virus), and Togaviridae (e.g., encephalitis viruses, yellow fever virus, and rubella virus)); a protozoon, such as Cryptosporidium parvum, Encephalitozoa, Plasmodium, Toxoplasma gondii, Acanthamoeba, Entamoeba histolytica, Giardia lamblia, Trichomonas vaginalis, Leishmania, or Trypanosoma (e.g., T. brucei and T. Cruzi); a helminth, such as cestodes (tapeworms), trematodes (flukes), or nematodes (roundworms, e.g., Ascaris lumbricoides, Trichuris trichiura, Necator americanus, or Ancylostoma duodenale); a parasite (e.g., any protozoa or helminths described herein); a fungus, such as Aspergilli, Candidae, Coccidioides immitis, and Cryptococci; an environmental contaminant; a water additive; an agricultural marker; a nucleic acid (e.g., oligonucleotides, polynucleotides, nucleotides, nucleosides, molecules of DNA, or molecules of RNA, including a chromosome, a plasmid, a viral genome, a primer, or a gene); a protein (e.g., a glycoprotein, a metalloprotein, an enzyme, a prion, or an immunoglobulin); a metabolite; a sugar; a lipid; a lipopolysaccharide; a salt; or an ion. Targets also include food-borne pathogens, such as Salmonella (e.g., Salmonella Typhimurium), pathogenic E. coli (e.g., O157:H7), Bacillus (e.g., B. cereus), Clostridium botulinum, Listeria monocytogenes, Yersinia (e.g., Y. enterocolitica), Norovirus (e.g., Norwalk virus), Shigella, Staphylococcus aureus, Toxoplasma gondii, Vibrio (e.g., V. vulnificus, V. cholera, V. parahaemolyticus), Campylobacter jejuni, and Clostridium perfringens; and weaponized pathogens, such as Bacillus anthracis, Yersinia pestis, Francisella tularensis, Brucella (e.g., B. suis), Burkholderia mallei, Burkholderia pseudomallei, Shigella, Clostridium botulinum, Variola (e.g., V. major), Filoviridae (e.g., Ebola virus and Marburg virus), Arenaviridae (e.g., Lassa virus and Machupo virus), Clostridium perfringens, any food-borne pathogen (e.g., Salmonella species, Escherichia coli O157:H7, or Shigella), Chlamydia psittaci, Coxiella burnetii, Staphylococcal aureus, Rickettsia (e.g., R. prowazekii or R. rickettsii), Alphavirus (e.g., Venezuelan equine encephalitis virus, eastern equine encephalitis virus, or western equine encephalitis virus), Vibrio cholerae, Cryptosporidium parvum, Henipavirus (e.g., Nipah virus), Bunyaviridae (e.g., Hantavirus or Rift Valley fever virus), Flaviviridae (e.g., Japanese encephalitis virus and Yellow fever virus), and Coccidioides spp.

Epitopes that can be detected as analytes or portions of an analyte are typically antigenic determinant sites on an antigen to which an immunogolublin (or antigen binding fragment thereof) can specifically bind. Epitopes can be formed both from contiguous amino acids or noncontiguous amino acids juxtaposed by tertiary folding of a protein. Epitopes can be found on the Fab (variable) region of immunoglobulins (referred to as "idiotypic determinants") and comprise the immunoglobulin's "idiotype". The epitope and antigen can be naturally occurring or artificially produced. Depending on the nature of the epitope or antigen, the epitope or antigen can be isolated or purified from a matrix or substance of origin, synthesized, or recombinantly produced, for example. Epitopes and antigens useful as analytes can be from a human or non-human animal, plant, bacteria, protozoan, parasite, virus, etc. In some embodiments, the analyte is a polypeptide, nucleic acid molecule, carbohydrate, glycoprotein, lipid, lipoprotein, glycolipid, or small molecule. In some embodiments, the analyte is selected from among a cancer antigen, autoantigen, allergen, endogenous antigen, infectious agent antigen, drug (small molecule) antigen, toxin, venom, biologic antigen, environmental antigen, transplant antigen, and implant antigen.

An analyte may comprise an epitope of a cancer antigen. In some embodiments, the analyte is a tumor-associated antigen. In some embodiments, the analyte is a tumor-specific antigen. In some embodiments of the invention, the analyte is a tumor-associated antigen (TAA), and the TAA is a carbohydrate antigen having one or more post-translational modifications that differ from the wild-type protein, comprises a fusion region of a protein resulting from a gene fusion that is present in malignant cells but not present in non-malignant cells, and/or wherein the TAA comprises a receptor tyrosine kinase (RTK) that is deregulated and/or dysfunctional in tumor cells due to autocrine activation, chromosomal translocations, RTK overexpression, or gain-of-function mutations in the RTK gene or protein. In some embodiments of the invention, the analyte is an immunoglobulin expressed by a B-cell malignancy. Examples of B-cell malignancies include, but are not limited to, non-Hodgkin's lymphoma, Hodgkin's lymphoma, chronic lymphocytic leukemia, mantle cell lymphoma and multiple myeloma. Additional B-cell malignancies include, for example. B-cell prolymphocytic leukemia, lymphoplasmocytic leukemia, splenic marginal zone lymphoma, marginal zone lymphoma (extra-nodal and nodal), plasma cell neoplasms (e.g., plasma cell myeloma, plasmacytoma, monoclonal immunoglobulin deposition diseases, heavy chain diseases), and follicular lymphoma (e.g., Grades I, II, III, or IV).

In some embodiments, the analyte is a tumor-associated antigen derived from tumor cells obtained from the subject. In some embodiments, the tumor-associated antigen is one or more antigens selected from among 17-1A, 707-AP, AFP, Annexin II, ART-4, BAGE, BAGE-1, .beta.-catenin, BCG, bcr/abl, Bcr/abl e14a2 fusion junction, bcr-abl (b3a2), bcr-abl (b3a2), bcr-abl p190 (ela2), bcr-abl p210 (b2a2), bcr-abl p210 (b3a2), bcr-abl p210 (b3a2), bullous pemphigoid antigen-1, CA19-9, CA125, CA215, CAG-3, CAMEL, Cancer-testis antigen, Caspase-8, CCL3, CCL4, CD16, CD20, CD3, CD30, CD55, CD63, CDC27, CDK-4, CDR3, CEA, cluster 5, cluster-5A, cyclin-dependent kinase-4, Cyp-B, DAM-10, DAM-6, Dekcain, E7, EGFR, EGFRvIII, EGP40, ELF2 M, EpCAM, FucGM1, G250, GA733, GAGE, GAGE-1-8, gastrin cancer associated antigen, GD2, GD3, globoH, glycophorin, GM1, GM2, GM3, GnTV, Gn-T-V, gp100, Her-2/neu, HERV-K-ME, high molecular weight-associated antigen, high molecular weight proteo-glycan (HMPG), HPV-16 E6, HPV-16 E7, HPVE6, HSP70-2M, HST-2, hTERT, human chorionic gonadotropin (HCG), Human milk fat globule (HMFG), iCE, KIAA0205, KK-LC-1, KM-HN-1, L6, LAGE-1, Lcose4Cer, LDLR/FUT, Lewis A, Lewis v/b, M protein, MAGE-1, MVC, MAGE-A1-12, MAGE-C2, MAHGE-3, MART-1/Melan-A, MC1R, ME491, MUC1, MUC2, mucin, MUM-1, MUM-2, MUM-3, mutated p53, Myosin, MZ2-E, N9 neuraminidase, NA88, NA88-A, nasopharyngeal carcinoma antigen, NGA, NK1/c-3, Novel bcr/ablk fusion BCR exons 1, 13, 14 with ABL exons 4, NY-ESO-1/LAGE-2, NY-ESO-1b, OC125, osteosarcoma associated antigen-1, P15, p190 mimor bcr-abl (ela2), p53, Pm1/RARa, Polysialic acid, PRAME, PSA, PSM, RU1, RU2, SAGE, SART-1, SART-2, SART-3, Sialyl LeA, Sp17, SSX-2, SSX-4, surface immunoglobulin, TAG-1, TAG-2, TEL/AML1, TPI, TRAG-3, TRP-1 (gp75), TRP-2, TRP2-INT2, hTRT, tumor associated glycoprotein-72 (TAG-72), tyrosinase, u-PA, WT1, and XAGE-1b, or an immunogenic fragment of any of the foregoing antigens. In some embodiments, the tumor associated antigen is identified by the SEREX (serological analysis of recombinant cDNA expression library) approach or based on the serological screening of cDNA expression library generated from tumor tissues of various origin or cancer cell lines, and identifying immunogenic tumor proteins based on their reactivity with autologous patient sera. In some embodiments, the analyte is a tumor-associated antigen that is acarbohydrate antigen having one or more post-translational modifications that differ from the wild-type protein. In some embodiments, the tumor-associated antigen comprises a fusion region of a protein resulting from a gene fusion that is resent in malignant cells but not present in non-malignant cells. In some embodiments, the tumor-associated antigen comprises a receptor tyrosine kinase that is deregulated and/or dysfunctional in tumor cells due to autocrine activation, chromosomal translocations, RTK overexpression, or gain-of-function mutations in the RTK gene or protein.

The analyte may comprise an epitope of an antigen of an infectious or noninfectious agent that can be either pathogenic or non-pathogenic to the subject. The analyte can be derived from a mutualistic, parasitic, or commensal microorganism, including any microorganism in a animal or plant biome, such as probiotic or commensal microorganisms in the human digestive tract, mucosal surfaces, or epithelium. In some embodiments, the bacterial pathogen is selected from among Acinetobacter baumannii (formerly Acinetobacter calcoaceticus), Actinobacillus, Actinomyces pyogenes (formerly Corynebacterium pyogenes), Actinomyces israelii, nocardia asteroids, N. brasiliensis, Aeromonas hydrophila, Amycolata autotrophica, Archanobacterium haemolyticum (formerly Corynebacterium haemolyticum), Arizona hinshawii--all serotypes, Bacillus anthracis, Bacteroides fragilis, Bartonella henselae, B. quintana, B. vinsonii, Bordetella including B. pertussis, Borrelia recurrentis, B. burgdorferi, Burkholderia (formerly Pseudomonas species) except those listed in BSL III), Campylobacter coli, C. fetus, C. jejuni, Chlamydia psittaci, C. trachomatis, C. pneumonia, Clostridium botulinum (neurotoxin producing species), Clostridium botulinum neurotoxins, Cl. chauvoei, Cl. haemolyticum, Cl. histolyticum, Cl. novyi, Cl. septicum, Cl. Tetani, Cl. Perfirngens epsilon toxin, Corynebacterium diphtheriae, C. pseudotuberculosis, C. renale, Dermatophilus congolensis, Edwardsiella tarda, Erysipelothrix rhusiopathiae, Escherichia coli--all enteropathogenic, enterotoxigenic, enteroinvasive and strains bearing K1 antigen, including E. coli O157:H7, Haemophilus ducreyi, H. influenzae, Helicobacter pylori, Klebsiella--all species except K. oxytoca (RG1), Legionella including L. pneumophila, Leptospira interrogans--all serotypes, Listeria, Moraxella, Mycobacterium (except those listed in BSL III) including M. avium complex, M. asiaticum, M. bovis BCG vaccine strain, M. chelonei, M. fortuitum, M. kansasii, M. leprae, M. malmoense, M. marinum, M. paratuberculosis, M. scrofulaceum, M. simiae, M. szulgai, M. ulcerans, M. xenopi, Mycoplasma, Neisseria gonorrhoeae, N. meningitides, Nocardia asteroides, N. brasiliensis, N. otitidiscaviarum, N. transvalensis, Proteus mirabilis, P. vulgaris, Rhodococcus equi, Salmonella including S. arizonae, S. cholerasuis, S. enteritidis, S. gallinarum-pullorum, S. meleagridis, S. paratyphi, A, B, C, S. typhi, S. typhimurium, Shigella including S. boydii, S. dysenteriae, type 1, S. flexneri, S. sonnei, Sphaerophorus necrophorus, Staphylococcus aureus, Streptobacillus moniliformis, Streptococcus including S. pneumoniae, S. pyogenes, Treponema pallidum, T. carateum, Vibrio cholerae, V. parahemolyticus, V. vulnificus, Yersinia enterocolitica, Bartonella, Brucella including B. abortus, B. canis, B. suis, B. melitensis, Burkholderia (Pseudomonas) mallei, B. pseudomallei, Coxiella burnetii, Francisella tularensis, Mycobacterium bovis (except BCG strain, BSL IIBacterial Agents Including Chlamydia), M. tuberculosis, Mycobacteria other than tuberculosis (MOTT), Pasteurella multocida type B--"buffalo" and other virulent strains. Rickettsia akari, R. australis, R. canada, R. conorii, R. prowazekii, R. rickettsii, R, siberica, R. tsutsugamushi, R. typhi (R. mooseri), Yersinia pestis.

The analyte can be derived from a viral pathogen. For example, in some embodiments, the analyte is derived from a viral pathogen selected from among Adenoviruses, human--all types, Alphaviruses (Togaviruses), Eastern equine encephalitis virus, Eastern equine encephalomyelitis virus, Venezuelan equine encephalomyelitis vaccine strain TC-83, Western equine encephalomyelitis virus, Arenaviruses, Lymphocytic choriomeningitis virus (non-neurotropic strains), Tacaribe virus complex, Bunyaviruses, Bunyamwera virus, Rift Valley fever virus vaccine strain MP-12, Calciviruses, Coronaviruses. Flaviviruses (Togaviruses)-Group B Arboviruses, Dengue virus serotypes 1, 2, 3, and 4, Yellow fever virus vaccine strain 17D, Hepatitis A, B, C, D, and E viruses, the Cytomegalovirus, Epstein Barr virus, Herpes simplex types 1 and 2, Herpes zoster, Human herpesvirus types 6 and 7, Influenza viruses types A, B, and C, Papovaviruses, Papilloma viruses, Newcastle disease virus, Measles virus, Mumps virus, Parainfluenza viruses types 1, 2, 3, and 4, polyomaviruses (JC virus, BK virus), Respiratory syncytial virus, Human parvovirus (B 19), Coxsackie viruses types A and B, Echoviruses, Polioviruses, Rhinoviruses, Alastrim (Variola minor virus), Smallpox (Variola major virus), Whitepox Reoviruses, Coltivirus, human Rotavirus, and Orbivirus (Colorado tick fever virus), Rabies virus, Vesicular stomatitis virus, Rubivirus (rubella), Semliki Forest virus, St. Louis encephalitis virus, Venezuelan equine encephalitis virus, Venezuelan equine encephalomyelitis virus, Arenaviruses (a.k.a. South American Haemorrhagic Fever virus), Flexal, Lymphocytic choriomeningitis virus (LCM) (neurotropic strains), Hantaviruses including Hantaan virus, Rift Valley fever virus, Japanese encephalitis virus, Yellow fever virus, Monkeypox virus, Human immunodeficiency virus (HIV) types 1 and 2, Human T cell lymphotropic virus (HTLV) types 1 and 2, Simian immunodeficiency virus (SIV), Vesicular stomatitis virus, Guanarito virus, Lassa fever virus, Junin virus, Machupo virus, Sabia, Crimean-Congo hemorrhagic fever virus, Ebola viruses, Marburg virus, Tick-borne encephalitis virus complex (flavi) including Central European tick-borne encephalitis, Far Eastern tick-borne encephalitis, Hanzalova, Hypr, Kumlinge, Kyasanur Forest disease, Omsk hemorrhagic fever, and Russian Spring Summer encephalitis viruses, Herpesvirus simiae (Herpes B or Monkey B virus), Cercopithecine herpesvirus 1 (Herpes B virus), Equine morbillivirus (Hendra and Hendra-like viruses), Nipah virus, Variola major virus (Smallpox virus), Variola minor virus (Alastrim), African swine fever virus, African horse sickness virus, Akabane virus, Avian influenza virus (highly pathogenic), Blue tongue virus, Camel pox virus, Classical swine fever virus, Cowdria ruminantium (heartwater), Foot and mouth disease virus, Goat pox virus, Japanese encephalitis virus, Lumpy skin disease virus, Malignant catarrhal fever virus, Menangle virus, Newcastle disease virus (VVND), Peste Des Petits Ruminants virus, Rinderpest virus, Sheep pox virus, Swine vesicular disease virus, Vesicular stomatitis virus (exotic).

The analyte can be derived from a parasite. For example, in some embodiments, the analyte is derived from a parasite selected from among Ancylostoma human hookworms including A. duodenale, A. ceylanicum, Ascaris including Ascaris lumbricoides suum, Babesia including B. divergens, B. microti, Brugia filaria worms including B. malayi, B. timori, Coccidia, Cryptosporidium including C. parvum, Cysticercus cellulosae (hydatid cyst, larva of T. solium), Echinococcus including E. granulosis, E. multilocularis, E. vogeli, Entamoeba histolytica, Enterobius, Fasciola including F. gigantica, F. hepatica, Giardia including G. lamblia, Heterophyes, Hymenolepis including H. diminuta, H. nana, Isospora, Leishmania including L. braziliensis, L. donovani, L. ethiopia, L. major, L. mexicana, L. peruvania, L. tropica, Loa loa filaria worms, Microsporidium, Naegleria fowleri, Necator human hookworms including N. americanus, Onchocerca filaria worms including, O. volvulus, Plasmodium cynomologi, P. falciparum, P. malariae, P. ovale, P. vivax, Sarcocystis including S. sui hominis, Schistosoma including S. haematobium, S. intercalatum, S. japonicum, S. mansoni, S. mekongi, Strongyloides including S. stercoralis, Taenia solium, Toxocara including T. canis, Toxoplasma including T. gondii, Trichinella spiralis, Trypanosoma including T. brucei brucei, T. brucei gambiense, T. brucei rhodesiense, T. cruzi, or Wuchereria bancrofti filaria worms.

The analyte can be a fungal pathogen. For example, in some embodiments, the analyte is derived from a fungal pathogen selected from among Aspergillus fumigates, Blastomyces dermatitidis, Cladosporium bantianum, Candida albicans, C. (Xylohypha) trichoides, Cryptococcus neoformans, Dactylaria galopava (Ochroconis gallopavum), Epidermophyton, Exophiala (Wangiella) dermatitidis, Fonsecaea pedrosoi, Microsporum, Paracoccidioides braziliensis, Penicillium marneffei, Pneumocystis carinii, Sporothrix schenckii, Trichophyton, Coccidioides immitis, Coccidioides posadasii, Histoplasma capsulatum, H. capsulatum var. duboisii.

The analyte can be a toxin. In some embodiments, the analyte is a toxin selected from among Abrin, Botulinum neurotoxins, Clostridium perfringens epsilon toxin, Conotoxins, Diacetoxyscirpenol, Ricin, Saxitoxin, Shiga-like ribosome inactivating proteins, Shigatoxin, Staphylococcal enterotoxins, T-2 toxin, and tetrodotoxin.

In some embodiments, the analyte is selected from among Hepatitis B surface antigen (HBsAg), B. burgdorferi OspA, HPV LI, RSV F protein, Influenza hamagglutanin, Influenza stem-loop region, Influenza M2, P. falciparum merozoite surface protein 1-10, GLURP, SERA, S-antigen, 6-cys family, AMA1, EBA175, 140, 181, MTRAP, PTRAMP, ASP, Rh1, 2a, 2b, 4, 5, RAP1, 2, 3, RAMA, RHOPH1, 2, 3, P. vivax circumsporozoite protein, sporozoite surface proetin2, SSP2/TRAP, CSP-N, CSP-R, CSP-C, MSP-1, MSP-9, DBPRIII, AMA-1, Pvs25, Pvs28, S. aureus capsular polysaccharide, poly-N-acetyl glucosamine, HIV gp120, gp41, and Dengue virus conserved regions.

In another embodiment the analyte comprises at least one epitope of an allergen. Allergens can be naturally occurring, or artificial such as allergens contained in allergy vaccines. Examples of allergens include, but are not limited to, animal products (for example, Fel d 1, fur dander, cockroach calyx, wool, dust mite excretion), drugs (for example, penicillin, sulfonamides, salicylates, local anaesthetic), food (for example, celery and celeriac, corn, eggs (e.g., albumin), fruit, legumes (for example, beans, peas, peanuts, soybeans), milk, seafood (e.g., shellfish), sesame, soy, tree nuts (for example, pecans, almonds), wheat, insect venom (for example, fire ants, bee sting venom, wasp sting venom), latex, metal, plant pollen (for example, grass (e.g., ryegrass, timothy-grass, weeds (e.g., ragweed, plantago, nettle, Artemisia vulgaris, chenopodium album, sorrel), and trees (e.g., birch, alder, hazel, hornbeam, aesculus, willow, poplar, platanus, tilia, olea, Ashe juniper).

In some embodiments, the analyte is an allergen derived from a latex protein, for example, unprocessed latex sap, raw latex containing ammonia, or finished latex product in which the proteins have been exposed to chemicals and high temperatures. In some embodiments, the allergen is the allergen of a mite, for example, Dermatophagoides farinae, Dermatophagoides pteronyssinus, Acarus siro, Blomia tropicalis, Chortoglyphus arcuatas, Euroglyphus cannei, Lepidoglyphus destructor, Tyrophagus putrescentiae, or Glyphagus demesticus. In some embodiments, the allergen is from venom, for example, Bombus spp., Vespa crabro, Apis mellifera, Dolichovespula spp., Polistes spp., Vespula spp., Dolichovespula maculata, or Dolichovespula arenaria. In some embodiments, the analyte is an allergen from an insect, for example, Camponotus pennsylvanicus, Solenopsis invicta, Solenopsis richteri, Periplaneta americana, Blattella germanica, Blatta orientails, Tebanus spp., Musca domestica, Ephemeroptera spp., Culicidae sp., or Heterocera spp.

In some embodiments, the allergen analyte is epithelia, dander, or hair from an organism, for example, Serinus canaria, Felis catus (domesticus), Bos taurus, Gallus gallus (domesticus), Canis familiaris, Arias platyrhynchos, Meriones unguiculatus, Capra hircus, Anser domesticus, Cavia porcellus (cobaya), Mesocrietus auratus, Sus scrofa, Equus caballus, Mus musculus, Psittacidae, Columba fasciata, Oryctolagus cuniculus, Rattus norvegicus, or Ovis aries.

In some embodiments, the allergen analyteis from fungi, for example, Cephalosporium acremonium, Alternaria tenuis, Aspergillus glaucus, Aspergillus flavus, Aspergillus fumigatus, Aspergillus nidulans, Aspergillus niger, Aspergillus terreus, Aspergillus versicolor, Aureobasidium pullulan (Pullularia pullulans), Drechslera sorokiniana, Helminthosporium sativum, Botrytis cinerea, Candida albicans, Chaetomium globosum, Cladosporium herbarum, Cladosporium sphaerospennum (Homodendrum hordei), Drechslera spicifera (Curvularia spicifera), Epicoccum nigrum (Epicoccum purpurascens), Epidermophyton floccosum, Fusarium moniliforme, Fusarium solani, Geotrichum candidum, Gliocladium viride, Helminthosporium solani, Microsporum canis, Mucor circinelloidesf circinelloides, Mucor circinelloidesf lusitanicus, Mucor plumbous, Mycogone perniciosa, Neurospora intermedia, Nigrospora oryzae, Paecilomyces variotii, Penicillum brevicompactum, Penicillum camembertii, Penicillum chrysogenum, Penicillum digitatum, Penicillum expansum, Penicillum notatum, Penicillum roquefortii, Phoma betae, Phoma herbarum, Rhizopus oryzae, Rhizopus stolonifer, Rhodotorula mucilaginosa, Saccharomyces cerevisiae, Scopulariopsis brevicaulis, Serpula lacrymans, Setosphaeria rostrata, Stemphylium botryosum, Stemphylium solani, Trichoderma harzianum, Trichophyton mentagrophytes, Trichophyton rubrum, or Trichothecium roseum. In some embodiments, the allergen is from a smut, for example, Ustilago nuda, Ustilago cynodontis, Ustilago candis, Sporisorium cruentum, Ustilago avenae, or Ustilago tritici.

In some embodiments, the allergen analyte is from a grass, for example, Paspalum notatum, Cynodon dactylon, Poa compressa, Bromus inennis, Phalaris arundinacea, Zea cans, Elytrigia repens (Agropyron repens), Sorghum haelpense, Poa pratensis, Festuca pratensis (elatior), Avena sativa, Dactylis glomerata, Agrostis gigantea (alba), Secale cereale, Leymus (Elymus) condensatus, Lolium perenne ssp. multiflorum, Lolium perenne, Anthoxanthum odoratum, Phleum pratense, Holcus lanatus, Triticum aestivum, or Elymus (Agropyron) smithii.

In some embodiments, the allergen analyte is from a weed, for example, Atriplex polycarpa, Baccharis halimifolia, Baccharis sarothroides, Hymenoclea salsola, Amaranthus hybridus, Xanthium strumarium (commune), Rumex crispus, Eupathium capillifolium, Solidago spp., Amaranthus tuberculatus (Acnida tamariscina), Allenrolfea occidentalis, Chenopodium botrys, Kochia scoparia, Chenopodium album, Iva xanthifolia, Iva angustifolia, Chenopodium ambrosioides, Artemisia vulgaris, Artemisia ludoviciana, Urtica dioica, Amaranthus spinosus, Plantago lanceolata, Iva axillaris, Atriplex lentiformis, Ambrosia dumosa, Ambrosia acanthicarpa, Ambrosia trifida, Ambrosia artemisiifolia, Ambrosia confertiflora, Ambrosia bidentata, Ambrosia psilostachya, Salsola kali (pestifer), Artemisia californica, Artemisiafrigida, Artemisia tridentata, Atriplex wrightii, Atriplex confertifolia, or Artemisia annua.

In some embodiments, the allergen analyte is from a tree, for example, Acasia spp., Alnus glutinosa, Alnus rubra, Alnus incana ssp. rugosa, Alnus rhombifolia, Fraxinus velutina, Fraxinus pennsylvanica, Fraxinus latifolia, Fraxinus americana, Populus tremuloides, Myrica cerifera, Fagus grandifolia (americana), Casuarina equisetifolia, Betula lenta, Betula pendula, Betula nigra, Betula occudentalis (fontinalis), Betula populifolia, Acer negundo, Cryptomeria japonica, Juniperus ashei (sabinoides), Juniperus virginiana, Tamarix gallica, Populus balsamifera ssp. trichocarpa, Populus deltoides, Populusfremontii, Populus wislizeni, Populus monilifera (sargentii), Cupressus arizonoca, Taxodium distichum, Cupressus sempervirens, Ulmus americana, Ulmus crassifolia, Ulmus pumila, Eucalyptus globulus, Celtis occidentalis, Corylus americana, Corylus avellana, Carya ovata, Carya laciniosa, Carya alba, Juniferus monosperma, Juniperus princhotii, Juniperus scopulorum, Juniperus occidentalis, Robinia pseudoacacia, Mangifera indica, Acer macrophyllum, Acer rubrum, Acer saccharum, Melaleuca quinquenervia (leucadendron), Prosopis glandulosa (juliflora), Broussonetia papyrifera, Morus rubra, Morums alba, Quercus gambelii, Quercus velutina, Quercus macrocarpa, Quercus kelloggii, Quercus agrifolia, Quercus lobata, Quercus ilex, Quercus stellata, Quercus rubra, Quercus dumosa, Quercus virginiana, Quercus nigra, Quercus garryana, Quercus alba, Olea europaea, Elaegnus angustifolia, Citrus sinensis, Arecastrum romanzoffianum (Cocos plumosa), Carya illnoensis, Schinus molle, Schinus terebinthifolius, Pinus taeda, Pinus strobus, Pinus palustris, Pinus ponderosa, Pinus elliottii, Pinus virginiana, Pinus monticola, Pinus echinata, Populus nigra, Populus alba, Ligustrum vulgare, Liquidambar styraciflua, Platanus occidentalis, Platanus orientalis, Platanus racemosa, Platanus acerifolia, Juglans nigra, Juglans californica, Juglans regia, Salix lasiolepsis, Salix nigra, or Salix discolor. In some embodiments, the allergen is from a flower, for example, Chrysanthemum leucanthemum, Taraxacum officinale, or Helianthus annuus. In some embodiments, the allergen is from a farm plant, for example, Medicago sativa, Ricinus communis, Trifolium pratense, Brassica spp., or Beta vulgaris.

In some embodiments, the allergen analyte is from plant food (an edible plant), for example, Prunus dulcis, Malus pumila, Prunus armeniaca, Musa paradisiaca (sapientum), Hordeum vulgare, Phaseolus lanatus, Phaseolus vulgaris, Phaseolus sp., Phaseolus sp., Phaseolus vulgaris, Rubus allegheniensis, Vaccinium sp., Brassica oleracea var. botrytis, Fagopyrum esculentum, Brassica oleracea var. capitata, Theobroma cacao, Cucumis melo, Daucus carota, Brassica oleracea var. botrytis, Apium graveolens var. dulce, Prunus sp., Cinnamomum verum, Coffea arabic, Zea cans, Vaccinium macrocarpon, Cucumis sativus, Allium sativum, Zingiber officinale, Vitis sp., Citrus paradisi, Humulus lupulus, Citrus limon, Lactuca sativa, Agaricus campestris, Brassica sp., Myristica fragrans, Avena sativa, Olea europaea, Allium cepa var. cepa, Citrus sinensis, Vigna unguiculata, Pisum sativum, Prunus persica, Pyrus communis, Piper nigrum, Capsicum annuum var. annuum, Ananas comosus, Ipomoea batatas, Solanum tuberosum, Rubus idaeus var. idaeus, Oryza sativa, Secale cereale, Sesamum orientale (indicum), Glycine max, Spinacia oleracea, Cucurbita pepo var. melopepo, Fragaria chiloensis, Lycopersicon esculentum (lycopersicum), Brassica rapa var. rapa, Vanilla planifolia, Citrullus lanatus var. lanatus, or Triticun aestivum.

In some embodiments the allergen analyte is from fish or shellfish, for example, Micropterus sp., Ictalurus punctatus, Mercenaria mercenaria, Gadus morhua, Callinectes sapidus, Platichthys sp., Hippoglossus sp., Homarus americanus, Scomber scombrus, Crassostrea virginica, Sebastes marinus, Salmo salar, Clupeiformes, Pecten magellanicus, Penaeus sp., Salvelinus sp., or Thunnus sp. In some embodiments, the allergen is an animal food product, for example, from Bos taurus, Ovis aries, or Sus scrofa. In some embodiments, the allergen is a poultry product, for example, chicken (Gallus gallus) products or turkey (Meleagris gallopavo) products. In some embodiments, the allergen is from a dairy product, for example, bovine casein or bovine milk. In some embodiments, the allergen is a nut, for example, Bertholletia excelsa, Anacardium oceidentale, Cocos nucifera, Corylus americana, Arachis hypogaea, Carya illinoensis, Juglans nigra, or Juglans regia. In some embodiments, the allergen is dust, for example, barley grain dust, corn grain dust, house dust, mattress dust, oat grain dust, wheat grain dust, upholstery dust, or latex dust.

In some embodiments, the antigen analyte is an autoantigen associated with an autoimmune disorder. In some embodiments, the autoimmune disorder is a cell or organ-specific autoimmune disorder, and the autoantigen analyte is selected from among: acetylcholine receptor (myasthenia gravis), actin (chronic active hepatitis, primary biliary cirrhosis), adenine nucleotide translocator (ANT) (dilated cardiomyoapthy, myocarditis), beta-adrenoreceptor (dilated .degree. cardiomyopathy), aromatic L-amino acid decarboxylase (autoimmune polyendocrine syndrome type I (APS-1)), asialoglycoprotein receptor (autoimmune hepatitis), bactericidal/permeability-increasing protein (Bpi) (cystic fibrosis vasculitides), calcium-sensing receptor (acquired hypoparathyroidism), cholesterol side-chain cleavage enzyme (CYPIIa) (APS-1), collagen type IV alpha3-chain (Goodpasture syndrome), cytochrome P450 2D6 (CYP2D6) (autoimmune hepatitis), desmin (Crohn disease, coronary artery disease), desmoglein 1 (pemphigus foliaceus), desmoglein 3 (pemphigus vulgaris), F-actin (autoimmune hepatitis), GM ganglioside (Guillain-Barre syndrome), glutamate decarboxylase (GAD65) (type 1 diabetes, stiff man syndrome), glutamate receptor (GLUR) (Rasmussen encephalitis), H/K ATPase (autoimmune gastritis), 17-alpha-hydroxylase (CYP17) (APS-1), 21-hydroxylase (CYP21) (Addison disease), IA-2 (ICA512) (type 1 diabetes), insulin (type 1 diabetes, insulin hypoglycemic syndrome (Hirata disease), type B insulin resistance, acanthosis, systemic lupus erythematosus (SLE)), intrinsic factor type 1 (pernicious anemia), leukocyte function-associated antigen (LFA-1) (treatment-resistant lyme arthritis), myelin-associated glycoprotein (MAG) (polyneuropathy), myelin basic protein (multiple sclerosis, demyelinating disease), myelin oligodendrocyte glycoprotein (MOG) (multiple sclerosis), myosin (rheumatic fever), p-80-Coilin (atopic dermatitis), pyruvae dehydrogenase complex-E2 (PDC E2) (primary biliary cirrhosis), sodium iodide symporter (NIS) (Graves disease, autoimmune hypothyroidism), SOX-10 (vitiligo), thyroid and eye muscle shared protein (autoimmune thyroiditis), thyroid peroxidase (autoimmune Hashimoto thyroiditis), thyrotropin receptor (Graves disease), tissue transglutaminase (celiac disease), transcription coactivator p75 (atopic dermatitis), tryptophan hydroxylase (APS-1), tyroisinase (vitiligo, metastatic melanoma), and tyrosine hydroxylase (APS-1), wherein the associated autoimmune disorder(s) is listed parenthetically immediately after each autoantigen analyte.

In some embodiments, the autoimmune disorder is a systemic autoimmune disorder, and the autoantigen analyte is selected from among: ACTH (ACTH deficiency), aminoacyl-tRNA histidyl synthetase (myositis, dermatomyositis), aminoacyl-tRNA synthetase (polymyositis, dermatomyositis), cardiolipin (SLE), carbonic anhydrase II (SLE, Sjogren syndrome, systemic sclerosis), collagen (rheumatoid arthritis (RA), SLE, progressive systemic sclerosis), centromere-associated protein (systemic sclerosis), DNA-dependent nucleosome-stimulated ATPase (dermatomyositis), fibrillarin (scleroderma), fibronectin (SLE, RA, morphea), glucose-6-phosphate isomerase (RA), Beta2-glycoprotein I (Beta2-GPI) (primary antiphospholipid syndrome), golgin (95, 97, 160, and/or 180) (Sjogren syndrome, SLE, RA), heat shock protein (various immune related disorders), hemidesmosomal protein 180 (bullous pemphigoid, herpes gestationis, cicatricial pemphigoid, histone H2A-H2B-DNA (SLE), IgE receptor (chronic idiopathic urticaria), keratin (RA), Ku-DNA-protein kinase (SLE), Ku-nucleoprotein (connective tissue syndromes), La phosphoprotein (La 55-B) (Sjoren syndrome), myeloperoxidase (necrotizing and cescentic glomerulonephritis (NCGN), system vasculitis), proteinase 3 (PR3) (Wegener granulomatosis, Churg-Strauss syndrome), RNA polymerase I-III (RNP) (systemic sclerosis, SLE), signal recognition protein (SRP54) (polymyositis), topoisomerase-1 (Scl-70) (scleroderma, Raynaud syndrome), tubulin (chronic liver disease, visceral leishmaniasis), and vimentin (systemic autoimmune disease), wherein the associated autoimmune disorder(s) is listed parenthetically immediately after each autoantigen.

In some embodiments, the autoimmune disorder is a plasma protein autoimmune disorder or cytokine autoimmune disorder, and the autoantigen analyte is selected from among: C1 inhibitor (autoimmune C1 deficiency), C1q (SLE, membrane proliferative glomerulonephritis (MPGN)), cytokine (e.g., IL-1 alpha, IL-1beta, IL-, IL-10, LIF) (RA, systemic sclerosis), factor II (prolonged coagulation time), factor V (prolonged coagulation time), factor VII (prolonged coagulation time), factor VIII (prolonged coagulation time), factor IX (prolonged coagulation time), factor X (prolonged coagulation time), factor XI (prolonged coagulation time), factor XII (prolonged coagulation time), thrombin (prolonged coagulation time), vWF (prolonged coagulation time), glycoprotein IIb/IIIg and Ib/IX (autoimmune thrombocytopenia purpura), IgA (immunodeficiency), and oxidized LDL (OxLDL) (atherosclerosis), wherein the associated autoimmune disorder(s) is listed parenthetically immediately after each autoantigen analyte.

In some embodiments, the autoimmune disorder is a cancer or paraneoplastic autoimmune disorder, and the autoantigen analyte is selected from among: amphiphysin (neuropathy, small lung cell cancer), cyclin B 1 (hepatocellular carcinoma), DNA topoisomerase II (liver cancer), desmoplakin (paraneoplastic pemphigus), gephyrin (paraneoplastic stiff man syndrome), Hu protein (paraneoplastic encephalomyelitis), neuronal nicotinic acetylcholine receptor (subacute autonomic neuropathy, cancer), p53 (cancer, SLE), p62 (IGF-II mRNA-binding protein) (hepatocellular carcinoma), recoverin (cancer-associated retinopathy), R1 protein (paraneoplastic opsoclonus myoclonus ataxia), beta IV spectrin (lower motor neuron syndrome), synaptotagmin (Lambert-Eaton myasthenic syndrome), voltage-gated calcium channels (Lambert-Eaton myasthenic syndrome) and Yo protein (paraneoplastic cerebellar degeneration).

In some embodiments, the antigen analyte is an endogenous antigen that is an aberrantly expressed polypeptide. Examples of such endogenous antigens include, but are not limited to, amyloid beta (A-beta or A.beta.), alpha synuclein, cystatin C, tau, ABri, ADan, superoxide dismutase (SOD), mutant Huntington, PrP.sup.sc or a fragment of any of the foregoing.

In some embodiments of the invention, the analyte comprises at least one epitope of an implant to be introduced into a subject, metabolic or degradation products of an implant material, or substances that specifically bind to an epitope of an implant material,, such as antibodies developed to an implant material or its degradation products Such implants can include, for example, electrically powered implants (for example, artificial pacemakers), bioimplants (biomaterial surgically implanted in a subject's body to replace damaged tissue (for example, orthopedic reconstructive prosthesis), cardiac prostheses (artificial valves), skin, and cornea), contraceptive implants, dental implants, orthopedic implants, and adhesion prevention devices. Examples of implant materials that can bear epitopes include latex; silicone; metals, such as cobalt chrome (Co--Cr) alloys, titanium, and titanium alloys; polymers, such as ultrahigh molecular weight polyethylene (UHMWPE) and polymethyl methacrylate cement (PMMA); and bioceramics, such as hydroxyapatite and Bioglass.

### Non-Antibody Signal Transducing Element

Exemplary embodiments of the present invention include various non-antibody signal transducing elements. Each signal transducing element is adapted to receive, i.e., bind, a detector molecule that is itself adapted to receive, i.e., bind, a specific analyte of interest. In one embodiment, the signal transducing element is a transmembrane chimeric fusion protein that is engineered into and expressed on the surface of the biosensor cell, and that is adapted to activate the signal transduction pathway that ultimately results in the reporter protein emitting a detectable signal. In another embodiment, the signal transducing element is a soluble chimeric fusion protein that is adapted to bind to a cell surface signal transducer, such as a native receptor or receptor protein that is adapted to activate the signal transduction pathway that ultimately results in the reporter protein emitting a detectable signal. In still another embodiment, the signal transducing element is a soluble chimeric fusion protein that is engineered into and expressed by the biosensor cell. The soluble chimeric fusion protein is then secreted/excreted into the extracellular space where it binds to a cell surface signal transducer, such as a native receptor or receptor protein that is adapted to activate the signal transduction pathway that ultimately results in the reporter protein emitting a detectable signal.

The chimeric fusion proteins of this invention may include: (i) a component of a protein that is adapted to bind to the at least one type of detector molecule (e.g., a soluble antibody); and (ii) a component of a receptor complex normally expressed by the living, engineered biosensor cell. In some embodiments, the component of the protein that is adapted to bind to the at least one type of detector molecule may be derived from a bacterial binding protein (i.e., an antibody binding protein derived from a bacteria) such as, for example, the IgG binding domain of a strep G protein (referred to herein as IgGbp or Igbp in the Figures). Tandem repeats of this IgG binding domain may be included to increase the affinity of the binding protein for the soluble antibody. In an alternate embodiment, the component of the chimeric fusion protein that is adapted to bind to the at least one type of detector molecule is an antibody binding domain derived from a receptor protein such as, for example, the murine Fc gamma RI (FcγRI) receptor. In various exemplary embodiments, the component of the receptor complex normally expressed by the living, engineered biosensor cell is IgM (for B cell biosensors); Igα/β (for B cell biosensors); IgE (for mast cell biosensors); CD19 (for B cell biosensors), CD3zeta (for T cell biosensors), or FcεRI (for mast cell biosensors).

The non-antibody signal transducing elements of this invention may include either complete protein sequences or engineered protein fragments such as selected protein domains derived from larger protein molecules. One of ordinary skill in the art will appreciate that fragments of larger molecules may be created using standard genetic engineering techniques such as synthetic gene technology. When fragments of larger proteins are used to engineer antibody binding motifs as aspects of chimeric fusion proteins it is important to design the engineered proteins to ensure proper conformational folding of the selected protein fragments. Therefore, it is useful to include (in the fusion proteins) short spacer or linker elements that do not readily form protein secondary structures. Short combinations of amino acids such as glycine, serine and alanine, for example, may be used for these spacer or linker elements. In an exemplary embodiment, the amino acid sequence glycine (G), serine (S), alanine (A), serine (S), glycine (G), serine (S), glycine (G) is used to separate a binding domain from a component of a receptor complex in an engineered protein molecule (see SEQ ID NO: 19). With regard to the peptide linker or spacer used to connect the detector element to the signal-transducing element or interconnect different segments of a signal-transducing element: the linker typically joins the carboxyl terminus of one element to the amino terminus of another. Peptide linkers may vary from 0 to 25 amino acids in length or any intermediate integer value and typically, but not always, comprise hydrophilic amino acids such as glycine (G) and serine (S).

As previously stated, each signal transducing element binds to a detector molecule that binds to a specific analyte of interest. A detector molecule that has bound to an analyte will either (i) bind to a transmembrane signal transducing element; or (ii) to a signal transducing element that will itself bind to a cell surface signal transducer (e.g., native receptor). In the first situation, upon the binding of a sufficient number of analytes to a sufficient number of detector molecules that are themselves bound to transmembrane non-antibody signal transducing elements, an aggregation of signal transducing elements occurs on the biosensor cell surface, the signal transduction pathway is activated, the biological process occurs, and the detectable signal is emitted by the reporter protein. In the second case, upon the binding of a sufficient number of analytes to a sufficient number of detector molecules to a sufficient number of non-antibody signal transducing elements that are themselves bound to the appropriate native receptor, an aggregation of the receptors occurs on the cell surface, the signal transduction pathway is activated, the increase in intracellular calcium occurs, and detectable light is emitted by the reporter protein.

A first non-antibody signal transducing element in accordance with an exemplary embodiment of the present invention includes a bacterial binding protein (IgGbp) fused to the IgM heavy chain constant domain (B cell) with a GSASGSG linker. SEQ ID NO: 1 provides the DNA sequence for signal transducing element IgGbp-IgM and SEQ ID NO: 2 provides the protein sequence for signal transducing element IgGbp-IgM.

A second non-antibody signal transducing element in accordance with an exemplary embodiment of the present invention includes a bacterial binding protein (IgGbp) fused to the Igα/β component of the B cell receptor with a GSASGSG linker. SEQ ID NO: 3 provides the DNA sequence for signal transducing element IgGbp-Igα/β and SEQ ID NO: 4 provides the protein sequence for signal transducing element IgGbp-Igα/β.

A third non-antibody signal transducing element in accordance with an exemplary embodiment of the present invention includes a bacterial binding protein (IgGbp) fused to the CD3 zeta chain of the T-cell receptor with a GSASGSG linker. SEQ ID NO: 5 provides the DNA sequence for signal transducing element IgGbp-CD3ζ and SEQ ID NO: 6 provides the protein sequence for signal transducing element IgGbp-CD3ζ.

A fourth non-antibody signal transducing element in accordance with an exemplary embodiment of the present invention includes the FcγRI antibody binding domain fused to the IgM heavy chain constant domain (B cell) with a GSASGSG linker. SEQ ID NO: 7 provides the DNA sequence for signal transducing element FcγRI-IgM and SEQ ID NO: 8 provides the protein sequence for signal transducing element FcγRI-IgM.

A fifth non-antibody signal transducing element in accordance with an exemplary embodiment of the present invention includes the FcγRI antibody binding domain fused to the Igα/β component of the B-cell receptor with a GSASGSG linker. SEQ ID NO: 9 provides the DNA sequence for signal transducing element FcγRI-Igα/β and SEQ ID NO: 10 provides the protein sequence for signal transducing element FcγRI-Igα/β.

A sixth non-antibody signal transducing element in accordance with an exemplary embodiment of the present invention includes the FcγRI antibody binding domain fused to the CD3 zeta chain of the T-cell receptor with a GSASGSG linker. SEQ ID NO: 11 provides the DNA sequence for signal transducing element FcγRI-CD3ζ and SEQ ID NO: 12 provides the protein sequence for signal transducing element FcγRI-CD3ζ.

A seventh exemplary non-antibody signal transducing element in accordance with the present invention includes a bacterial binding protein (IgGbp) fused to the IgE constant domain (B cell) with a GSASGSG linker. SEQ ID NO: 13 provides the DNA sequence for signal transducing element IgGbp-IgE and SEQ ID NO: 14 provides the protein sequence for signal transducing element IgGbp-IgE.

An eighth exemplary non-antibody signal transducing element in accordance with the present invention includes the FcγRI antibody binding domain fused to the IgE constant domain (B cell) with a GSASGSG linker. SEQ ID NO: 15 provides the DNA sequence for signal transducing element FcγRI-IgE and SEQ ID NO: 16 provides the protein sequence for signal transducing element FcγRI-IgE.

A ninth exemplary non-antibody signal transducing element in accordance with the present invention includes monomeric streptavidin fused to the CD3 zeta chain of the T-cell receptor with a GSASGSG linker. SEQ ID NO: 17 provides the DNA sequence for signal transducing element mSA-CD3ζ and SEQ ID NO: 18 provides the protein sequence for signal transducing element mSA-CD3ζ. Monomeric streptavidin is a recombinant form of streptavidin that includes mutations that break the streptavidin tetramer into a monomer and to enhance the solubility of the resultant isolated subunit.

### Example Biosensor I

With reference to FIGS. 1a-b, a first biosensor 100 in accordance with an exemplary embodiment of the present invention includes Jurkat T cells 102 that have been engineered to produce aequorin 104 and that have been charged with CTZ 106 to form an aequorin/CTZ complex, as previously described. This particular biosensor has also been engineered to express the transmembrane non-antibody signal transducing element 108, which is IgGbp-CD3ζ (SEQ ID NOS: 5-6), although the transmembrane non-antibody signal transducing element FcγRI-CD3ζ (SEQ ID NO: 11-12) may also be used with biosensor 100. Biosensor cell 102 also includes at least one signal transduction pathway 110, the activation of which results in an increase of intracellular Ca2+ 112. As shown in FIG. 1b, when a sufficient number of detector molecules 114 (e.g., soluble antibodies) to which target analyte 116 (e.g., *E. coli* 0157) is bound bind to transmembrane non-antibody signal transducing elements 108, signal transduction pathway 110 is activated, intracellular Ca2+ 112 increases, the aequorin/CTZ complex undergoes a conformational change and emits a signal (photon) of light 118 which is detected by photo multiplier tube 120, and spike 122 is graphically displayed on a testing device (see description below), indicating the presence of target analyte 116 within a sample being tested. The display may be both qualitative and quantitative with regard to target analyte 116.

### Example Biosensor II

With reference to FIGS. 2a-b, a second biosensor 200 in accordance with an exemplary embodiment of the present invention includes MC/9 (ATCC® CRL-8306™) mast cells 202 that have been engineered to produce aequorin 204 and that have been charged with CTZ 206 to form an aequorin/CTZ complex, as previously described. This particular biosensor expresses the native Fc epsilon receptor (i.e., FcεRI) 207, which binds to soluble non-antibody signal transducing element 208, which is IgGbp-IgE (SEQ ID NOS: 13-14), although the non-antibody signal transducing element FcγRI-IgE. (SEQ ID NO: 15-16) may also be used with biosensor 200. As shown in FIG. 2b, when a sufficient number of detector molecules 214 (e.g., soluble antibodies) to which target analyte 216 (e.g., *E. coli* 0157) is bound bind to non-antibody signal transducing elements 208 that have previously bound to native Fc epsilon receptors 207, signal transduction pathway 210 is activated, intracellular Ca2+ 212 increases, the aequorin/CTZ complex undergoes a conformational change and emits a signal (photon) of light 218 which is detected by photo multiplier tube 220, and spike 222 is graphically displayed on a testing device (see description below), indicating the presence of target analyte 216 within a sample being tested. The display may be both qualitative and quantitative with regard to target analyte 216.

### Example Biosensor III

With reference to FIGS. 3a-b, a third biosensor 300 in accordance with an exemplary embodiment of the present invention includes MC/9 (ATCC® CRL-8306™) mast cells 302 that have been engineered to produce aequorin 304 and that have been charged with CTZ 306 to form an aequorin/CTZ complex, as previously described. This particular biosensor expresses the native Fc epsilon receptor (i.e., FcεRI) 307, which binds to non-antibody signal transducing element 308, which is IgGbp-IgE (SEQ ID NOS: 13-14), although the non-antibody signal transducing element FcγRI-IgE. (SEQ ID NO: 15-16) may also be used with biosensor 300. In this particular embodiment, biosensor cells 302 have been further engineered to express IgGbp-IgE and excrete this non-antibody signal transducing element into the extracellular space, wherein it binds to the native FcεRI expressed on the cell surface. As shown in FIG. 3b, when a sufficient number of detector molecules 314 (e.g., soluble antibodies) to which target analyte 316 (e.g., *E. coli* 0157) is bound bind to non-antibody signal transducing elements 308 that have previously bound to native Fc epsilon receptors 307, signal transduction pathway 310 is activated, intracellular Ca2+ 312 increases, the aequorin/CTZ complex undergoes a conformational change and emits a signal (photon) of light 318 which is detected by photo multiplier tube 320, and spike 322 is graphically displayed on a testing device (see description below), indicating the presence of target analyte 316 within a sample being tested. The display may be both qualitative and quantitative with regard to target analyte 316.

### Example Biosensor IV

With reference to FIGS. 4, a fourth biosensor 400 in accordance with an exemplary embodiment of the present invention includes biosensor cells 402 that have been engineered to produce aequorin and to express transmembrane non-antibody signal transducing element 408, which is mSA-CD3ζ (SEQ ID NO: 17-18). Non-antibody signal transducing element mSA-CD3ζ (monomeric streptavidin-CD3ζ) binds to a biotinylated detector molecule 414, which specifically binds to a target molecule 416 such as, for example, epidermal growth factor (EGF). An anti-target molecule antibody 417 such as, for example, anti-EGF, creates target multimers that cluster multiple signal transducing elements and induce signal transduction as previously described. In other embodiments, the monomeric streptavidin component is replaced with a biotinylated component and alternate linkage means may be employed.

### Example Biosensor V

With reference to FIG. 5, a fifth biosensor 500 in accordance with an exemplary embodiment of the present invention includes biosensor cells 502 that have been engineered to produce aequorin and to express transmembrane non-antibody signal transducing element 508, which is mSA-CD3ζ (SEQ ID NO: 17-18). Non-antibody signal transducing element mSA-CD3ζ (monomeric streptavidin-CD3ζ) binds to a biotinylated detector molecule 514, which in some embodiments is an autoantigen molecule. Biotinylated detector molecule 514 specifically binds to a target molecule 516, which in some embodiments is an anti-autoantigen molecule. Autoantibodies in a serum sample create target multimers that cluster multiple signal transducing elements and induce signal transduction as previously described. In other embodiments, the monomeric streptavidin component is replaced with a biotinylated component and alternate linkage means may be employed.

The amino acid sequences of the signal-transducing polypeptide used to produce the chimeric proteins of the invention may have at least 70%, 75%, 80%, 85%, 87.5%, 90%, 92.5%, 95%, 97.5%, 98%, 99% sequence identity or similarity to the proteins or domains identified by or in the following accession numbers: IgM heavy chain (GenBank: CAC20458.1), Tg-alpha (P11912.2, GI:547896), Ig-beta (P40259.1 GI:728994), CD19 (AAA69966.1 GI:901823), CD3zeta (P20963.2, GI: 23830999), IgE alpha (1F2Q A, GI:9257150) and Fc-epsilonR1 subunit alpha (P12319.1, GI: 119865).

*Staphylococcus aureus* Protein A (P02976.3, GI: 110283003) is encoded by the *spa* gene of *Staphylococcus aureus* and its structure, including its Ig-binding segments, and immunoglobulin-binding properties are well-known and are incorporated by reference to Graille, et al., Proc Natl Acad Sci U S A. 2000 May 9;97(10):5399-404; and Roben, et al. J Immunol. 1995 Jun 15;154(12):6437-45. Variants of Protein A or its immunoglobulin-binding segments having at least 70%, 75%, 80%, 85%, 87.5%, 90%, 92.5%, 95%, 97.5%, 98%, 99% sequence identity or similarity to known Protein A amino acid sequences and the capacity to bind to an immunoglobulin or other analyte, such as those described by Graille, *et al.* and Roben, *et al*., may be produced by molecular biological techniques well-known in the art including by direct synthesis of a nucleic acid encoding an immunoglobulin-binding amino acid sequence.

Other bacterial immunoglobulin-binding proteins, such as *Streptococcus* Protein G and engineered variants of such proteins, are known and are incorporated by reference to Bailey, et al., J Immunol Methods. 2014 Dec 15; 415:24-30 (doi: 10.1016/j.jim.2014.10.003) (Epub 2014 Oct 22); and to Watanabe, et al., J Biol Chem. 2009 May 1; 284(18):12373-8 (doi: 10.1074/jbc.M809236200)(Epub 2009 Mar 6). Variants of Protein G or its immunoglobulin-binding segments having at least 70%, 75%, 80%, 85%, 87.5%, 90%, 92.5%, 95%, 97.5%, 98%, 99% sequence identity or similarity to known Protein G amino acid sequences and the capacity to bind to an immunoglobulin or other analyte, such as those described by Bailey, *et al.* and Watanabe, *et al.* may be produced by molecular biological techniques well-known in the art including by direct synthesis of a nucleic acid encoding an immunoglobulin-binding amino acid sequence.

Fc receptors (FcR) bind to the Fc portion of an immunoglobulin and many types such Fc receptors are known, including FcγRI and FcεRI. The structural and functional binding characteristics of these FcRs are incorporated by reference to Fridman, FASEB J. 1991 Sep; 5(12):2684-90. Variants of FcRs or their immunoglobulin-binding segments having at least 70%, 75%, 80%, 85%, 87.5%, 90%, 92.5%, 95%, 97.5%, 98%, 99% sequence identity or similarity to a known FcR amino acid sequence, such as a sequence described by Fridman, may be produced by molecular biological techniques well-known in the art including by direct synthesis of a nucleic acid encoding an immunoglobulin-binding amino acid sequence.

A signal transducing protein according to the invention may have at least 70%, 75%, 80%, 85%, 87.5%, 90%, 92.5%, 95%, 97.5%, 98%, 99% sequence identity or similarity to the disclosed chimeric signal transducing proteins described by SEQ ID NOS: 2, 4, 6, 8, 10, 12, 14, 16, and 18 and also have the ability to bind an analyte, such as an immunoglobulin, and then transduce a signal into the engineered biosensor cell. Such variants may be constructed by methods well known in the molecular biological arts or by chemical synthesis of polynucleotides encoding the variant chimeric reporter proteins, insertion of the encoding sequences into a vector, and transformation or transfection of a competent cell with the vector.

### Cell Sorting and Cloning

The design and construction of the biosensors of this invention resulted in a mixed population of biosensor cells when cultured. Some cells did not express the engineered factors while others expressed the factors at varying levels. Following successful electroporation and gene insertion, biosensor cells were cultured and tested for biological response (flash signal) as mixed populations. Single cell sorting was performed using a Flow Cytometer. Cells were isolated and then expanded for analysis to select those that expressed high levels of the desired proteins. For this process, fluorescently labeled antibodies were used to target different receptors on the biosensor cells, thereby enabling the sorting process. Individual clones were screened for signaling and the best clones were selected. Through this process, the most suitable clones were identified and isolated. Fluorescence-Activated Cell Sorting (FACS) and live cell staining for extracellular protein was conducted as described below.

Engineered biosensor cells were counted, gently centrifuged, and re-suspended in wash buffer (HBSS + 2% BSA) to a final concentration of 1x10⁷-1x10⁸ cells/mL. In each experiment, either a full antibody with the Fc region or F(ab)₂ was used. When using the full antibody, 1-0.5 µg of Fc receptor blocking antibody was added to each empty 12 x 15 mm tube that was to receive cells. Into each of these tubes, 100 µL of cells (1x10⁶ to 1x10⁷ cells) was added on top of the Fc blocking antibody. Cells were mixed gently and incubated for 15 minutes at 4°C or room temperature. When using F(ab)₂, the previous step of blocking Fc was omitted. A total of 1 µg of primary antibody (against the receptor of choice) was added, and the cells were then mixed gently before incubating for 20-40 minutes on ice (or at 4°C). This temperature prevented receptor internalization. Cells were gently agitated (swirled) intermittently to encourage labeling. A 2 mL volume of cold wash buffer was added then cells were centrifuged at 4°C and the supernatant discarded. The wash step was repeated before re-suspending cells in 100 µL of wash/sorting buffer. A secondary FITC-labeled antibody was added (0.5-1 µg) to the cells and mixed before incubating on ice (or at 4°C) for 20-40 minutes. Cells were protected from light during the entire process. A 2 mL volume of cold wash buffer was added then cells centrifuged and supernatant discarded. The wash step was repeated and cells re-suspend in 0.5-1 mL of wash buffer. Cells were incubated on ice until the time for sorting. Sorting was done as soon as possible (at least on the same day). Cloning and culturing cells after single cell sorting was conducted as described below.

Biosensor cells were sorted into 96 well plates with each well containing one cell and 100-200 µL cell growth media. Plates were scanned/monitored for the next 10 to 14 days to determine the rate of growth and to judge when to transfer to a 24 well plate. During scanning, different markings were used for different conditions. Some wells were marked if they contained live cells, but were not ready for transfer and contaminated wells were also marked. Cells were transferred to a 24 well plate containing 1.0 mL of the appropriate media in each well. In cases of contaminated cells, washing was done by adding all of the cell suspension from a well into 5ml of sterile 1x PBS in a 15mL conical tube. Cells were then centrifuged at 170 RCF for 10 minutes and the supernatant discarded. The pellet was re-suspended in 1.0 mL of fresh media in a 24 well plate to be cultured. Following continued growth, cells were transferred to a 12 well plate with 1.5 mL of fresh media per well.

For clone screening, after growing in a 12 well plate, cells were counted to determine if they were ready for charging and flash testing. During flash testing, a single iteration involved 25,000 cells. Enough cells were grown to accommodate testing and also leave some to continue growing. This step marked the first round of clone screening. Selected clones were grown further and subjected to subsequent tests depending on the desired properties. For biological response, for example, Jurkat-FcγRI-CD3ζ clones were screened using anti-CD3ε antibodies (positive control) and monoclonal antibodies against bacteria with the respective bacteria while Digitonin was used for chemical response test. MC/9-Aeq clones were screened using anti-FcεRI antibodies (biological response) and Digitonin (chemical response).

In summary, fluorescence-activated cell sorting (FACS) was performed using fluorescent antibody labels to select and isolate cells which were highly expressing the desired proteins, which in this case were the engineered receptors. This process resulted in a population of highly-expressing biosensor cells which was further confirmed by flash testing using the PMT in the testing device. During the entire process, cells were counted using an automatic cell counter to eliminate human error and enhance consistency and efficiency. Different clones of Jurkat-FcγRI-CD3ζ gave different levels of biological response when tested with Anti-*E. coli* O111 mAb and *E. coli* O111 bacteria. Many clones were tested the same way and the highest responders were saved in a clone bank. Likewise, chemical response results obtained from testing different MC/9-Aeq clones using the chemical Digitonin indicated that different clones gave different levels of chemical response depending on the level of Aequorin expression. The clones with the highest signal were saved in the clone bank.

### Culturing Biosensor Cells

Different culture media formulations were used for different cell lines to ensure optimal growth conditions. MC/9 mast cells were cultured in Complete Mast Cell Media (DMEM - Sigma, Cat. No. D5796; 1x Pen/Strep; 10% FBS; 10% T-Stim Supplement; 50 µM β-mercaptoethanol). Jurkat T-cells were cultured in complete RPMI media (RPMI-ThermoFisher; 10% FBS; 1x Pen/Strep). Depending on the characteristics of the electroporated constructs, different antibiotics were used for selection in cell culture. Appropriate antibiotics were added to the growth media 2-3 days after electroporation to select for cells that had successfully integrated the linearized DNA constructs. Cell concentration was kept between 4.0 x 10⁵ and 1.0 x 10⁶ cells/mL for optimal cell growth. Different cell lines and clones were processed for long term storage and stocks were frozen in liquid nitrogen as follows: (i) cells were centrifuged at 150 RCF for 10 minutes and the supernatant was discarded; (ii) the cell pellet was re-suspended in freezing media (RPMI; 50% FBS; 10% DMSO) at a concentration of 5.0 x 10⁵ cells/mL; and (iii) volumes of 1 mL were aliquoted into 2 mL Nunc Cryo-vials and frozen at -80°C for 24 hours before being transferred to liquid nitrogen for long term storage.

### Charging Biosensor Cells

The biosensor cells of this invention were centrifuged in 50 mL conical tubes at 150 RCF for 10 minutes. The supernatant was discarded and pellet re-suspended in charging media (RPMI; 10% antibody-depleted FBS; 1x pen/strep; 0.1% Pluronic F68 and 1.5 mM coelenterazine) at a concentration of 25,000 cells/180 µL. Additionally, cells were also been charged at different concentrations such as, for example, 100,000 cells/180 µL and 400,000 cells/180 µL. Cells were charged at room temperature with gentle shaking/rocking for 24-26 hours. Before use, the commercially available antibody-depleted FBS may be purified further using other antibody depletion systems.

### Concentrating Cells

The biosensor cells of this invention were demonstrated to be more effectively charged at lower concentrations rather than higher concentrations. For example, charging cells at a density of 25,000 cells/180 µL versus 400,000 cells/180 µL was shown to result in a two-fold increase in detectable signal. Jurkat-FcγRI-CD3ζ clone P5G7 cells were charged at both 400,000 cells/180 µL and 25,000 cells/180 µL then tested at 400,000 cells/180 µL in each reaction. Overnight *E. coli* O111 bacteria culture was used with 23 nM anti-*E. coli* O111 mAb. Biosensor cells charged at a lower concentration gave a higher signal for the same number of bacteria cells tested. Biosensor cell density is mostly changed by concentrating the cells after charging to allow for optimal pathogen detection. Different concentrations were used for pathogen detection depending on the target pathogen and quality of the antibody, when the detector molecule is a soluble antibody. Biosensor cells are concentrated by centrifugation at 150 RCF for 10 minutes and the cell pellet is re-suspended in the desired volume of the testing medium. The charging medium may also serve as the testing medium. In certain instances, addition of normal FBS to the media triggered a biological response resulting in a biosensor signal (flash) due to high concentration of antibodies in normal FBS. Therefore, commercially available antibody-depleted FBS was used in the charging process, which reduced the antibody triggered signal without totally eliminating it. Additional methods were used to further deplete traces of antibodies in the commercially available antibody-depleted FBS.

### Bioassay

In exemplary embodiments of this invention, the analyte bioassay is formatted with the biosensor cell and a soluble monoclonal antibody (mAb) that is specific for that analyte (e.g., pathogen). In these embodiments, the specificity of the bioassay is directly related to the selective binding of the soluble antibody to the target analyte and the specificity and sensitivity of the biosensor is determined by detection and measurement of bioluminescence. In this process, biosensor cells are initially charged using the light-emitting molecule, coelenterazine (CTZ). The soluble antibody of choice and the sample being analyzed are then added. If a target pathogen is present in the sample, it interacts with the soluble antibody, which binds to a fusion protein expressed by the biosensor cell, ultimately triggering a signal cascade that results in light emission from the biosensor cell. The emitted light is detected by a photo multiplier tube (PMT) in the testing device and the signal emitted by the biosensor cell is displayed as photon counts per second. As described below, various methods have been developed to detect pathogens based on the soluble antibody and the target pathogen. Three such methods, described in detail below, include: (i) instant addition of detector molecules (e.g., antibodies); (ii) coating biosensor cells with detector molecules (e.g., antibodies); and (iii) coating analytes (e.g., bacteria) with antibodies.

### Testing Unit

The bioassay aspect of the present invention herein may be carried out in a testing subunit or test cartridge designed for use with a bench-top or portable testing system and device such as that disclosed in U.S. Patent Application No. 13/711,296 (U.S. Patent No. 9,023,640), which is incorporated by reference herein, in its entirety. The test cartridge, which may be a single-use, disposable item, receives both the sample and the biosensor and introducing the biosensor into the test cartridge mixes the sample and the biosensor in a predictable and controlled manner. The test cartridge further includes a reaction chamber for receiving the test sample and the biosensor, wherein the reaction chamber has a predetermined internal geometry and has been further adapted to minimize or eliminate background noise for the purpose of improving the overall signal to noise ratio. At least one stabilizer may be located in the reaction chamber for minimizing shear force damage to the test sample and biosensor during the mixing process.

In an exemplary embodiment, the reaction chamber and fluid channels that lead to the reaction chamber within the test cartridge are designed to achieve several objectives. An inlet channel for fluid entering the reaction chamber includes a tubular shape and the diameter of the tube is relatively small and tapers to become smaller at the inlet to the reaction chamber. This increases the velocity of fluid entering the reaction chamber and promotes more vigorous and homogenous mixing due to the bulk motion of the reagents within the reaction chamber. It is desirable to mix the reagents and sample in a way to promote mixing beyond molecular diffusion, in order to minimize the duration of the test by ensuring that any infectious agent present in the sample rapidly encounters the biosensor. The inlet channel may be offset from the central axis of the reaction chamber to promote a clockwise or counterclockwise rotational motion of the reagents around the central axis of the test chamber as the fluids are mixed in order to increase homogeneity of the mixture. The inlet channel is also approximately tangent to the interior surface of the reaction chamber for allowing incoming fluid to travel from the inlet channel to the reaction chamber while remaining in contact with the side surface of the reaction chamber, which allows for a minimally turbulent flow and minimal introduction of air bubbles into the mixed fluids. Bubbles are undesirable due to the unpredictable refraction of light they cause as light emitted by the reagents travels through bubbles within the mixed reagents or on the surface of the mixed reagents. The axis of the inlet channel may be angled above horizontal (e.g., about 30 degrees) to provide a partially downward direction to the incoming fluid flow to ensure that the reagent is mixed with the fluid residing at the bottom of the reaction chamber. Alternatively, the reagents may be introduced to the test chamber using alternative fluid delivery means such as a vertical channel to deliver the reagents to the bottom of the reaction chamber, or delivering the fluid directly on the central axis of the test chamber in order to create a column of reagent flowing into the test chamber thereby promoting mixing through entrainment.

The shape (i.e., predetermined geometry) of the reaction chamber may be a revolved section facilitating clockwise or counterclockwise motion of the mixing fluids around the central axis of the reaction chamber. Alternatively, if desired, a reaction chamber shape other than a revolved section such as a rectangular or irregular shape may be utilized. In one embodiment, the revolved section used to form the reaction chamber is a portion of an ellipse for facilitating the collection of stray light emitted by the reagents and reflecting this light toward the surface of the detector, which may be a photomultiplier tube (PMT) (Hamamatsu). The surface of the reaction chamber may be reflective, in order to enhance the light collection properties of the elliptical shape. In some embodiments, the maximum diameter of the surface of the PMT is limited to achieve a maximum signal to noise ratio of the output of the system. The diameter of the reaction chamber may be designed to approximately match the diameter of the PMT, which influences the elliptical shape that can be achieved in a reaction chamber designed to hold a specific volume of fluids. Due to the constrained elliptical shape, the reaction chamber surface color may be a partially diffusing white due to the additional light collection that occurs when light that would not otherwise be reflected directly to the PMT surface is partially diffused by the white surface and a fraction of this is directed toward the PMT surface. Alternatively, other surface finishes and materials such as a near-mirror finish aluminum, or a transparent material could be used if desired. Further, it is desirable for the reaction chamber material to be minimally phosphorescent, in order to prevent light emitted from the reaction chamber itself from eclipsing any emitted light from the reagents and preventing detection. Although white polymeric materials such as acrylonitrile butadiene styrene or other such polymeric materials have been found to exhibit a low level of phosphorescence, the additional light collection provided by the combination of light reflection and diffusion has been found to be a benefit to the signal to noise ratio of the light sensing circuit output.

In an exemplary embodiment, the testing subunit provides a system for use in sample analysis. The system includes a biosensor reagent, wherein the biosensor reagent includes living biological cells; a reservoir card, having a long loop portion and a short loop portion, wherein the reservoir card stores the biosensor reagent; and a test cartridge base, wherein the test cartridge base is configured to accept the reservoir card. The test cartridge base further includes: (i) a reaction chamber having a central axis, wherein the reaction chamber has the shape of a revolved half ellipse; and (ii) an inlet channel connected to the reaction chamber, wherein the inlet channel is positioned above the reaction chamber at an angle of 15-60 degrees above the horizontal, wherein the inlet channel is offset from the central axis of the reaction chamber, and wherein upon introducing a sample to be analyzed into the test cartridge base through the inlet channel, the sample is homogeneously mixed with the biosensor reagent while minimizing damage to the living biological cells.

In another exemplary embodiment, the testing subunit provides a system for rapidly detecting the presence of an analyte in a biological sample. This system includes a biosensor reagent including at least one antibody specific for a predetermined analyte and a bioluminescent agent, wherein the at least one antibody is expressed on the surface of living, engineered lymphocytes and wherein the bioluminescent agent is expressed by the living, engineered lymphocytes, the biosensor reagent being operative to: (i) detect the presence of a specific analyte in a sample to be tested, and (ii) emit a detectable light signal when the biosensor reagent reacts with the sample and detects the presence of the specific analyte in the sample. A test cartridge is also included. The test cartridge further includes: (i) a reservoir card, wherein the reservoir card further includes the biosensor reagent; and (ii) a test cartridge base, wherein the test cartridge base is configured to accept the reservoir card. The test cartridge base further includes: a) a reaction chamber having a central axis, wherein the reaction chamber has the shape of a revolved half ellipse; b) an inlet channel connected to the reaction chamber, wherein the inlet channel is positioned above the reaction chamber at an angle of 15-60 degrees above the horizontal, and wherein the inlet channel is offset from the central axis of the reaction chamber; and c) wherein upon introducing the sample into the test cartridge base through the inlet channel, the sample is homogeneously mixed with the biosensor reagent while minimizing damage to the living, engineered lymphocytes and minimizing any bubbling of the mixed biosensor reagent and sample in the reaction chamber. A testing unit adapted to receive the test cartridge is also included. The testing unit including a sensor for detecting the detectable light signal emitted by the biosensor reagent upon reacting with the sample, the detection of the emitted detectable light signal being indicative of the presence of the analyte in the sample and, wherein detection of the specific analyte in the sample occurs in real time.

### Example Bioassay 1: Instant Addition of Antibodies

In an exemplary embodiment of the bioassay of the present invention, wherein the detector molecule is a soluble antibody, the soluble antibody and sample to be tested are mixed together immediately prior introduction of the biosensor cells to the test sample. In this embodiment, charged biosensor cells are centrifuged and concentrated to about 400,000 cells/180 µL (adequate for a single reaction) in the charging medium. A 180 µL (about 400,000 cells) aliquot of the charged biosensor cells is then loaded into the long loop portion of the reservoir card. For a positive control, 30 µL of anti-CD3ε antibody in RPMI media is loaded into the short loop portion of the reservoir card. The reservoir card is then locked into the test cartridge base. A 2 µL volume of an antibody (at 0.5 mg/mL) against the target pathogen, such as anti-*E. coli* O111 (wherein the target pathogen is *E. coli* O111), is mixed with 28 µL of the sample to be tested in the cartridge mixing chamber. The test cartridge base is inserted into the testing device and the charged biosensor cells are injected into the reaction chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

### Example Bioassay 2: Coating Biosensor Cells with Antibodies

In another exemplary embodiment of the bioassay of the present invention, wherein the detector molecule is a soluble antibody, the biosensor cells are coated with the soluble antibody for a period of time prior to mixing the sample to be tested with the biosensor cells. In this embodiment, charged biosensor cells are centrifuged and concentrated to about 400,000 cells/180 µL (adequate for one reaction) in the charging medium. A 180 µL (about 400,000 cells) aliquot of the biosensor cells is then mixed with a 2 µL volume of an antibody (at 0.5 mg/mL) against the target pathogen, such as anti-*E. coli* O111 (wherein the target pathogen is *E. coli* O111), in an Eppendorf tube. The biosensor cells mixed with the antibody are incubated at room temperature for 10 minutes and then loaded into the long loop portion of the reservoir card. For a positive control, 30 µL of anti-CD3ε antibody in RPMI media is loaded into the short loop portion of the reservoir card. The reservoir card is then locked into the test cartridge base. A 30 µL volume of the sample to be tested is added into the reaction chamber. The test cartridge base is inserted into the testing device and the biosensor cells are injected into the mixing chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

### Example Bioassay 3: Coating Analyte with Antibodies

In another exemplary embodiment of the bioassay of the present invention, wherein the detector molecule is a soluble antibody, the analyte (e.g., pathogenic bacteria) is coated with the soluble antibody for a period of time prior to mixing the sample to be tested with the biosensor. In this embodiment, charged biosensor cells are centrifuged and concentrated to about 400,000 cells/180 µL (adequate for one reaction) in the charging medium. A 180 µL (about 400,000 cells) aliquot of the biosensor cells is loaded into the long loop portion of the reservoir card. For a positive control, 30 µL of anti-CD3ε antibody in RPMI media is loaded into the short loop portion of the reservoir card. The reservoir card is then locked into the cartridge base. A 2 µL volume of an antibody (at 0.5 mg/mL) against the target pathogen, such as anti-*E. coli* O111 (wherein the target pathogen is *E. coli* O111), is mixed with 28 µL of the sample to be tested in an Eppendorf tube. The sample is incubated at room temperature for 10 minutes then added into the cartridge mixing chamber. The cartridge is inserted into the PMT and the biosensor cells are injected into the mixing chamber to initiate the reaction. The resulting signal is recorded for 4 to 8 minutes and at the end of the test period, the 30 µL of anti-CD3ε antibody is injected from the short loop of the reservoir into reaction chamber as a positive control reaction that is recorded for 2 minutes. As an alternative positive control, 30 µL of 0.61 mM Digitonin can be used rather than anti-CD3ε antibody. A negative control test can be performed using a predetermined pathogen that is not specific for the antibody being used.

The exemplary bioassays described herein may include other additives that reduce background noise and enhance signal. Using anti-CD3ε antibody as a positive control, the system has been demonstrated to detect fewer than 10 charged biosensor cells in a mixture of 50,000 uncharged biosensor cells. The biosensor itself has been demonstrated to detect 230 CFU of bacteria in a sample of 30 µL. In the bioassays described above, a proprietary monoclonal antibody (1F11) against *E. coli* O111 bacteria was used to detect *E. coli* O111 bacteria with *E. coli* 0157 being used as a negative control. *E. coli* 0157 was demonstrated to give negative results, thereby proving the specificity of the system. Numerous commercially available antibodies may also be used with the described bioassay. With regard to the proprietary monoclonal antibody (1F11), antibody analysis and selection of the monoclonal antibody was accomplished as described below.

Antibody production was determined by an ELISA performed in 96-well multiwell plates. Each well was coated with different LPS (*E. coli* 0157, *E. coli* 0127, *E. coli* O111, *E. coli* 026, *Klebsiella pnuemoniae, Salmonella enterioa* and naïve sera) or bacteria cells (*E. coli* 0157, *E. coli* O111, *E. coli* 26 and *E. coli* DH5α). Hybridoma supernatant from different clones of mAb 0157 or mAb O111 were added to the wells. Horseradish peroxidase-conjugated (HRP) goat anti-mouse IgG was used for detection (Appendix III.A.3). The two hybridoma clones (1B10 and 6G1) *of E. coli* 0157 exclusively recognize LPS *of E. coli* 0157 and *E. coli* 0157. The nine hybridoma clones *of E. coli* O111 specifically recognize LPS *of E. coli* O111 and *E. coli* O111. The clones from the highest optical density (OD) reading were chosen for validation, which was accomplished as described below.

The hybridoma cell pellets were collected and stored at -80°C before RNA extraction. The extracted RNA was used as a template for reverse transcription to cDNA, followed by nested PCR amplification. All positive PCR products were cloned into TA cloning vectors and sent for sequencing. The variable regions of the light chain and the heavy chain were determined after analysis of sequences. Four single chain antibodies (scFv) of 0157 (1B10) (customized mAb produced by FSC) and ATCC HB 10452, as well as two single chain antibodies of O111 produced by FSC (1F11 and 1F2) were recombinantly expressed and purified by Immobilized metal ion affinity chromatography (IMAC). The sequence of scFv was constructed as the following order: pel B secretion signal + amino acid Alanine + Histidine tag + amino acids Glycine-Serine-Serine-Glycine + TEV cleavage site + amino acids Glycine-Serine-Serine-Glycine + heavy chain variable region + Linker region Serine-Alanine-Aspartic Acid-Aspartic Acid-Alanine-lysine-lysine-Aspartic Acid-Alanine-Alanine-Lysine-Lysine-Aspartic Acid- Aspartic Acid-Alanine-Lysine-Lysine-Aspartic Acid- Aspartic Acid + light chain variable region. The purified scFvs were tested using multi-well plates coated with LPS of O157 or O111.

The purpose of this study was to investigate the interactions of monoclonal antibody (mAb) to whole bacterial cells (*E. coli* 0157 or *E. coli* O111), and to estimate the kinetic constant (KD) of antibody-bacterial interaction. In these assays, goat anti-*E. coli* 0157 polyclonal antibody, goat anti-*E. coli* O111 polyclonal antibody, three monoclonal antibodies (1022, 1024 and 1061) against *E. coli* 0157, and one monoclonal antibody against *E. coli* O111 were used. A CM5 sensor chip and amine coupling kit were also used. All assays were performed on a Biacore X100 instrument. In the protocol, one polyclonal antibody (against a select bacteria) was immobilized onto a CM5 sensor chip. The select bacteria was then bound followed by injection of a monoclonal antibody against the same bacteria in a continuous buffer flow. The interaction was monitored in real time. The relative binding of the antibody to each bacterium was recorded in resonance units (RUs). Results of the BIAcore analysis of binding an *E. coli* 0157 specific antibody (mAb FF754) to *E. coli* 0157 and *E. coli* O111 indicated that 0157 mAb was specific for its target antigen.

The present invention has been illustrated by the description of exemplary embodiments thereof. Additional advantages and modifications will readily appear to those skilled in the art.

## Claims

1. A system for rapid detection of analytes, comprising:
(a) a living, engineered biosensor cell, wherein the living, engineered biosensor cell is derived from a cellular component of the mammalian immune system;
(b) a reporter protein, wherein the reporter protein is engineered into and expressed by the living, engineered biosensor cell, and wherein the reporter protein emits a detectable signal in response to certain predetermined cytosolic changes in the living, engineered biosensor cell;
(c) a signal transduction pathway engineered into or occurring naturally within the living, engineered biosensor cell, wherein the signal transduction pathway controls a biological process within the cytosol of the living, engineered biosensor cell, and wherein the biological process, when it occurs, causes the reporter protein to emit a detectable signal;
(d) at least one type of detector molecule, wherein each detector molecule is adapted to bind to a specific analyte;
(e) at least one analyte, wherein the at least one analyte binds to the detector molecule that is specific to that analyte;
(f) a plurality of transmembrane, non-antibody signal transducing elements expressed by the living, engineered biosensor cell, wherein each signal transducing element is adapted to receive a detector molecule; and
(g) wherein upon the binding of a sufficient number of analytes to a sufficient number of detector molecules that are themselves bound to transmembrane non-antibody signal transducing elements, an aggregation of signal transducing elements occurs on the biosensor cell surface, the signal transduction pathway is activated, the biologicalprocess occurs, and the detectable signal is emitted by the reporter protein.

2. The system of claim 1, wherein the living, engineered cell is derived from a B-cell or a T-cell, the reporter protein is aequorin, the signal transduction pathway involves an increase in intracellular calcium, the detector molecule is an antibody that binds to a food-borne pathogen, the analyte is a food borne pathogen, and the plurality of signal transducing elements is adapted to receive an antibody through binding to a portion of a bacterial IgG binding protein.

3. The system of claim 1, wherein the biosensor cell is derived from a B cell or a T cell.

4. The system of claim 1, wherein the reporter protein is aequorin and the detectable signal is a flash of blue light.

5. The system of claim 1, wherein the biological process controlled by the signal transduction pathway further includes an increase in intracellular calcium.

6. The system of claim 1, wherein the non-antibody signal transducing element is a chimeric fusion protein expressed by the biosensor cell, and wherein the fusion protein further includes:
(a) at least one component of a protein that is adapted to bind to the at least one type of detector molecule; and
(b) at least one component of a receptor complex normally expressed on the surface of a type of immunoctye from which the biosensor cell was derived;
and wherein optionally
the at least one component of the protein that is adapted to bind to the at least one type of detector molecule is derived from a bacterial antibody binding protein;
or wherein optionally
the at least one component of the protein that is adapted to bind to the at least one type of detector molecule is an antibody binding domain derived from an FcR receptor protein or other receptor protein.

7. The system of claim 6, wherein the at least one component of the receptor complex normally expressed on the surface of the immunoctye is IgM; Igα/β; IgE; CD19; CD3ζ, or combinations thereof.

8. The system of claim 1, wherein the non-antibody signal transducing element is encoded by a DNA sequence having at least 95% identity to a DNA sequence selected from the group consisting of SEQ ID NOs: 1, 3, 5, 7, 9, 11, and 17.

9. The system of claim 1, wherein the non-antibody signal transducing element is a chimeric fusion protein having an amino acid sequence selected from the group consisting of SEQ ID NOs: 2, 4, 6, 8, 10, 12, and 18.

10. The system of claim 1, wherein the at least one detector molecule is a soluble antibody.

11. The system of claim 1, wherein the at least one detector molecule is soluble IgG.

12. The system of claim 1, wherein the analyte is a food-borne infectious agent.

13. The system of claim 1, wherein the analyte is either a bacterium or a virus.

14. A method for detecting an analyte, comprising contacting a sample containing the analyte with the system of claim 1 and detecting the analyte when a signal is emitted by the reporter protein.

15. A method for making the system of claim 1, comprising transfecting or transforming an immunocyte with one or more polynucleotides encoding elements (b), (c), and (f) of the system.

## Patentansprüche

1. System zur schnellen Detektion von Analyten, umfassend:
(a) eine lebende, manipulierte Biosensorzelle, wobei die lebende, manipulierte Biosensorzelle von einer zellulären Komponente des Säugerimmunsystems abgeleitet ist;
(b) ein Reporterprotein, wobei das Reporterprotein in die lebende, manipulierte Biosensorzelle eingeführt ist und von dieser exprimiert wird, und wobei das Reporterprotein ein detektierbares Signal als Reaktion auf bestimmte vorbestimmte zytosolische Veränderungen in der lebenden, manipulierten Biosensorzelle emittiert;
(c) einen Signaltransduktionsweg, der in die lebende, manipulierte Biosensorzelle eingeführt ist oder in ihr natürlich vorkommt, wobei der Signaltransduktionsweg einen biologischen Prozess im Zytosols der lebenden, manipulierten Biosensorzelle kontrolliert und wobei der biologische Prozess, wenn dieser auftritt, das Reporterprotein veranlasst, ein detektierbares Signal zu emittieren;
(d) mindestens eine Art von Detektormolekül, wobei jedes Detektormolekül zur Bindung an einen spezifischen Analyten angepasst ist;
(e) mindestens einen Analyten, wobei der mindestens eine Analyt an das Detektormolekül bindet, das für diesen Analyten spezifisch ist;
(f) eine Mehrzahl von Transmembran-Nicht-Antikörper-Signaltransduktionselementen, die von der lebenden, manipulierten Biosensorzelle exprimiert werden, wobei jedes Signaltransduktionselement angepasst ist, ein Detektormolekül zu empfangen; und
(g) wobei bei Bindung einer ausreichenden Anzahl von Analyten an eine ausreichende Anzahl von Detektormolekülen, die selbst an Transmembran-Nicht-Antikörper-Signaltransduktionselemente gebunden sind, eine Aggregation von Signaltransduktionselementen auf der Biosensorzelloberfläche auftritt, der Signaltransduktionsweg aktiviert wird, der biologische Prozess auftritt und das detektierbare Signal von dem Reporterprotein emittiert wird.

2. System nach Anspruch 1, wobei die lebende, manipulierte Zelle von einer B-Zelle oder einer T-Zelle abgeleitet ist, das Reporterprotein Aequorin ist, der Signaltransduktionsweg einen Anstieg von intrazellulärem Kalzium umfasst, das Detektormolekül ein Antikörper ist, der an ein durch Lebensmittel übertragenes Pathogen bindet, der Analyt ein durch Lebensmittel übertragenes Pathogen ist und die Mehrzahl von signalübertragenden Elementen angepasst ist, um einen Antikörper durch Bindung an einen Teil eines bakteriellen IgG-Bindungsproteins zu empfangen.

3. Das System nach Anspruch 1, wobei die Biosensorzelle von einer B-Zelle oder einer T-Zelle abgeleitet ist.

4. Das System nach Anspruch 1, wobei das Reporterprotein Aequorin ist und das detektierbare Signal ein Blitz blauen Lichts ist.

5. System nach Anspruch 1, wobei der biologische Prozess, der durch den Signaltransduktionsweg gesteuert wird, weiterhin einen Anstieg von intrazellulärem Kalzium umfasst.

6. System nach Anspruch 1, wobei das Nicht-Antikörper-Signaltransduktionselement ein chimäres Fusionsprotein ist, das von der Biosensorzelle exprimiert wird, und wobei das Fusionsprotein weiterhin umfasst:
(a) mindestens eine Komponente eines Proteins, welche angepasst ist um an den mindestens einen Typ von Detektormolekülen zu binden; und
(b) mindestens eine Komponente eines Rezeptorkomplexes, welcher normalerweise auf der Oberfläche eines Typs von Immunzellen, von dem die Biosensorzelle abgeleitet wurde, exprimiert wird;
und wobei optional die mindestens eine Komponente des Proteins, das zur Bindung an den mindestens einen Typ des Detektormoleküls angepasst ist, von einem bakteriellen Antikörperbindungsprotein abgeleitet ist;
oder wobei optional
die mindestens eine Komponente des Proteins, welches zur Bindung an den mindestens einen Typ von Detektormolekül angepasst ist, eine von einem FcR-Rezeptorprotein oder einem anderen Rezeptorprotein abgeleitete Antikörperbindungsdomäne ist.

7. Das System nach Anspruch 6, wobei die mindestens eine Komponente des Rezeptorkomplexes, der normalerweise auf der Oberfläche der Immunzelle exprimiert wird, IgM; Igα/β; IgE; CD19; CD3ζ oder eine Kombination derselben ist.

8. System nach Anspruch 1, wobei das Nicht-Antikörper-Signaltransduktionselement durch eine DNA-Sequenz kodiert wird, aufweisen mindestens 95% Identität mit einer DNA-Sequenz ausgewählt aus der Gruppe bestehend aus SEQ ID NOs: 1, 3, 5, 7, 9, 11 und 17.

9. System nach Anspruch 1, wobei das Nicht-Antikörper-Signaltransduktionselement ein chimäres Fusionsprotein ist, aufweisend eine Aminosäuresequenz die aus der Gruppe bestehend aus den SEQ ID NOs: 2, 4, 6, 8, 10, 12 und 18 ausgewählt ist.

10. Das System nach Anspruch 1, wobei das mindestens eine Detektormolekül ein löslicher Antikörper ist.

11. Das System nach Anspruch 1, wobei das mindestens eine Detektormolekül lösliches IgG ist.

12. System nach Anspruch 1, wobei der Analyt ein durch Lebensmittel übertragenes infektiöses Agen ist.

13. System nach Anspruch 1, wobei der Analyt entweder ein Bakterium oder ein Virus ist.

14. Verfahren zum Nachweis eines Analyten, umfassend das Inkontaktbringen einer den Analyten enthaltenden Probe mit dem System nach Anspruch 1 und Detektion des Analyten, wenn ein Signal von dem Reporterprotein emittiert wird.

15. Verfahren zur Herstellung des Systems nach Anspruch 1, umfassend das Transfizieren oder Transformieren einer Immunzelle mit einem oder mehreren Polynukleotiden, die die Elemente (b), (c) und (f) des Systems kodieren.

## Revendications

1. Système pour la détection rapide d'analytes, comprenant :
(a) une cellule biocapteur créée par ingénierie, vivante, laquelle cellule biocapteur créée par ingénierie, vivante, est dérivée d'un composant cellulaire du système immunitaire d'un mammifère ;
(b) une protéine rapportrice, laquelle protéine rapportrice est créée par ingénierie dans la cellule biocapteur créée par ingénierie, vivante, et est exprimée par cette dernière, et laquelle protéine rapportrice émet un signal détectable en réponse à certains changements prédéterminés dans le cytosol de la cellule biocapteur créée par ingénierie, vivante;
(c) une voie de transduction du signal créée par ingénierie dans ou survenant naturellement à l'intérieur de la cellule biocapteur créée par ingénierie, vivante, laquelle voie de transduction du signal contrôle un processus biologique à l'intérieur du cytosol de la cellule biocapteur créée par ingénierie, vivante, lequel processus biologique, lorsqu'il survient, amène la protéine rapportrice à émettre un signal détectable ;
(d) au moins un type de molécule de détection, chaque molécule de détection étant adaptée pour se lier à un analyte spécifique ;
(e) au moins un analyte, lequel au moins un analyte se lie à la molécule de détection qui est spécifique de cet analyte ;
(f) de multiples éléments transmembranaires de transduction du signal qui ne sont pas des anticorps, exprimés par la cellule biocapteur créée par ingénierie, vivante, chaque élément de transduction du signal étant adapté pour recevoir une molécule de détection ; et
(g) dans lequel, suite à la liaison d'un nombre suffisant d'analytes à un nombre suffisant de molécules de détection qui sont elles-mêmes liées à des éléments transmembranaires de transduction du signal qui ne sont pas des anticorps, il se produit une agrégation d'éléments de transduction du signal sur la surface de la cellule biocapteur, la voie de transduction du signal est activée, le processus biologique survient, et le signal détectable est émis par la protéine rapportrice.

2. Système selon la revendication 1, dans lequel la cellule vivante créée par ingénierie est dérivée d'une cellule B ou d'une cellule T, la protéine rapportrice est l'aéquorine, la voie de transduction du signal implique une augmentation du calcium intracellulaire, la molécule de détection est un anticorps qui se lie à un pathogène d'origine alimentaire, l'analyte est un pathogène d'origine alimentaire, et les multiples éléments de transduction du signal sont adaptés pour recevoir un anticorps par liaison à une partie d'une protéine bactérienne se liant à l'IgG.

3. Système selon la revendication 1, dans lequel la cellule biocapteur est dérivée d'une cellule B ou d'une cellule T.

4. Système selon la revendication 1, dans lequel la protéine rapportrice est l'aéquorine et le signal détectable est un flash de lumière bleue.

5. Système selon la revendication 1, dans lequel le processus biologique contrôlé par la voie de transduction du signal comprend en outre une augmentation du calcium intracellulaire.

6. Système selon la revendication 1, dans lequel l'élément de transduction du signal qui n'est pas un anticorps est une protéine de fusion chimère exprimée par la cellule biocapteur, et dans lequel la protéine de fusion contient en outre :
(a) au moins un composant d'une protéine qui est adaptée pour se lier à l'au moins un type de molécule de détection ; et
(b) au moins un composant d'un complexe de récepteur normalement exprimé sur la surface d'un type d'immunocyte dont dérive la cellule biocapteur ;
et dans lequel optionnellement
l'au moins un composant de la protéine qui est adaptée pour se lier à l'au moins un type de molécule de détection est dérivé d'une protéine bactérienne se liant à un anticorps ;
ou dans lequel optionnellement
l'au moins un composant de la protéine qui est adaptée pour se lier à l'au moins un type de molécule de détection est un domaine se liant à un anticorps dérivé d'une protéine de récepteur FcRou une autre protéine de récepteur.

7. Système selon la revendication 6, dans lequel l'au moins un composant du complexe de récepteur normalement exprimé sur la surface de l'immunocyte est IgM ; Ig□/ □ ; IgE ; CD19 ; CD3□, ou leurs combinaisons.

8. Système selon la revendication 1, dans lequel l'élément de transduction du signal qui n'est pas un anticorps est codé par une séquence d'ADN ayant une identité d'au moins 95 %avec une séquence d'ADN choisie dans le groupe constitué par les SEQ ID NO : 1, 3, 5, 7, 9, 11 et 17.

9. Système selon la revendication 1, dans lequel l'élément de transduction du signal qui n'est pas un anticorps est une protéine de fusion chimère ayant une séquence d'acides aminés choisie dans le groupe constitué par les SEQ ID NO : 2, 4, 6, 8, 10, 12 et 18.

10. Système selon la revendication 1, dans lequel l'au moins une molécule de détection est un anticorps soluble.

11. Système selon la revendication 1, dans lequel l'au moins une molécule de détection est une IgG soluble.

12. Système selon la revendication 1, dans lequel l'analyte est un agent infectieux d'origine alimentaire.

13. Système selon la revendication 1, dans lequel l'analyte est soit une bactérie soit un virus.

14. Procédé pour détecter un analyte, comprenant la mise en contact d'un échantillon contenant l'analyte avec le système de la revendication 1 et la détection de l'analyte quand un signal est émis par la protéine rapportrice.

15. Procédé pour produire le système de la revendication 1, comprenant la transfection ou la transformation d'un immunocyte avec un ou plusieurs polynucléotides codant les éléments (b), (c) et (f) du système.


### SEQUENCE LISTING

<110> Fundamental Solutions Corporation
   Zupancic, Thomas J.
   Zeng, Lingchun
   Vedamoorthy, Srikanth
   Lwande, Joel S.
   Joseph, Kittle D.
<120> Systems for the Rapid Detection of Analytes
<130> 35044-0006
<160> 19
<170> PatentIn version 3.5
<210> 1
   <211> 1929
   <212> DNA
   <213> Artificial Sequence
<220>
   <223> Chimeric Protein: BBP-IgM
<220>
   <221> CDS
   <222> (1)..(1929)
<400> 1
   atg gtg ctg cag acg caa gtg ttt atc tcc ctg ctg ctc tgg atc agc      48
   Met Val Leu Gln Thr Gln Val Phe Ile Ser Leu Leu Leu Trp Ile Ser
   1               5                   10                  15
   gga gcg tac ggc act tac aag ctg gtg atc aac ggt aaa acc ttg aag      96
   Gly Ala Tyr Gly Thr Tyr Lys Leu Val Ile Asn Gly Lys Thr Leu Lys
               20                  25                  30
   ggt gag acc acc act gag gca gtc gac gcc gcc act gcc gag aag gtc     144
   Gly Glu Thr Thr Thr Glu Ala Val Asp Ala Ala Thr Ala Glu Lys Val
           35                  40                  45
   ttt aaa cag tat gcc aat gat aac ggc gtg gac ggc gag tgg acc tac     192
   Phe Lys Gln Tyr Ala Asn Asp Asn Gly Val Asp Gly Glu Trp Thr Tyr
       50                  55                  60
   gat gac gcc act aag aca ttc act gtg act gaa aag ccc gag gtg att     240
   Asp Asp Ala Thr Lys Thr Phe Thr Val Thr Glu Lys Pro Glu Val Ile
   65                  70                  75                  80
   gac gcg tcc gaa ttg aca cct gcg gtg acc acc tac aaa ctg gtt atc     288
   Asp Ala Ser Glu Leu Thr Pro Ala Val Thr Thr Tyr Lys Leu Val Ile
                   85                  90                  95
   aac ggc aag act ctg aag ggc gag acc acc aca gag gca gtc gat gcc     336
   Asn Gly Lys Thr Leu Lys Gly Glu Thr Thr Thr Glu Ala Val Asp Ala
               100                 105                 110
   gcc acc gcc gag aag gtc ttc aag caa tat gcc aac gac aac ggg gtg     384
   Ala Thr Ala Glu Lys Val Phe Lys Gln Tyr Ala Asn Asp Asn Gly Val
           115                 120                 125
   gac ggg gag tgg acc tac gat gat gcc acc aag acc ttc acc gtg acc        432
   Asp Gly Glu Trp Thr Tyr Asp Asp Ala Thr Lys Thr Phe Thr Val Thr
       130                 135                 140
   gag aag ccc gaa gtg atc gat gcg agt gaa ctg act ccc gcc gtg aca        480
   Glu Lys Pro Glu Val Ile Asp Ala Ser Glu Leu Thr Pro Ala Val Thr
   145                 150                 155                 160
   ggt tct gct tct ggt tct ggt ggt tca gca tca gca cca act ttg ttt        528
   Gly Ser Ala Ser Gly Ser Gly Gly Ser Ala Ser Ala Pro Thr Leu Phe
                   165                 170                 175
   cca ctt gtc tca tgt gag aac tcg cca tcg gat acc tcg agc gta gcg        576
   Pro Leu Val Ser Cys Glu Asn Ser Pro Ser Asp Thr Ser Ser Val Ala
               180                 185                 190
   gtc gga tgt ctc gct caa gac ttt ctt ccg gac agc atc acg ttt tca        624
   Val Gly Cys Leu Ala Gln Asp Phe Leu Pro Asp Ser Ile Thr Phe Ser
           195                 200                 205
   tgg aag tat aag aac aat tcg gat att tcg agc acg cga gga ttt ccc        672
   Trp Lys Tyr Lys Asn Asn Ser Asp Ile Ser Ser Thr Arg Gly Phe Pro
       210                 215                 220
   agc gta ttg aga ggg gga aag tac gcg gca aca agc cag gtg ctg ctc        720
   Ser Val Leu Arg Gly Gly Lys Tyr Ala Ala Thr Ser Gln Val Leu Leu
   225                 230                 235                 240
   cca agc aag gat gtg atg cag ggc act gac gag cat gta gta tgc aag        768
   Pro Ser Lys Asp Val Met Gln Gly Thr Asp Glu His Val Val Cys Lys
                   245                 250                 255
   gta cag cac ccc aat gga aac aag gaa aag aat gtc cct ctg cct gta        816
   Val Gln His Pro Asn Gly Asn Lys Glu Lys Asn Val Pro Leu Pro Val
               260                 265                 270
   att gcc gag ctc cct cct aaa gtg tca gtg ttc gtg ccg ccc aga gat        864
   Ile Ala Glu Leu Pro Pro Lys Val Ser Val Phe Val Pro Pro Arg Asp
           275                 280                 285
   ggg ttc ttt gga aac ccg cga tcg aag tcg aaa ctg atc tgc cag gcc        912
   Gly Phe Phe Gly Asn Pro Arg Ser Lys Ser Lys Leu Ile Cys Gln Ala
       290                 295                 300
   acg gga ttc agc cct cgg cag att caa gtg tcg tgg ttg cgg gag gga        960
   Thr Gly Phe Ser Pro Arg Gln Ile Gln Val Ser Trp Leu Arg Glu Gly
   305                 310                 315                 320
   aaa cag gtg gga tcg ggg gtg acc aca gac cag gtg cag gcg gag gct       1008
   Lys Gln Val Gly Ser Gly Val Thr Thr Asp Gln Val Gln Ala Glu Ala
                   325                 330                 335
   aaa gaa agc ggt ccc acc aca tat aag gtc act tcc acc ctt act att       1056
   Lys Glu Ser Gly Pro Thr Thr Tyr Lys Val Thr Ser Thr Leu Thr Ile
               340                 345                 350
   aag gaa tcg gat tgg ttg tca cag tcg atg ttc aca tgt aga gtc gat       1104
   Lys Glu Ser Asp Trp Leu Ser Gln Ser Met Phe Thr Cys Arg Val Asp
           355                 360                 365
   cat cgc gga ctc acg ttt caa cag aac gcg tca tca atg tgt gta ccc       1152
   His Arg Gly Leu Thr Phe Gln Gln Asn Ala Ser Ser Met Cys Val Pro
       370                 375                 380
   gat caa gat acg gcg atc aga gta ttc gcc att ccg cct agc ttc gca        1200
   Asp Gln Asp Thr Ala Ile Arg Val Phe Ala Ile Pro Pro Ser Phe Ala
   385                 390                 395                 400
   tcc att ttt ctc acc aaa agc aca aag ctg aca tgt ctt gtg aca gac        1248
   Ser Ile Phe Leu Thr Lys Ser Thr Lys Leu Thr Cys Leu Val Thr Asp
                   405                 410                 415
   ctc aca acg tac gat tca gtc aca att tca tgg acc agg cag aat ggg        1296
   Leu Thr Thr Tyr Asp Ser Val Thr Ile Ser Trp Thr Arg Gln Asn Gly
               420                 425                 430
   gag gcg gta aag acg cac acc aac att tcg gaa agc cat ccc aac gcc        1344
   Glu Ala Val Lys Thr His Thr Asn Ile Ser Glu Ser His Pro Asn Ala
           435                 440                 445
   acg ttt tcg gcg gtc ggg gag gca tcg att tgt gag gac gat tgg aat        1392
   Thr Phe Ser Ala Val Gly Glu Ala Ser Ile Cys Glu Asp Asp Trp Asn
       450                 455                 460
   tca ggg gag cgc ttc aca tgc acg gtc act cac acg gat ctc cca tcc        1440
   Ser Gly Glu Arg Phe Thr Cys Thr Val Thr His Thr Asp Leu Pro Ser
   465                 470                 475                 480
   ccg ttg aag cag aca atc tcg cga ccc aaa ggt gtc gca ctg cac agg        1488
   Pro Leu Lys Gln Thr Ile Ser Arg Pro Lys Gly Val Ala Leu His Arg
                   485                 490                 495
   ccc gac gtc tac ctc ctg cct cct gcc agg gaa cag ctc aac ctc cgg        1536
   Pro Asp Val Tyr Leu Leu Pro Pro Ala Arg Glu Gln Leu Asn Leu Arg
               500                 505                 510
   gaa tca gca acg atc acg tgt ctt gta acc ggg ttt tca ccg gct gac        1584
   Glu Ser Ala Thr Ile Thr Cys Leu Val Thr Gly Phe Ser Pro Ala Asp
           515                 520                 525
   gtc ttt gtc caa tgg atg cag cgg gga caa ccc ttg tca cca gag aag        1632
   Val Phe Val Gln Trp Met Gln Arg Gly Gln Pro Leu Ser Pro Glu Lys
       530                 535                 540
   tat gtg aca tca gcg ccc atg ccc gag cca cag gct ccg ggt agg tat        1680
   Tyr Val Thr Ser Ala Pro Met Pro Glu Pro Gln Ala Pro Gly Arg Tyr
   545                 550                 555                 560
   ttt gcc cat tcc atc ctc act gtg tcc gag gaa gag tgg aac acc ggc        1728
   Phe Ala His Ser Ile Leu Thr Val Ser Glu Glu Glu Trp Asn Thr Gly
                   565                 570                 575
   gaa acg tac acg tgc gtc gta gca cac gaa gcg ttg ccc aat aga gtg        1776
   Glu Thr Tyr Thr Cys Val Val Ala His Glu Ala Leu Pro Asn Arg Val
               580                 585                 590
   act gag aga act gta gat aag tcc act gag ggc gaa gta agc gcg gat        1824
   Thr Glu Arg Thr Val Asp Lys Ser Thr Glu Gly Glu Val Ser Ala Asp
           595                 600                 605
   gaa gaa ggt ttc gaa aac ttg tgg gct aca gcg agc acg ttt atc gtg        1872
   Glu Glu Gly Phe Glu Asn Leu Trp Ala Thr Ala Ser Thr Phe Ile Val
       610                 615                 620
   ttg ttc ttg ctt tca ctc ttc tac tcc aca act gta acc ctg ttc aag        1920
   Leu Phe Leu Leu Ser Leu Phe Tyr Ser Thr Thr Val Thr Leu Phe Lys
   625                 630                 635                 640
   gtc aag tag                                                           1929
   Val Lys
<210> 2
   <211> 642
   <212> PRT
   <213> Artificial Sequence
<220>
   <223> Synthetic Construct
<400> 2
   Met Val Leu Gln Thr Gln Val Phe Ile Ser Leu Leu Leu Trp Ile Ser
   1               5                   10                  15
   Gly Ala Tyr Gly Thr Tyr Lys Leu Val Ile Asn Gly Lys Thr Leu Lys
               20                  25                  30
   Gly Glu Thr Thr Thr Glu Ala Val Asp Ala Ala Thr Ala Glu Lys Val
           35                  40                  45
   Phe Lys Gln Tyr Ala Asn Asp Asn Gly Val Asp Gly Glu Trp Thr Tyr
       50                  55                  60
   Asp Asp Ala Thr Lys Thr Phe Thr Val Thr Glu Lys Pro Glu Val Ile
   65                  70                  75                  80
   Asp Ala Ser Glu Leu Thr Pro Ala Val Thr Thr Tyr Lys Leu Val Ile
                   85                  90                  95
   Asn Gly Lys Thr Leu Lys Gly Glu Thr Thr Thr Glu Ala Val Asp Ala
               100                 105                 110
   Ala Thr Ala Glu Lys Val Phe Lys Gln Tyr Ala Asn Asp Asn Gly Val
           115                 120                 125
   Asp Gly Glu Trp Thr Tyr Asp Asp Ala Thr Lys Thr Phe Thr Val Thr
       130                 135                 140
   Glu Lys Pro Glu Val Ile Asp Ala Ser Glu Leu Thr Pro Ala Val Thr
   145                 150                 155                 160
   Gly Ser Ala Ser Gly Ser Gly Gly Ser Ala Ser Ala Pro Thr Leu Phe
                   165                 170                 175
   Pro Leu Val Ser Cys Glu Asn Ser Pro Ser Asp Thr Ser Ser Val Ala
               180                 185                 190
   Val Gly Cys Leu Ala Gln Asp Phe Leu Pro Asp Ser Ile Thr Phe Ser
           195                 200                 205
   Trp Lys Tyr Lys Asn Asn Ser Asp Ile Ser Ser Thr Arg Gly Phe Pro
       210                 215                 220
   Ser Val Leu Arg Gly Gly Lys Tyr Ala Ala Thr Ser Gln Val Leu Leu
   225                 230                 235                 240
   Pro Ser Lys Asp Val Met Gln Gly Thr Asp Glu His Val Val Cys Lys
                   245                 250                 255
   Val Gln His Pro Asn Gly Asn Lys Glu Lys Asn Val Pro Leu Pro Val
               260                 265                 270
   Ile Ala Glu Leu Pro Pro Lys Val Ser Val Phe Val Pro Pro Arg Asp
           275                 280                 285
   Gly Phe Phe Gly Asn Pro Arg Ser Lys Ser Lys Leu Ile Cys Gln Ala
       290                 295                 300
   Thr Gly Phe Ser Pro Arg Gln Ile Gln Val Ser Trp Leu Arg Glu Gly
   305                 310                 315                 320
   Lys Gln Val Gly Ser Gly Val Thr Thr Asp Gln Val Gln Ala Glu Ala
                   325                 330                 335
   Lys Glu Ser Gly Pro Thr Thr Tyr Lys Val Thr Ser Thr Leu Thr Ile
               340                 345                 350
   Lys Glu Ser Asp Trp Leu Ser Gln Ser Met Phe Thr Cys Arg Val Asp
           355                 360                 365
   His Arg Gly Leu Thr Phe Gln Gln Asn Ala Ser Ser Met Cys Val Pro
       370                 375                 380
   Asp Gln Asp Thr Ala Ile Arg Val Phe Ala Ile Pro Pro Ser Phe Ala
   385                 390                 395                 400
   Ser Ile Phe Leu Thr Lys Ser Thr Lys Leu Thr Cys Leu Val Thr Asp
                   405                 410                 415
   Leu Thr Thr Tyr Asp Ser Val Thr Ile Ser Trp Thr Arg Gln Asn Gly
               420                 425                 430
   Glu Ala Val Lys Thr His Thr Asn Ile Ser Glu Ser His Pro Asn Ala
               435                 440                 445
   Thr Phe Ser Ala Val Gly Glu Ala Ser Ile Cys Glu Asp Asp Trp Asn
       450                 455                 460
   Ser Gly Glu Arg Phe Thr Cys Thr Val Thr His Thr Asp Leu Pro Ser
   465                 470                 475                 480
   Pro Leu Lys Gln Thr Ile Ser Arg Pro Lys Gly Val Ala Leu His Arg
                   485                 490                 495
   Pro Asp Val Tyr Leu Leu Pro Pro Ala Arg Glu Gln Leu Asn Leu Arg
               500                 505                 510
   Glu Ser Ala Thr Ile Thr Cys Leu Val Thr Gly Phe Ser Pro Ala Asp
           515                 520                 525
   Val Phe Val Gln Trp Met Gln Arg Gly Gln Pro Leu Ser Pro Glu Lys
       530                 535                 540
   Tyr Val Thr Ser Ala Pro Met Pro Glu Pro Gln Ala Pro Gly Arg Tyr
   545                 550                 555                 560
   Phe Ala His Ser Ile Leu Thr Val Ser Glu Glu Glu Trp Asn Thr Gly
                   565                 570                 575
   Glu Thr Tyr Thr Cys Val Val Ala His Glu Ala Leu Pro Asn Arg Val
               580                 585                 590
   Thr Glu Arg Thr Val Asp Lys Ser Thr Glu Gly Glu Val Ser Ala Asp
           595                 600                 605
   Glu Glu Gly Phe Glu Asn Leu Trp Ala Thr Ala Ser Thr Phe Ile Val
       610                 615                 620
   Leu Phe Leu Leu Ser Leu Phe Tyr Ser Thr Thr Val Thr Leu Phe Lys
   625                 630                 635                 640
   Val Lys
<210> 3
   <211> 1884
   <212> DNA
   <213> Artificial Sequence
<220>
   <223> Chimeric Protein: BBP-IgAB
<220>
   <221> CDS
   <222> (1)..(1884)
<400> 3
   atg ccg ggt gga ccc gga gtg ctc cag gca ttg cct gca acc atc ttc          48
   Met Pro Gly Gly Pro Gly Val Leu Gln Ala Leu Pro Ala Thr Ile Phe
   1               5                   10                  15
   ctg ctg ttc ttg ttg tcc gcc gtc tac ctt gga cct ggt tgc cag gca          96
   Leu Leu Phe Leu Leu Ser Ala Val Tyr Leu Gly Pro Gly Cys Gln Ala
               20                  25                  30
   act tac aag ctg gtg atc aac ggt aaa acc ttg aag ggt gag acc acc         144
   Thr Tyr Lys Leu Val Ile Asn Gly Lys Thr Leu Lys Gly Glu Thr Thr
           35                  40                  45
   act gag gca gtc gac gcc gcc act gcc gag aag gtc ttt aaa cag tat         192
   Thr Glu Ala Val Asp Ala Ala Thr Ala Glu Lys Val Phe Lys Gln Tyr
       50                  55                  60
   gcc aat gat aac ggc gtg gac ggc gag tgg acc tac gat gac gcc act         240
   Ala Asn Asp Asn Gly Val Asp Gly Glu Trp Thr Tyr Asp Asp Ala Thr
   65                  70                  75                  80
   aag aca ttc act gtg act gaa aag ccc gag gtg att gac gcg tcc gaa         288
   Lys Thr Phe Thr Val Thr Glu Lys Pro Glu Val Ile Asp Ala Ser Glu
                   85                  90                  95
   ttg aca cct gcg gtg acc acc tac aaa ctg gtt atc aac ggc aag act         336
   Leu Thr Pro Ala Val Thr Thr Tyr Lys Leu Val Ile Asn Gly Lys Thr
               100                 105                 110
   ctg aag ggc gag acc acc aca gag gca gtc gat gcc gcc acc gcc gag         384
   Leu Lys Gly Glu Thr Thr Thr Glu Ala Val Asp Ala Ala Thr Ala Glu
           115                 120                 125
   aag gtc ttc aag caa tat gcc aac gac aac ggg gtg gac ggg gag tgg         432
   Lys Val Phe Lys Gln Tyr Ala Asn Asp Asn Gly Val Asp Gly Glu Trp
       130                 135                 140
   acc tac gat gat gcc acc aag acc ttc acc gtg acc gag aag ccc gaa         480
   Thr Tyr Asp Asp Ala Thr Lys Thr Phe Thr Val Thr Glu Lys Pro Glu
   145                 150                 155                 160
   gtg atc gat gcg agt gaa ctg act ccc gcc gtg aca ggt tct gct tcg         528
   Val Ile Asp Ala Ser Glu Leu Thr Pro Ala Val Thr Gly Ser Ala Ser
                   165                 170                 175
   ggc tca gga ctg tgg atg cat aag gtg cct gca tcg ctc atg gtg agc         576
   Gly Ser Gly Leu Trp Met His Lys Val Pro Ala Ser Leu Met Val Ser
               180                 185                 190
   ctg ggc gaa gat gca cat ttt cag tgt ccc cat aac agc tcc aac aac         624
   Leu Gly Glu Asp Ala His Phe Gln Cys Pro His Asn Ser Ser Asn Asn
           195                 200                 205
   gcg aac gtg acc tgg tgg cgg gtg ctc cat ggc aat tac acc tgg ccg         672
   Ala Asn Val Thr Trp Trp Arg Val Leu His Gly Asn Tyr Thr Trp Pro
       210                 215                 220
   cct gaa ttt ctc gga ccg gga gag gac ccg aat ggg acc ctt atc atc         720
   Pro Glu Phe Leu Gly Pro Gly Glu Asp Pro Asn Gly Thr Leu Ile Ile
   225                 230                 235                 240
   cag aac gtg aat aag tcc cac gga gga atc tac gtc tgc cgc gtg caa        768
   Gln Asn Val Asn Lys Ser His Gly Gly Ile Tyr Val Cys Arg Val Gln
                   245                 250                 255
   gag gga aat gag agc tac caa cag tca tgc gga acg tac ctc cgc gtc        816
   Glu Gly Asn Glu Ser Tyr Gln Gln Ser Cys Gly Thr Tyr Leu Arg Val
               260                 265                 270
   cgg cag cca cca ccg agg ccg ttc ctc gat atg gga gag gga act aag        864
   Arg Gln Pro Pro Pro Arg Pro Phe Leu Asp Met Gly Glu Gly Thr Lys
           275                 280                 285
   aac cgg atc att acc gcc gaa ggc atc atc ctc ctc ttc tgc gcc gtc        912
   Asn Arg Ile Ile Thr Ala Glu Gly Ile Ile Leu Leu Phe Cys Ala Val
       290                 295                 300
   gtg ccg ggg act ctg ctt ctg ttc cgg aaa agg tgg caa aac gaa aag        960
   Val Pro Gly Thr Leu Leu Leu Phe Arg Lys Arg Trp Gln Asn Glu Lys
   305                 310                 315                 320
   ctg ggt ctg gac gct ggg gac gaa tac gag gat gaa aac ttg tac gag       1008
   Leu Gly Leu Asp Ala Gly Asp Glu Tyr Glu Asp Glu Asn Leu Tyr Glu
                   325                 330                 335
   ggc ctg aat ctg gac gat tgc tcg atg tat gag gac att agc aga gga       1056
   Gly Leu Asn Leu Asp Asp Cys Ser Met Tyr Glu Asp Ile Ser Arg Gly
               340                 345                 350
   ctg cag ggt acc tac caa gac gtg gga agc ctg aac atc ggg gat gtg       1104
   Leu Gln Gly Thr Tyr Gln Asp Val Gly Ser Leu Asn Ile Gly Asp Val
           355                 360                 365
   cag ctc gag aaa cca agg aaa aga aga gct agc gaa gga cgc gga tca       1152
   Gln Leu Glu Lys Pro Arg Lys Arg Arg Ala Ser Glu Gly Arg Gly Ser
       370                 375                 380
   ctg ctt act tgc ggc gat gtc gaa gaa aat ccc gga cca atg gcc aga       1200
   Leu Leu Thr Cys Gly Asp Val Glu Glu Asn Pro Gly Pro Met Ala Arg
   385                 390                 395                 400
   ttg gcg ctg tcg ccg gtc cct tcg cac tgg atg gtg gcc ctg ctg ctc       1248
   Leu Ala Leu Ser Pro Val Pro Ser His Trp Met Val Ala Leu Leu Leu
                   405                 410                 415
   ttg ctt tcg gcg gct gag ccg gtg cca gcg gct aga tcg gag gac cgc       1296
   Leu Leu Ser Ala Ala Glu Pro Val Pro Ala Ala Arg Ser Glu Asp Arg
               420                 425                 430
   tac aga aat ccg aag ggt tcc gct tgc tca cgc atc tgg caa tca cca       1344
   Tyr Arg Asn Pro Lys Gly Ser Ala Cys Ser Arg Ile Trp Gln Ser Pro
           435                 440                 445
   cgc ttc atc gcg cgc aaa cgc ggc ttc act gtc aag atg cac tgc tat       1392
   Arg Phe Ile Ala Arg Lys Arg Gly Phe Thr Val Lys Met His Cys Tyr
       450                 455                 460
   atg aac tcg gcc agc ggg aat gtg tcg tgg ctg tgg aag cag gaa atg       1440
   Met Asn Ser Ala Ser Gly Asn Val Ser Trp Leu Trp Lys Gln Glu Met
   465                 470                 475                 480
   gac gag aat ccg cag caa ctg aaa ctg gag aag ggc cgg atg gaa gaa      1488
   Asp Glu Asn Pro Gln Gln Leu Lys Leu Glu Lys Gly Arg Met Glu Glu
                   485                 490                 495
   tcc cag aat gag tcg ctg gcc acc ctt act atc caa ggt atc cgg ttt      1536
   Ser Gln Asn Glu Ser Leu Ala Thr Leu Thr Ile Gln Gly Ile Arg Phe
               500                 505                 510
   gaa gat aac ggg atc tac ttc tgt caa cag aag tgt aac aac act tca      1584
   Glu Asp Asn Gly Ile Tyr Phe Cys Gln Gln Lys Cys Asn Asn Thr Ser
           515                 520                 525
   gag gtg tac cag gga tgc ggc acc gaa ctc cgc gtc atg gga ttc tcc      1632
   Glu Val Tyr Gln Gly Cys Gly Thr Glu Leu Arg Val Met Gly Phe Ser
       530                 535                 540
   acc ctc gcc caa ctg aag cag cgc aac acg ctg aag gac ggc atc att      1680
   Thr Leu Ala Gln Leu Lys Gln Arg Asn Thr Leu Lys Asp Gly Ile Ile
   545                 550                 555                 560
   atg atc cag acc ctg ctg atc atc ctg ttc atc att gtg ccg atc ttt      1728
   Met Ile Gln Thr Leu Leu Ile Ile Leu Phe Ile Ile Val Pro Ile Phe
                   565                 570                 575
   ctg ctc ttg gat aag gac gac tcg aaa gcc gga atg gaa gag gac cac      1776
   Leu Leu Leu Asp Lys Asp Asp Ser Lys Ala Gly Met Glu Glu Asp His
               580                 585                 590
   acg tac gaa ggt ctg gac atc gac caa act gcg act tac gag gac att      1824
   Thr Tyr Glu Gly Leu Asp Ile Asp Gln Thr Ala Thr Tyr Glu Asp Ile
           595                 600                 605
   gtg acc ctc cgg act ggc gaa gtc aag tgg tcc gtg ggt gaa cac cct      1872
   Val Thr Leu Arg Thr Gly Glu Val Lys Trp Ser Val Gly Glu His Pro
       610                 615                 620
   gga cag gaa tag                                                      1884
   Gly Gln Glu
   625
<210> 4
   <211> 627
   <212> PRT
   <213> Artificial Sequence
<220>
   <223> Synthetic Construct
<400> 4
   Met Pro Gly Gly Pro Gly Val Leu Gln Ala Leu Pro Ala Thr Ile Phe
   1               5                   10                  15
   Leu Leu Phe Leu Leu Ser Ala Val Tyr Leu Gly Pro Gly Cys Gln Ala
               20                  25                  30
   Thr Tyr Lys Leu Val Ile Asn Gly Lys Thr Leu Lys Gly Glu Thr Thr
           35                  40                  45
   Thr Glu Ala Val Asp Ala Ala Thr Ala Glu Lys Val Phe Lys Gln Tyr
       50                  55                  60
   Ala Asn Asp Asn Gly Val Asp Gly Glu Trp Thr Tyr Asp Asp Ala Thr
   65                  70                  75                  80
   Lys Thr Phe Thr Val Thr Glu Lys Pro Glu Val Ile Asp Ala Ser Glu
                   85                  90                  95
   Leu Thr Pro Ala Val Thr Thr Tyr Lys Leu Val Ile Asn Gly Lys Thr
               100                 105                 110
   Leu Lys Gly Glu Thr Thr Thr Glu Ala Val Asp Ala Ala Thr Ala Glu
           115                 120                 125
   Lys Val Phe Lys Gln Tyr Ala Asn Asp Asn Gly Val Asp Gly Glu Trp
       130                 135                 140
   Thr Tyr Asp Asp Ala Thr Lys Thr Phe Thr Val Thr Glu Lys Pro Glu
   145                 150                 155                 160
   Val Ile Asp Ala Ser Glu Leu Thr Pro Ala Val Thr Gly Ser Ala Ser
                   165                 170                 175
   Gly Ser Gly Leu Trp Met His Lys Val Pro Ala Ser Leu Met Val Ser
               180                 185                 190
   Leu Gly Glu Asp Ala His Phe Gln Cys Pro His Asn Ser Ser Asn Asn
           195                 200                 205
   Ala Asn Val Thr Trp Trp Arg Val Leu His Gly Asn Tyr Thr Trp Pro
       210                 215                 220
   Pro Glu Phe Leu Gly Pro Gly Glu Asp Pro Asn Gly Thr Leu Ile Ile
   225                 230                 235                 240
   Gln Asn Val Asn Lys Ser His Gly Gly Ile Tyr Val Cys Arg Val Gln
                   245                 250                 255
   Glu Gly Asn Glu Ser Tyr Gln Gln Ser Cys Gly Thr Tyr Leu Arg Val
               260                 265                 270
   Arg Gln Pro Pro Pro Arg Pro Phe Leu Asp Met Gly Glu Gly Thr Lys
           275                 280                 285
   Asn Arg Ile Ile Thr Ala Glu Gly Ile Ile Leu Leu Phe Cys Ala Val
       290                 295                 300
   Val Pro Gly Thr Leu Leu Leu Phe Arg Lys Arg Trp Gln Asn Glu Lys
   305                 310                 315                 320
   Leu Gly Leu Asp Ala Gly Asp Glu Tyr Glu Asp Glu Asn Leu Tyr Glu
                   325                 330                 335
   Gly Leu Asn Leu Asp Asp Cys Ser Met Tyr Glu Asp Ile Ser Arg Gly
               340                 345                 350
   Leu Gln Gly Thr Tyr Gln Asp Val Gly Ser Leu Asn Ile Gly Asp Val
           355                 360                 365
   Gln Leu Glu Lys Pro Arg Lys Arg Arg Ala Ser Glu Gly Arg Gly Ser
       370                 375                 380
   Leu Leu Thr Cys Gly Asp Val Glu Glu Asn Pro Gly Pro Met Ala Arg
   385                 390                 395                 400
   Leu Ala Leu Ser Pro Val Pro Ser His Trp Met Val Ala Leu Leu Leu
                   405                 410                 415
   Leu Leu Ser Ala Ala Glu Pro Val Pro Ala Ala Arg Ser Glu Asp Arg
               420                 425                 430
   Tyr Arg Asn Pro Lys Gly Ser Ala Cys Ser Arg Ile Trp Gln Ser Pro
           435                 440                 445
   Arg Phe Ile Ala Arg Lys Arg Gly Phe Thr Val Lys Met His Cys Tyr
       450                 455                 460
   Met Asn Ser Ala Ser Gly Asn Val Ser Trp Leu Trp Lys Gln Glu Met
   465                 470                 475                 480
   Asp Glu Asn Pro Gln Gln Leu Lys Leu Glu Lys Gly Arg Met Glu Glu
                   485                 490                 495
   Ser Gln Asn Glu Ser Leu Ala Thr Leu Thr Ile Gln Gly Ile Arg Phe
               500                 505                 510
   Glu Asp Asn Gly Ile Tyr Phe Cys Gln Gln Lys Cys Asn Asn Thr Ser
           515                 520                 525
   Glu Val Tyr Gln Gly Cys Gly Thr Glu Leu Arg Val Met Gly Phe Ser
       530                 535                 540
   Thr Leu Ala Gln Leu Lys Gln Arg Asn Thr Leu Lys Asp Gly Ile Ile
   545                 550                 555                 560
   Met Ile Gln Thr Leu Leu Ile Ile Leu Phe Ile Ile Val Pro Ile Phe
                   565                 570                 575
   Leu Leu Leu Asp Lys Asp Asp Ser Lys Ala Gly Met Glu Glu Asp His
               580                 585                 590
   Thr Tyr Glu Gly Leu Asp Ile Asp Gln Thr Ala Thr Tyr Glu Asp Ile
           595                 600                 605
   Val Thr Leu Arg Thr Gly Glu Val Lys Trp Ser Val Gly Glu His Pro
       610                 615                 620
   Gly Gln Glu
   625
<210> 5
   <211> 936
   <212> DNA
   <213> Artificial Sequence
<220>
   <223> Chimeric Protein: BBP-CD3Z
<220>
   <221> CDS
   <222> (1)..(936)
<400> 5
   atg aag tgg aag gcg ctt ttc acc gcg gcc atc ctg cag gca cag ttg       48
   Met Lys Trp Lys Ala Leu Phe Thr Ala Ala Ile Leu Gln Ala Gln Leu
   1               5                   10                  15
   ccg att aca gag gca act tac aag ctg gtg atc aac ggt aaa acc ttg       96
   Pro Ile Thr Glu Ala Thr Tyr Lys Leu Val Ile Asn Gly Lys Thr Leu
               20                  25                  30
   aag ggt gag acc acc act gag gca gtc gac gcc gcc act gcc gag aag      144
   Lys Gly Glu Thr Thr Thr Glu Ala Val Asp Ala Ala Thr Ala Glu Lys
           35                  40                  45
   gtc ttt aaa cag tat gcc aat gat aac ggc gtg gac ggc gag tgg acc      192
   Val Phe Lys Gln Tyr Ala Asn Asp Asn Gly Val Asp Gly Glu Trp Thr
       50                  55                  60
   tac gat gac gcc act aag aca ttc act gtg act gaa aag ccc gag gtg      240
   Tyr Asp Asp Ala Thr Lys Thr Phe Thr Val Thr Glu Lys Pro Glu Val
   65                  70                  75                  80
   att gac gcg tcc gaa ttg aca cct gcg gtg acc acc tac aaa ctg gtt      288
   Ile Asp Ala Ser Glu Leu Thr Pro Ala Val Thr Thr Tyr Lys Leu Val
                   85                  90                  95
   atc aac ggc aag act ctg aag ggc gag acc acc aca gag gca gtc gat      336
   Ile Asn Gly Lys Thr Leu Lys Gly Glu Thr Thr Thr Glu Ala Val Asp
               100                 105                 110
   gcc gcc acc gcc gag aag gtc ttc aag caa tat gcc aac gac aac ggg      384
   Ala Ala Thr Ala Glu Lys Val Phe Lys Gln Tyr Ala Asn Asp Asn Gly
           115                 120                 125
   gtg gac ggg gag tgg acc tac gat gat gcc acc aag acc ttc acc gtg      432
   Val Asp Gly Glu Trp Thr Tyr Asp Asp Ala Thr Lys Thr Phe Thr Val
       130                 135                 140
   acc gag aag ccc gaa gtg atc gat gcg agt gaa ctg act ccc gcc gtg      480
   Thr Glu Lys Pro Glu Val Ile Asp Ala Ser Glu Leu Thr Pro Ala Val
   145                 150                 155                 160
   aca ggt tct gct tct ggt tct ggt cag agc ttt ggc ctg ctg gat ccc      528
   Thr Gly Ser Ala Ser Gly Ser Gly Gln Ser Phe Gly Leu Leu Asp Pro
                   165                 170                 175
   aaa ctc tgc tac ctg ctg gat gga atc ctc ttc atc tat ggt gtc att      576
   Lys Leu Cys Tyr Leu Leu Asp Gly Ile Leu Phe Ile Tyr Gly Val Ile
               180                 185                 190
   ctc act gcc ttg ttc ctg aga gtg aag ttc agc agg agc gca gac gcc      624
   Leu Thr Ala Leu Phe Leu Arg Val Lys Phe Ser Arg Ser Ala Asp Ala
           195                 200                 205
   ccc gcg tac cag cag ggc cag aac cag ctc tat aac gag ctc aat cta      672
   Pro Ala Tyr Gln Gln Gly Gln Asn Gln Leu Tyr Asn Glu Leu Asn Leu
       210                 215                 220
   gga cga aga gag gag tac gat gtt ttg gac aag aga cgt ggc cgg gac      720
   Gly Arg Arg Glu Glu Tyr Asp Val Leu Asp Lys Arg Arg Gly Arg Asp
   225                 230                 235                 240
   cct gag atg ggg gga aag ccg cag aga agg aag aac cct cag gaa ggc      768
   Pro Glu Met Gly Gly Lys Pro Gln Arg Arg Lys Asn Pro Gln Glu Gly
                   245                 250                 255
   ctg tac aat gaa ctg cag aaa gat aag atg gcg gag gcc tac agt gag      816
   Leu Tyr Asn Glu Leu Gln Lys Asp Lys Met Ala Glu Ala Tyr Ser Glu
               260                 265                 270
   att ggg atg aaa ggc gag cgc cgg agg ggc aag ggg cac gat ggc ctt      864
   Ile Gly Met Lys Gly Glu Arg Arg Arg Gly Lys Gly His Asp Gly Leu
           275                 280                 285
   tac cag ggt ctc agt aca gcc acc aag gac acc tac gac gcc ctt cac      912
   Tyr Gln Gly Leu Ser Thr Ala Thr Lys Asp Thr Tyr Asp Ala Leu His
       290                 295                 300
   atg cag gcc ctg ccc cct cgc taa                                      936
   Met Gln Ala Leu Pro Pro Arg
   305                 310
<210> 6
   <211> 311
   <212> PRT
   <213> Artificial Sequence
<220>
   <223> Synthetic Construct
<400> 6
   Met Lys Trp Lys Ala Leu Phe Thr Ala Ala Ile Leu Gln Ala Gln Leu
   1               5                   10                  15
   Pro Ile Thr Glu Ala Thr Tyr Lys Leu Val Ile Asn Gly Lys Thr Leu
               20                  25                  30
   Lys Gly Glu Thr Thr Thr Glu Ala Val Asp Ala Ala Thr Ala Glu Lys
           35                  40                  45
   Val Phe Lys Gln Tyr Ala Asn Asp Asn Gly Val Asp Gly Glu Trp Thr
       50                  55                  60
   Tyr Asp Asp Ala Thr Lys Thr Phe Thr Val Thr Glu Lys Pro Glu Val
   65                  70                  75                  80
   Ile Asp Ala Ser Glu Leu Thr Pro Ala Val Thr Thr Tyr Lys Leu Val
                   85                  90                  95
   Ile Asn Gly Lys Thr Leu Lys Gly Glu Thr Thr Thr Glu Ala Val Asp
               100                 105                 110
   Ala Ala Thr Ala Glu Lys Val Phe Lys Gln Tyr Ala Asn Asp Asn Gly
           115                 120                 125
   Val Asp Gly Glu Trp Thr Tyr Asp Asp Ala Thr Lys Thr Phe Thr Val
       130                 135                 140
   Thr Glu Lys Pro Glu Val Ile Asp Ala Ser Glu Leu Thr Pro Ala Val
   145                 150                 155                 160
   Thr Gly Ser Ala Ser Gly Ser Gly Gln Ser Phe Gly Leu Leu Asp Pro
                   165                 170                 175
   Lys Leu Cys Tyr Leu Leu Asp Gly Ile Leu Phe Ile Tyr Gly Val Ile
               180                 185                 190
   Leu Thr Ala Leu Phe Leu Arg Val Lys Phe Ser Arg Ser Ala Asp Ala
           195                 200                 205
   Pro Ala Tyr Gln Gln Gly Gln Asn Gln Leu Tyr Asn Glu Leu Asn Leu
       210                 215                 220
   Gly Arg Arg Glu Glu Tyr Asp Val Leu Asp Lys Arg Arg Gly Arg Asp
   225                 230                 235                 240
   Pro Glu Met Gly Gly Lys Pro Gln Arg Arg Lys Asn Pro Gln Glu Gly
                   245                 250                 255
   Leu Tyr Asn Glu Leu Gln Lys Asp Lys Met Ala Glu Ala Tyr Ser Glu
               260                 265                 270
   Ile Gly Met Lys Gly Glu Arg Arg Arg Gly Lys Gly His Asp Gly Leu
           275                 280                 285
   Tyr Gln Gly Leu Ser Thr Ala Thr Lys Asp Thr Tyr Asp Ala Leu His
       290                 295                 300
   Met Gln Ala Leu Pro Pro Arg
   305                 310
<210> 7
   <211> 2340
   <212> DNA
   <213> Artificial Sequence
<220>
   <223> Chimeric Protein: FcgammaRl-IgM
<220>
   <221> CDS
   <222> (1)..(2340)
<400> 7
   atg att ctt acc agc ttt gga gat gac atg tgg ctt cta aca act ctg        48
   Met Ile Leu Thr Ser Phe Gly Asp Asp Met Trp Leu Leu Thr Thr Leu
   1               5                   10                  15
   cta ctt tgg gtt cca gtc ggt ggg gaa gtg gtt aat gcc acc aag gct        96
   Leu Leu Trp Val Pro Val Gly Gly Glu Val Val Asn Ala Thr Lys Ala
               20                  25                  30
   gtg atc acc ttg cag cct cca tgg gtc agt att ttc cag aag gaa aat       144
   Val Ile Thr Leu Gln Pro Pro Trp Val Ser Ile Phe Gln Lys Glu Asn
           35                  40                  45
   gtc act tta tgg tgt gag ggg cct cac ctg cct gga gac agt tcc aca       192
   Val Thr Leu Trp Cys Glu Gly Pro His Leu Pro Gly Asp Ser Ser Thr
       50                  55                  60
   caa tgg ttt atc aac gga aca gcc gtt cag atc tcc acg cct agt tat       240
   Gln Trp Phe Ile Asn Gly Thr Ala Val Gln Ile Ser Thr Pro Ser Tyr
   65                  70                  75                  80
   agc atc cca gag gcc agt ttt cag gac agt ggc gaa tac agg tgt cag       288
   Ser Ile Pro Glu Ala Ser Phe Gln Asp Ser Gly Glu Tyr Arg Cys Gln
                   85                  90                  95
   ata ggt tcc tca atg cca agt gac cct gtg cag ttg caa atc cac aat       336
   Ile Gly Ser Ser Met Pro Ser Asp Pro Val Gln Leu Gln Ile His Asn
               100                 105                 110
   gat tgg ctg cta ctc cag gcc tcc cgc aga gtc ctc aca gaa gga gaa       384
   Asp Trp Leu Leu Leu Gln Ala Ser Arg Arg Val Leu Thr Glu Gly Glu
           115                 120                 125
   ccc ctg gcc ttg agg tgt cac gga tgg aag aat aaa ctg gtg tac aat        432
   Pro Leu Ala Leu Arg Cys His Gly Trp Lys Asn Lys Leu Val Tyr Asn
       130                 135                 140
   gtg gtt ttc tat aga aat gga aaa tcc ttt cag ttt tct tca gat tcg        480
   Val Val Phe Tyr Arg Asn Gly Lys Ser Phe Gln Phe Ser Ser Asp Ser
   145                 150                 155                 160
   gag gtc gcc att ctg aaa acc aac ctg agt cac agc ggc atc tac cac        528
   Glu Val Ala Ile Leu Lys Thr Asn Leu Ser His Ser Gly Ile Tyr His
                   165                 170                 175
   tgc tca ggc acg gga aga cac cgc tac aca tct gca gga gtg tcc atc        576
   Cys Ser Gly Thr Gly Arg His Arg Tyr Thr Ser Ala Gly Val Ser Ile
               180                 185                 190
   acg gtg aaa gag ctg ttt acc acg cca gtg ctg aga gca tcc gtg tca        624
   Thr Val Lys Glu Leu Phe Thr Thr Pro Val Leu Arg Ala Ser Val Ser
           195                 200                 205
   tct ccc ttc ccg gag ggg agt ctg gtc acc ctg aac tgt gag acg aat        672
   Ser Pro Phe Pro Glu Gly Ser Leu Val Thr Leu Asn Cys Glu Thr Asn
       210                 215                 220
   ttg ctc ctg cag aga ccc ggc tta cag ctt cac ttc tcc ttc tac gtg        720
   Leu Leu Leu Gln Arg Pro Gly Leu Gln Leu His Phe Ser Phe Tyr Val
   225                 230                 235                 240
   ggc agc aag atc ctg gag tac agg aac aca tcc tca gag tac cat ata        768
   Gly Ser Lys Ile Leu Glu Tyr Arg Asn Thr Ser Ser Glu Tyr His Ile
                   245                 250                 255
   gca agg gcg gaa aga gaa gat gct gga ttc tac tgg tgt gag gta gcc        816
   Ala Arg Ala Glu Arg Glu Asp Ala Gly Phe Tyr Trp Cys Glu Val Ala
               260                 265                 270
   acg gag gac agc agt gtc ctt aag cgc agc cct gag ttg gag ctc caa        864
   Thr Glu Asp Ser Ser Val Leu Lys Arg Ser Pro Glu Leu Glu Leu Gln
           275                 280                 285
   gtg ctt ggt ccc cag tca tca gct cct ggt tct gct tct ggt tct ggt        912
   Val Leu Gly Pro Gln Ser Ser Ala Pro Gly Ser Ala Ser Gly Ser Gly
       290                 295                 300
   ggt tca gca tca gca cca act ttg ttt cca ctt gtc tca tgt gag aac        960
   Gly Ser Ala Ser Ala Pro Thr Leu Phe Pro Leu Val Ser Cys Glu Asn
   305                 310                 315                 320
   tcg cca tcg gat acc tcg agc gta gcg gtc gga tgt ctc gct caa gac       1008
   Ser Pro Ser Asp Thr Ser Ser Val Ala Val Gly Cys Leu Ala Gln Asp
                   325                 330                 335
   ttt ctt ccg gac agc atc acg ttt tca tgg aag tat aag aac aat tcg       1056
   Phe Leu Pro Asp Ser Ile Thr Phe Ser Trp Lys Tyr Lys Asn Asn Ser
               340                 345                 350
   gat att tcg agc acg cga gga ttt ccc agc gta ttg aga ggg gga aag       1104
   Asp Ile Ser Ser Thr Arg Gly Phe Pro Ser Val Leu Arg Gly Gly Lys
           355                 360                 365
   tac gcg gca aca agc cag gtg ctg ctc cca agc aag gat gtg atg cag       1152
   Tyr Ala Ala Thr Ser Gln Val Leu Leu Pro Ser Lys Asp Val Met Gln
           370                 375                 380
   ggc act gac gag cat gta gta tgc aag gta cag cac ccc aat gga aac          1200
   Gly Thr Asp Glu His Val Val Cys Lys Val Gln His Pro Asn Gly Asn
   385                 390                 395                 400
   aag gaa aag aat gtc cct ctg cct gta att gcc gag ctc cct cct aaa          1248
   Lys Glu Lys Asn Val Pro Leu Pro Val Ile Ala Glu Leu Pro Pro Lys
                   405                 410                 415
   gtg tca gtg ttc gtg ccg ccc aga gat ggg ttc ttt gga aac ccg cga          1296
   Val Ser Val Phe Val Pro Pro Arg Asp Gly Phe Phe Gly Asn Pro Arg
               420                 425                 430
   tcg aag tcg aaa ctg atc tgc cag gcc acg gga ttc agc cct cgg cag          1344
   Ser Lys Ser Lys Leu Ile Cys Gln Ala Thr Gly Phe Ser Pro Arg Gln
           435                 440                 445
   att caa gtg tcg tgg ttg cgg gag gga aaa cag gtg gga tcg ggg gtg          1392
   Ile Gln Val Ser Trp Leu Arg Glu Gly Lys Gln Val Gly Ser Gly Val
       450                 455                 460
   acc aca gac cag gtg cag gcg gag gct aaa gaa agc ggt ccc acc aca          1440
   Thr Thr Asp Gln Val Gln Ala Glu Ala Lys Glu Ser Gly Pro Thr Thr
   465                 470                 475                 480
   tat aag gtc act tcc acc ctt act att aag gaa tcg gat tgg ttg tca          1488
   Tyr Lys Val Thr Ser Thr Leu Thr Ile Lys Glu Ser Asp Trp Leu Ser
                   485                 490                 495
   cag tcg atg ttc aca tgt aga gtc gat cat cgc gga ctc acg ttt caa          1536
   Gln Ser Met Phe Thr Cys Arg Val Asp His Arg Gly Leu Thr Phe Gln
               500                 505                 510
   cag aac gcg tca tca atg tgt gta ccc gat caa gat acg gcg atc aga          1584
   Gln Asn Ala Ser Ser Met Cys Val Pro Asp Gln Asp Thr Ala Ile Arg
           515                 520                 525
   gta ttc gcc att ccg cct agc ttc gca tcc att ttt ctc acc aaa agc          1632
   Val Phe Ala Ile Pro Pro Ser Phe Ala Ser Ile Phe Leu Thr Lys Ser
       530                 535                 540
   aca aag ctg aca tgt ctt gtg aca gac ctc aca acg tac gat tca gtc          1680
   Thr Lys Leu Thr Cys Leu Val Thr Asp Leu Thr Thr Tyr Asp Ser Val
   545                 550                 555                 560
   aca att tca tgg acc agg cag aat ggg gag gcg gta aag acg cac acc          1728
   Thr Ile Ser Trp Thr Arg Gln Asn Gly Glu Ala Val Lys Thr His Thr
                   565                 570                 575
   aac att tcg gaa agc cat ccc aac gcc acg ttt tcg gcg gtc ggg gag          1776
   Asn Ile Ser Glu Ser His Pro Asn Ala Thr Phe Ser Ala Val Gly Glu
               580                 585                 590
   gca tcg att tgt gag gac gat tgg aat tca ggg gag cgc ttc aca tgc          1824
   Ala Ser Ile Cys Glu Asp Asp Trp Asn Ser Gly Glu Arg Phe Thr Cys
           595                 600                 605
   acg gtc act cac acg gat ctc cca tcc ccg ttg aag cag aca atc tcg          1872
   Thr Val Thr His Thr Asp Leu Pro Ser Pro Leu Lys Gln Thr Ile Ser
       610                 615                 620
   cga ccc aaa ggt gtc gca ctg cac agg ccc gac gtc tac ctc ctg cct          1920
   Arg Pro Lys Gly Val Ala Leu His Arg Pro Asp Val Tyr Leu Leu Pro
   625                 630                 635                 640
   cct gcc agg gaa cag ctc aac ctc cgg gaa tca gca acg atc acg tgt      1968
   Pro Ala Arg Glu Gln Leu Asn Leu Arg Glu Ser Ala Thr Ile Thr Cys
                   645                 650                 655
   ctt gta acc ggg ttt tca ccg gct gac gtc ttt gtc caa tgg atg cag      2016
   Leu Val Thr Gly Phe Ser Pro Ala Asp Val Phe Val Gln Trp Met Gln
               660                 665                 670
   cgg gga caa ccc ttg tca cca gag aag tat gtg aca tca gcg ccc atg      2064
   Arg Gly Gln Pro Leu Ser Pro Glu Lys Tyr Val Thr Ser Ala Pro Met
           675                 680                 685
   ccc gag cca cag gct ccg ggt agg tat ttt gcc cat tcc atc ctc act      2112
   Pro Glu Pro Gln Ala Pro Gly Arg Tyr Phe Ala His Ser Ile Leu Thr
       690                 695                 700
   gtg tcc gag gaa gag tgg aac acc ggc gaa acg tac acg tgc gtc gta      2160
   Val Ser Glu Glu Glu Trp Asn Thr Gly Glu Thr Tyr Thr Cys Val Val
   705                 710                 715                 720
   gca cac gaa gcg ttg ccc aat aga gtg act gag aga act gta gat aag      2208
   Ala His Glu Ala Leu Pro Asn Arg Val Thr Glu Arg Thr Val Asp Lys
                   725                 730                 735
   tcc act gag ggc gaa gta agc gcg gat gaa gaa ggt ttc gaa aac ttg      2256
   Ser Thr Glu Gly Glu Val Ser Ala Asp Glu Glu Gly Phe Glu Asn Leu
               740                 745                 750
   tgg gct aca gcg agc acg ttt atc gtg ttg ttc ttg ctt tca ctc ttc      2304
   Trp Ala Thr Ala Ser Thr Phe Ile Val Leu Phe Leu Leu Ser Leu Phe
           755                 760                 765
   tac tcc aca act gta acc ctg ttc aag gtc aag tag                      2340
   Tyr Ser Thr Thr Val Thr Leu Phe Lys Val Lys
       770                 775
<210> 8
   <211> 779
   <212> PRT
   <213> Artificial Sequence
<220>
   <223> Synthetic Construct
<400> 8
   Met Ile Leu Thr Ser Phe Gly Asp Asp Met Trp Leu Leu Thr Thr Leu
   1               5                   10                  15
   Leu Leu Trp Val Pro Val Gly Gly Glu Val Val Asn Ala Thr Lys Ala
               20                  25                  30
   Val Ile Thr Leu Gln Pro Pro Trp Val Ser Ile Phe Gln Lys Glu Asn
           35                  40                  45
   Val Thr Leu Trp Cys Glu Gly Pro His Leu Pro Gly Asp Ser Ser Thr
           50                  55                  60
   Gln Trp Phe Ile Asn Gly Thr Ala Val Gln Ile Ser Thr Pro Ser Tyr
   65                  70                  75                  80
   Ser Ile Pro Glu Ala Ser Phe Gln Asp Ser Gly Glu Tyr Arg Cys Gln
                   85                  90                  95
   Ile Gly Ser Ser Met Pro Ser Asp Pro Val Gln Leu Gln Ile His Asn
               100                 105                 110
   Asp Trp Leu Leu Leu Gln Ala Ser Arg Arg Val Leu Thr Glu Gly Glu
           115                 120                 125
   Pro Leu Ala Leu Arg Cys His Gly Trp Lys Asn Lys Leu Val Tyr Asn
       130                 135                 140
   Val Val Phe Tyr Arg Asn Gly Lys Ser Phe Gln Phe Ser Ser Asp Ser
   145                 150                 155                 160
   Glu Val Ala Ile Leu Lys Thr Asn Leu Ser His Ser Gly Ile Tyr His
                   165                 170                 175
   Cys Ser Gly Thr Gly Arg His Arg Tyr Thr Ser Ala Gly Val Ser Ile
               180                 185                 190
   Thr Val Lys Glu Leu Phe Thr Thr Pro Val Leu Arg Ala Ser Val Ser
           195                 200                 205
   Ser Pro Phe Pro Glu Gly Ser Leu Val Thr Leu Asn Cys Glu Thr Asn
       210                 215                 220
   Leu Leu Leu Gln Arg Pro Gly Leu Gln Leu His Phe Ser Phe Tyr Val
   225                 230                 235                 240
   Gly Ser Lys Ile Leu Glu Tyr Arg Asn Thr Ser Ser Glu Tyr His Ile
                   245                 250                 255
   Ala Arg Ala Glu Arg Glu Asp Ala Gly Phe Tyr Trp Cys Glu Val Ala
               260                 265                 270
   Thr Glu Asp Ser Ser Val Leu Lys Arg Ser Pro Glu Leu Glu Leu Gln
           275                 280                 285
   Val Leu Gly Pro Gln Ser Ser Ala Pro Gly Ser Ala Ser Gly Ser Gly
       290                 295                 300
   Gly Ser Ala Ser Ala Pro Thr Leu Phe Pro Leu Val Ser Cys Glu Asn
   305                 310                 315                 320
   Ser Pro Ser Asp Thr Ser Ser Val Ala Val Gly Cys Leu Ala Gln Asp
                   325                 330                 335
   Phe Leu Pro Asp Ser Ile Thr Phe Ser Trp Lys Tyr Lys Asn Asn Ser
               340                 345                 350
   Asp Ile Ser Ser Thr Arg Gly Phe Pro Ser Val Leu Arg Gly Gly Lys
           355                 360                 365
   Tyr Ala Ala Thr Ser Gln Val Leu Leu Pro Ser Lys Asp Val Met Gln
       370                 375                 380
   Gly Thr Asp Glu His Val Val Cys Lys Val Gln His Pro Asn Gly Asn
   385                 390                 395                 400
   Lys Glu Lys Asn Val Pro Leu Pro Val Ile Ala Glu Leu Pro Pro Lys
                   405                 410                 415
   Val Ser Val Phe Val Pro Pro Arg Asp Gly Phe Phe Gly Asn Pro Arg
               420                 425                 430
   Ser Lys Ser Lys Leu Ile Cys Gln Ala Thr Gly Phe Ser Pro Arg Gln
           435                 440                 445
   Ile Gln Val Ser Trp Leu Arg Glu Gly Lys Gln Val Gly Ser Gly Val
       450                 455                 460
   Thr Thr Asp Gln Val Gln Ala Glu Ala Lys Glu Ser Gly Pro Thr Thr
   465                 470                 475                 480
   Tyr Lys Val Thr Ser Thr Leu Thr Ile Lys Glu Ser Asp Trp Leu Ser
                   485                 490                 495
   Gln Ser Met Phe Thr Cys Arg Val Asp His Arg Gly Leu Thr Phe Gln
               500                 505                 510
   Gln Asn Ala Ser Ser Met Cys Val Pro Asp Gln Asp Thr Ala Ile Arg
           515                 520                 525
   Val Phe Ala Ile Pro Pro Ser Phe Ala Ser Ile Phe Leu Thr Lys Ser
       530                 535                 540
   Thr Lys Leu Thr Cys Leu Val Thr Asp Leu Thr Thr Tyr Asp Ser Val
   545                 550                 555                 560
   Thr Ile Ser Trp Thr Arg Gln Asn Gly Glu Ala Val Lys Thr His Thr
                   565                 570                 575
   Asn Ile Ser Glu Ser His Pro Asn Ala Thr Phe Ser Ala Val Gly Glu
               580                 585                 590
   Ala Ser Ile Cys Glu Asp Asp Trp Asn Ser Gly Glu Arg Phe Thr Cys
           595                 600                 605
   Thr Val Thr His Thr Asp Leu Pro Ser Pro Leu Lys Gln Thr Ile Ser
       610                 615                 620
   Arg Pro Lys Gly Val Ala Leu His Arg Pro Asp Val Tyr Leu Leu Pro
   625                 630                 635                 640
   Pro Ala Arg Glu Gln Leu Asn Leu Arg Glu Ser Ala Thr Ile Thr Cys
                   645                 650                 655
   Leu Val Thr Gly Phe Ser Pro Ala Asp Val Phe Val Gln Trp Met Gln
               660                 665                 670
   Arg Gly Gln Pro Leu Ser Pro Glu Lys Tyr Val Thr Ser Ala Pro Met
           675                 680                 685
   Pro Glu Pro Gln Ala Pro Gly Arg Tyr Phe Ala His Ser Ile Leu Thr
       690                 695                 700
   Val Ser Glu Glu Glu Trp Asn Thr Gly Glu Thr Tyr Thr Cys Val Val
   705                 710                 715                 720
   Ala His Glu Ala Leu Pro Asn Arg Val Thr Glu Arg Thr Val Asp Lys
                   725                 730                 735
   Ser Thr Glu Gly Glu Val Ser Ala Asp Glu Glu Gly Phe Glu Asn Leu
               740                 745                 750
   Trp Ala Thr Ala Ser Thr Phe Ile Val Leu Phe Leu Leu Ser Leu Phe
           755                 760                 765
   Tyr Ser Thr Thr Val Thr Leu Phe Lys Val Lys
       770                 775
<210> 9
   <211> 2259
   <212> DNA
   <213> Artificial Sequence
<220>
   <223> Chimeric Protein: FcgammaRl-IgAB
<220>
   <221> CDS
   <222> (1) .. (2259)
<400> 9
   atg att ctt acc agc ttt gga gat gac atg tgg ctt cta aca act ctg      48
   Met Ile Leu Thr Ser Phe Gly Asp Asp Met Trp Leu Leu Thr Thr Leu
   1               5                   10                  15
   cta ctt tgg gtt cca gtc ggt ggg gaa gtg gtt aat gcc acc aag gct      96
   Leu Leu Trp Val Pro Val Gly Gly Glu Val Val Asn Ala Thr Lys Ala
               20                  25                  30
   gtg atc acc ttg cag cct cca tgg gtc agt att ttc cag aag gaa aat      144
   Val Ile Thr Leu Gln Pro Pro Trp Val Ser Ile Phe Gln Lys Glu Asn
           35                  40                  45
   gtc act tta tgg tgt gag ggg cct cac ctg cct gga gac agt tcc aca      192
   Val Thr Leu Trp Cys Glu Gly Pro His Leu Pro Gly Asp Ser Ser Thr
       50                  55                  60
   caa tgg ttt atc aac gga aca gcc gtt cag atc tcc acg cct agt tat      240
   Gln Trp Phe Ile Asn Gly Thr Ala Val Gln Ile Ser Thr Pro Ser Tyr
   65                  70                  75                  80
   agc atc cca gag gcc agt ttt cag gac agt ggc gaa tac agg tgt cag      288
   Ser Ile Pro Glu Ala Ser Phe Gln Asp Ser Gly Glu Tyr Arg Cys Gln
                   85                  90                  95
   ata ggt tcc tca atg cca agt gac cct gtg cag ttg caa atc cac aat      336
   Ile Gly Ser Ser Met Pro Ser Asp Pro Val Gln Leu Gln Ile His Asn
               100                 105                 110
   gat tgg ctg cta ctc cag gcc tcc cgc aga gtc ctc aca gaa gga gaa      384
   Asp Trp Leu Leu Leu Gln Ala Ser Arg Arg Val Leu Thr Glu Gly Glu
           115                 120                 125
   ccc ctg gcc ttg agg tgt cac gga tgg aag aat aaa ctg gtg tac aat      432
   Pro Leu Ala Leu Arg Cys His Gly Trp Lys Asn Lys Leu Val Tyr Asn
       130                 135                 140
   gtg gtt ttc tat aga aat gga aaa tcc ttt cag ttt tct tca gat tcg      480
   Val Val Phe Tyr Arg Asn Gly Lys Ser Phe Gln Phe Ser Ser Asp Ser
   145                 150                 155                 160
   gag gtc gcc att ctg aaa acc aac ctg agt cac agc ggc atc tac cac      528
   Glu Val Ala Ile Leu Lys Thr Asn Leu Ser His Ser Gly Ile Tyr His
                   165                 170                 175
   tgc tca ggc acg gga aga cac cgc tac aca tct gca gga gtg tcc atc      576
   Cys Ser Gly Thr Gly Arg His Arg Tyr Thr Ser Ala Gly Val Ser Ile
               180                 185                 190
   acg gtg aaa gag ctg ttt acc acg cca gtg ctg aga gca tcc gtg tca      624
   Thr Val Lys Glu Leu Phe Thr Thr Pro Val Leu Arg Ala Ser Val Ser
           195                 200                 205
   tct ccc ttc ccg gag ggg agt ctg gtc acc ctg aac tgt gag acg aat      672
   Ser Pro Phe Pro Glu Gly Ser Leu Val Thr Leu Asn Cys Glu Thr Asn
       210                 215                 220
   ttg ctc ctg cag aga ccc ggc tta cag ctt cac ttc tcc ttc tac gtg        720
   Leu Leu Leu Gln Arg Pro Gly Leu Gln Leu His Phe Ser Phe Tyr Val
   225                 230                 235                 240
   ggc agc aag atc ctg gag tac agg aac aca tcc tca gag tac cat ata        768
   Gly Ser Lys Ile Leu Glu Tyr Arg Asn Thr Ser Ser Glu Tyr His Ile
                   245                 250                 255
   gca agg gcg gaa aga gaa gat gct gga ttc tac tgg tgt gag gta gcc        816
   Ala Arg Ala Glu Arg Glu Asp Ala Gly Phe Tyr Trp Cys Glu Val Ala
               260                 265                 270
   acg gag gac agc agt gtc ctt aag cgc agc cct gag ttg gag ctc caa        864
   Thr Glu Asp Ser Ser Val Leu Lys Arg Ser Pro Glu Leu Glu Leu Gln
           275                 280                 285
   gtg ctt ggt ccc cag tca tca gct cct ggt tct gct tcg ggc tca gga        912
   Val Leu Gly Pro Gln Ser Ser Ala Pro Gly Ser Ala Ser Gly Ser Gly
       290                 295                 300
   ctg tgg atg cat aag gtg cct gca tcg ctc atg gtg agc ctg ggc gaa        960
   Leu Trp Met His Lys Val Pro Ala Ser Leu Met Val Ser Leu Gly Glu
   305                 310                 315                 320
   gat gca cat ttt cag tgt ccc cat aac agc tcc aac aac gcg aac gtg       1008
   Asp Ala His Phe Gln Cys Pro His Asn Ser Ser Asn Asn Ala Asn Val
                   325                 330                 335
   acc tgg tgg cgg gtg ctc cat ggc aat tac acc tgg ccg cct gaa ttt       1056
   Thr Trp Trp Arg Val Leu His Gly Asn Tyr Thr Trp Pro Pro Glu Phe
               340                 345                 350
   ctc gga ccg gga gag gac ccg aat ggg acc ctt atc atc cag aac gtg       1104
   Leu Gly Pro Gly Glu Asp Pro Asn Gly Thr Leu Ile Ile Gln Asn Val
           355                 360                 365
   aat aag tcc cac gga gga atc tac gtc tgc cgc gtg caa gag gga aat       1152
   Asn Lys Ser His Gly Gly Ile Tyr Val Cys Arg Val Gln Glu Gly Asn
       370                 375                 380
   gag agc tac caa cag tca tgc gga acg tac ctc cgc gtc cgg cag cca       1200
   Glu Ser Tyr Gln Gln Ser Cys Gly Thr Tyr Leu Arg Val Arg Gln Pro
   385                 390                 395                 400
   cca ccg agg ccg ttc ctc gat atg gga gag gga act aag aac cgg atc       1248
   Pro Pro Arg Pro Phe Leu Asp Met Gly Glu Gly Thr Lys Asn Arg Ile
                   405                 410                 415
   att acc gcc gaa ggc atc atc ctc ctc ttc tgc gcc gtc gtg ccg ggg       1296
   Ile Thr Ala Glu Gly Ile Ile Leu Leu Phe Cys Ala Val Val Pro Gly
               420                 425                 430
   act ctg ctt ctg ttc cgg aaa agg tgg caa aac gaa aag ctg ggt ctg       1344
   Thr Leu Leu Leu Phe Arg Lys Arg Trp Gln Asn Glu Lys Leu Gly Leu
           435                 440                 445
   gac gct ggg gac gaa tac gag gat gaa aac ttg tac gag ggc ctg aat       1392
   Asp Ala Gly Asp Glu Tyr Glu Asp Glu Asn Leu Tyr Glu Gly Leu Asn
       450                 455                 460
   ctg gac gat tgc tcg atg tat gag gac att agc aga gga ctg cag ggt       1440
   Leu Asp Asp Cys Ser Met Tyr Glu Asp Ile Ser Arg Gly Leu Gln Gly
   465                 470                 475                 480
   acc tac caa gac gtg gga agc ctg aac atc ggg gat gtg cag ctc gag        1488
   Thr Tyr Gln Asp Val Gly Ser Leu Asn Ile Gly Asp Val Gln Leu Glu
                   485                 490                 495
   aaa cca agg aaa aga aga gct agc gaa gga cgc gga tca ctg ctt act        1536
   Lys Pro Arg Lys Arg Arg Ala Ser Glu Gly Arg Gly Ser Leu Leu Thr
               500                 505                 510
   tgc ggc gat gtc gaa gaa aat ccc gga cca atg gcc aga ttg gcg ctg        1584
   Cys Gly Asp Val Glu Glu Asn Pro Gly Pro Met Ala Arg Leu Ala Leu
           515                 520                 525
   tcg ccg gtc cct tcg cac tgg atg gtg gcc ctg ctg ctc ttg ctt tcg        1632
   Ser Pro Val Pro Ser His Trp Met Val Ala Leu Leu Leu Leu Leu Ser
       530                 535                 540
   gcg gct gag ccg gtg cca gcg gct aga tcg gag gac cgc tac aga aat        1680
   Ala Ala Glu Pro Val Pro Ala Ala Arg Ser Glu Asp Arg Tyr Arg Asn
   545                 550                 555                 560
   ccg aag ggt tcc gct tgc tca cgc atc tgg caa tca cca cgc ttc atc        1728
   Pro Lys Gly Ser Ala Cys Ser Arg Ile Trp Gln Ser Pro Arg Phe Ile
                   565                 570                 575
   gcg cgc aaa cgc ggc ttc act gtc aag atg cac tgc tat atg aac tcg        1776
   Ala Arg Lys Arg Gly Phe Thr Val Lys Met His Cys Tyr Met Asn Ser
               580                 585                 590
   gcc agc ggg aat gtg tcg tgg ctg tgg aag cag gaa atg gac gag aat        1824
   Ala Ser Gly Asn Val Ser Trp Leu Trp Lys Gln Glu Met Asp Glu Asn
           595                 600                 605
   ccg cag caa ctg aaa ctg gag aag ggc cgg atg gaa gaa tcc cag aat        1872
   Pro Gln Gln Leu Lys Leu Glu Lys Gly Arg Met Glu Glu Ser Gln Asn
       610                 615                 620
   gag tcg ctg gcc acc ctt act atc caa ggt atc cgg ttt gaa gat aac        1920
   Glu Ser Leu Ala Thr Leu Thr Ile Gln Gly Ile Arg Phe Glu Asp Asn
   625                 630                 635                 640
   ggg atc tac ttc tgt caa cag aag tgt aac aac act tca gag gtg tac        1968
   Gly Ile Tyr Phe Cys Gln Gln Lys Cys Asn Asn Thr Ser Glu Val Tyr
                   645                 650                 655
   cag gga tgc ggc acc gaa ctc cgc gtc atg gga ttc tcc acc ctc gcc        2016
   Gln Gly Cys Gly Thr Glu Leu Arg Val Met Gly Phe Ser Thr Leu Ala
               660                 665                 670
   caa ctg aag cag cgc aac acg ctg aag gac ggc atc att atg atc cag        2064
   Gln Leu Lys Gln Arg Asn Thr Leu Lys Asp Gly Ile Ile Met Ile Gln
           675                 680                 685
   acc ctg ctg atc atc ctg ttc atc att gtg ccg atc ttt ctg ctc ttg        2112
   Thr Leu Leu Ile Ile Leu Phe Ile Ile Val Pro Ile Phe Leu Leu Leu
       690                 695                 700
   gat aag gac gac tcg aaa gcc gga atg gaa gag gac cac acg tac gaa        2160
   Asp Lys Asp Asp Ser Lys Ala Gly Met Glu Glu Asp His Thr Tyr Glu
   705                 710                 715                 720
   ggt ctg gac atc gac caa act gcg act tac gag gac att gtg acc ctc        2208
   Gly Leu Asp Ile Asp Gln Thr Ala Thr Tyr Glu Asp Ile Val Thr Leu
                   725                 730                 735
   cgg act ggc gaa gtc aag tgg tcc gtg ggt gaa cac cct gga cag gaa        2256
   Arg Thr Gly Glu Val Lys Trp Ser Val Gly Glu His Pro Gly Gln Glu
               740                 745                 750
   tag                                                                    2259
<210> 10
   <211> 752
   <212> PRT
   <213> Artificial Sequence
<220>
   <223> Synthetic Construct
<400> 10
   Met Ile Leu Thr Ser Phe Gly Asp Asp Met Trp Leu Leu Thr Thr Leu
   1               5                   10                  15
   Leu Leu Trp Val Pro Val Gly Gly Glu Val Val Asn Ala Thr Lys Ala
               20                  25                  30
   Val Ile Thr Leu Gln Pro Pro Trp Val Ser Ile Phe Gln Lys Glu Asn
           35                  40                  45
   Val Thr Leu Trp Cys Glu Gly Pro His Leu Pro Gly Asp Ser Ser Thr
       50                  55                  60
   Gln Trp Phe Ile Asn Gly Thr Ala Val Gln Ile Ser Thr Pro Ser Tyr
   65                  70                  75                  80
   Ser Ile Pro Glu Ala Ser Phe Gln Asp Ser Gly Glu Tyr Arg Cys Gln
                   85                  90                  95
   Ile Gly Ser Ser Met Pro Ser Asp Pro Val Gln Leu Gln Ile His Asn
               100                 105                 110
   Asp Trp Leu Leu Leu Gln Ala Ser Arg Arg Val Leu Thr Glu Gly Glu
           115                 120                 125
   Pro Leu Ala Leu Arg Cys His Gly Trp Lys Asn Lys Leu Val Tyr Asn
       130                 135                 140
   Val Val Phe Tyr Arg Asn Gly Lys Ser Phe Gln Phe Ser Ser Asp Ser
   145                 150                 155                 160
   Glu Val Ala Ile Leu Lys Thr Asn Leu Ser His Ser Gly Ile Tyr His
                   165                 170                 175
   Cys Ser Gly Thr Gly Arg His Arg Tyr Thr Ser Ala Gly Val Ser Ile
               180                 185                 190
   Thr Val Lys Glu Leu Phe Thr Thr Pro Val Leu Arg Ala Ser Val Ser
           195                 200                 205
   Ser Pro Phe Pro Glu Gly Ser Leu Val Thr Leu Asn Cys Glu Thr Asn
       210                 215                 220
   Leu Leu Leu Gln Arg Pro Gly Leu Gln Leu His Phe Ser Phe Tyr Val
   225                 230                 235                 240
   Gly Ser Lys Ile Leu Glu Tyr Arg Asn Thr Ser Ser Glu Tyr His Ile
                   245                 250                 255
   Ala Arg Ala Glu Arg Glu Asp Ala Gly Phe Tyr Trp Cys Glu Val Ala
               260                 265                 270
   Thr Glu Asp Ser Ser Val Leu Lys Arg Ser Pro Glu Leu Glu Leu Gln
           275                 280                 285
   Val Leu Gly Pro Gln Ser Ser Ala Pro Gly Ser Ala Ser Gly Ser Gly
       290                 295                 300
   Leu Trp Met His Lys Val Pro Ala Ser Leu Met Val Ser Leu Gly Glu
   305                 310                 315                 320
   Asp Ala His Phe Gln Cys Pro His Asn Ser Ser Asn Asn Ala Asn Val
                   325                 330                 335
   Thr Trp Trp Arg Val Leu His Gly Asn Tyr Thr Trp Pro Pro Glu Phe
               340                 345                 350
   Leu Gly Pro Gly Glu Asp Pro Asn Gly Thr Leu Ile Ile Gln Asn Val
           355                 360                 365
   Asn Lys Ser His Gly Gly Ile Tyr Val Cys Arg Val Gln Glu Gly Asn
       370                 375                 380
   Glu Ser Tyr Gln Gln Ser Cys Gly Thr Tyr Leu Arg Val Arg Gln Pro
   385                 390                 395                 400
   Pro Pro Arg Pro Phe Leu Asp Met Gly Glu Gly Thr Lys Asn Arg Ile
                   405                 410                 415
   Ile Thr Ala Glu Gly Ile Ile Leu Leu Phe Cys Ala Val Val Pro Gly
               420                 425                 430
   Thr Leu Leu Leu Phe Arg Lys Arg Trp Gln Asn Glu Lys Leu Gly Leu
   435 440 445
   Asp Ala Gly Asp Glu Tyr Glu Asp Glu Asn Leu Tyr Glu Gly Leu Asn
   450 455 460
   Leu Asp Asp Cys Ser Met Tyr Glu Asp Ile Ser Arg Gly Leu Gln Gly
   465 470 475 480
   Thr Tyr Gln Asp Val Gly Ser Leu Asn Ile Gly Asp Val Gln Leu Glu
   485 490 495
   Lys Pro Arg Lys Arg Arg Ala Ser Glu Gly Arg Gly Ser Leu Leu Thr
   500 505 510
   Cys Gly Asp Val Glu Glu Asn Pro Gly Pro Met Ala Arg Leu Ala Leu
   515 520 525
   Ser Pro Val Pro Ser His Trp Met Val Ala Leu Leu Leu Leu Leu Ser
   530 535 540
   Ala Ala Glu Pro Val Pro Ala Ala Arg Ser Glu Asp Arg Tyr Arg Asn
   545 550 555 560
   Pro Lys Gly Ser Ala Cys Ser Arg Ile Trp Gln Ser Pro Arg Phe Ile
   565 570 575
   Ala Arg Lys Arg Gly Phe Thr Val Lys Met His Cys Tyr Met Asn Ser
   580 585 590
   Ala Ser Gly Asn Val Ser Trp Leu Trp Lys Gln Glu Met Asp Glu Asn
   595 600 605
   Pro Gln Gln Leu Lys Leu Glu Lys Gly Arg Met Glu Glu Ser Gln Asn
   610 615 620
   Glu Ser Leu Ala Thr Leu Thr Ile Gln Gly Ile Arg Phe Glu Asp Asn
   625 630 635 640
   Gly Ile Tyr Phe Cys Gln Gln Lys Cys Asn Asn Thr Ser Glu Val Tyr
   645 650 655
   Gln Gly Cys Gly Thr Glu Leu Arg Val Met Gly Phe Ser Thr Leu Ala
   660 665 670
   Gln Leu Lys Gln Arg Asn Thr Leu Lys Asp Gly Ile Ile Met Ile Gln
       675                 680                 685
   Thr Leu Leu Ile Ile Leu Phe Ile Ile Val Pro Ile Phe Leu Leu Leu
       690                 695                 700
   Asp Lys Asp Asp Ser Lys Ala Gly Met Glu Glu Asp His Thr Tyr Glu
   705                 710                 715                 720
   Gly Leu Asp Ile Asp Gln Thr Ala Thr Tyr Glu Asp Ile Val Thr Leu
                   725                 730                 735
   Arg Thr Gly Glu Val Lys Trp Ser Val Gly Glu His Pro Gly Gln Glu
               740                 745                 750
<210> 11
   <211> 1335
   <212> DNA
   <213> Artificial Sequence
<220>
   <223> Chimeric Protein: FcgammaRl-CD3Z
<220>
   <221> CDS
   <222> (1) .. (1335)
<400> 11
   atg aag tgg aag gcg ctt ttc acc gcg gcc atc ctg cag gca cag ttg          48
   Met Lys Trp Lys Ala Leu Phe Thr Ala Ala Ile Leu Gln Ala Gln Leu
   1               5                   10                  15
   ccg att aca gag gca gaa gtg gtt aat gcc acc aag gct gtg atc acc          96
   Pro Ile Thr Glu Ala Glu Val Val Asn Ala Thr Lys Ala Val Ile Thr
               20                  25                  30
   ttg cag cct cca tgg gtc agt att ttc cag aag gaa aat gtc act tta         144
   Leu Gln Pro Pro Trp Val Ser Ile Phe Gln Lys Glu Asn Val Thr Leu
           35                  40                  45
   tgg tgt gag ggg cct cac ctg cct gga gac agt tcc aca caa tgg ttt         192
   Trp Cys Glu Gly Pro His Leu Pro Gly Asp Ser Ser Thr Gln Trp Phe
       50                  55                  60
   atc aac gga aca gcc gtt cag atc tcc acg cct agt tat agc atc cca         240
   Ile Asn Gly Thr Ala Val Gln Ile Ser Thr Pro Ser Tyr Ser Ile Pro
   65                  70                  75                  80
   gag gcc agt ttt cag gac agt ggc gaa tac agg tgt cag ata ggt tcc         288
   Glu Ala Ser Phe Gln Asp Ser Gly Glu Tyr Arg Cys Gln Ile Gly Ser
                   85                  90                  95
   tca atg cca agt gac cct gtg cag ttg caa atc cac aat gat tgg ctg         336
   Ser Met Pro Ser Asp Pro Val Gln Leu Gln Ile His Asn Asp Trp Leu
               100                 105                 110
   cta ctc cag gcc tcc cgc aga gtc ctc aca gaa gga gaa ccc ctg gcc         384
   Leu Leu Gln Ala Ser Arg Arg Val Leu Thr Glu Gly Glu Pro Leu Ala
           115                 120                 125
   ttg agg tgt cac gga tgg aag aat aaa ctg gtg tac aat gtg gtt ttc        432
   Leu Arg Cys His Gly Trp Lys Asn Lys Leu Val Tyr Asn Val Val Phe
       130                 135                 140
   tat aga aat gga aaa tcc ttt cag ttt tct tca gat tcg gag gtc gcc        480
   Tyr Arg Asn Gly Lys Ser Phe Gln Phe Ser Ser Asp Ser Glu Val Ala
   145                 150                 155                 160
   att ctg aaa acc aac ctg agt cac agc ggc atc tac cac tgc tca ggc        528
   Ile Leu Lys Thr Asn Leu Ser His Ser Gly Ile Tyr His Cys Ser Gly
                   165                 170                 175
   acg gga aga cac cgc tac aca tct gca gga gtg tcc atc acg gtg aaa        576
   Thr Gly Arg His Arg Tyr Thr Ser Ala Gly Val Ser Ile Thr Val Lys
               180                 185                 190
   gag ctg ttt acc acg cca gtg ctg aga gca tcc gtg tca tct ccc ttc        624
   Glu Leu Phe Thr Thr Pro Val Leu Arg Ala Ser Val Ser Ser Pro Phe
           195                 200                 205
   ccg gag ggg agt ctg gtc acc ctg aac tgt gag acg aat ttg ctc ctg        672
   Pro Glu Gly Ser Leu Val Thr Leu Asn Cys Glu Thr Asn Leu Leu Leu
       210                 215                 220
   cag aga ccc ggc tta cag ctt cac ttc tcc ttc tac gtg ggc agc aag        720
   Gln Arg Pro Gly Leu Gln Leu His Phe Ser Phe Tyr Val Gly Ser Lys
   225                 230                 235                 240
   atc ctg gag tac agg aac aca tcc tca gag tac cat ata gca agg gcg        768
   Ile Leu Glu Tyr Arg Asn Thr Ser Ser Glu Tyr His Ile Ala Arg Ala
                   245                 250                 255
   gaa aga gaa gat gct gga ttc tac tgg tgt gag gta gcc acg gag gac        816
   Glu Arg Glu Asp Ala Gly Phe Tyr Trp Cys Glu Val Ala Thr Glu Asp
               260                 265                 270
   agc agt gtc ctt aag cgc agc cct gag ttg gag ctc caa gtg ctt ggt        864
   Ser Ser Val Leu Lys Arg Ser Pro Glu Leu Glu Leu Gln Val Leu Gly
           275                 280                 285
   ccc cag tca tca gct cct ggt tct gct tct ggt tct ggt cag agc ttt        912
   Pro Gln Ser Ser Ala Pro Gly Ser Ala Ser Gly Ser Gly Gln Ser Phe
       290                 295                 300
   ggc ctg ctg gat ccc aaa ctc tgc tac ctg ctg gat gga atc ctc ttc        960
   Gly Leu Leu Asp Pro Lys Leu Cys Tyr Leu Leu Asp Gly Ile Leu Phe
   305                 310                 315                 320
   atc tat ggt gtc att ctc act gcc ttg ttc ctg aga gtg aag ttc agc       1008
   Ile Tyr Gly Val Ile Leu Thr Ala Leu Phe Leu Arg Val Lys Phe Ser
                   325                 330                 335
   agg agc gca gac gcc ccc gcg tac cag cag ggc cag aac cag ctc tat       1056
   Arg Ser Ala Asp Ala Pro Ala Tyr Gln Gln Gly Gln Asn Gln Leu Tyr
               340                 345                 350
   aac gag ctc aat cta gga cga aga gag gag tac gat gtt ttg gac aag       1104
   Asn Glu Leu Asn Leu Gly Arg Arg Glu Glu Tyr Asp Val Leu Asp Lys
           355                 360                 365
   aga cgt ggc cgg gac cct gag atg ggg gga aag ccg cag aga agg aag       1152
   Arg Arg Gly Arg Asp Pro Glu Met Gly Gly Lys Pro Gln Arg Arg Lys
       370                 375                 380
   aac cct cag gaa ggc ctg tac aat gaa ctg cag aaa gat aag atg gcg      1200
   Asn Pro Gln Glu Gly Leu Tyr Asn Glu Leu Gln Lys Asp Lys Met Ala
   385                 390                 395                 400
   gag gcc tac agt gag att ggg atg aaa ggc gag cgc cgg agg ggc aag      1248
   Glu Ala Tyr Ser Glu Ile Gly Met Lys Gly Glu Arg Arg Arg Gly Lys
                   405                 410                 415
   ggg cac gat ggc ctt tac cag ggt ctc agt aca gcc acc aag gac acc      1296
   Gly His Asp Gly Leu Tyr Gln Gly Leu Ser Thr Ala Thr Lys Asp Thr
               420                 425                 430
   tac gac gcc ctt cac atg cag gcc ctg ccc cct cgc taa                  1335
   Tyr Asp Ala Leu His Met Gln Ala Leu Pro Pro Arg
           435                 440
<210> 12
   <211> 444
   <212> PRT
   <213> Artificial Sequence
<220>
   <223> Synthetic Construct
<400> 12
   Met Lys Trp Lys Ala Leu Phe Thr Ala Ala Ile Leu Gln Ala Gln Leu
   1               5                   10                  15
   Pro Ile Thr Glu Ala Glu Val Val Asn Ala Thr Lys Ala Val Ile Thr
               20                  25                  30
   Leu Gln Pro Pro Trp Val Ser Ile Phe Gln Lys Glu Asn Val Thr Leu
           35                  40                  45
   Trp Cys Glu Gly Pro His Leu Pro Gly Asp Ser Ser Thr Gln Trp Phe
       50                  55                  60
   Ile Asn Gly Thr Ala Val Gln Ile Ser Thr Pro Ser Tyr Ser Ile Pro
   65                  70                  75                  80
   Glu Ala Ser Phe Gln Asp Ser Gly Glu Tyr Arg Cys Gln Ile Gly Ser
                   85                  90                  95
   Ser Met Pro Ser Asp Pro Val Gln Leu Gln Ile His Asn Asp Trp Leu
               100                 105                 110
   Leu Leu Gln Ala Ser Arg Arg Val Leu Thr Glu Gly Glu Pro Leu Ala
           115                 120                 125
   Leu Arg Cys His Gly Trp Lys Asn Lys Leu Val Tyr Asn Val Val Phe
           130                 135                 140
   Tyr Arg Asn Gly Lys Ser Phe Gln Phe Ser Ser Asp Ser Glu Val Ala
   145                 150                 155                 160
   Ile Leu Lys Thr Asn Leu Ser His Ser Gly Ile Tyr His Cys Ser Gly
                   165                 170                 175
   Thr Gly Arg His Arg Tyr Thr Ser Ala Gly Val Ser Ile Thr Val Lys
               180                 185                 190
   Glu Leu Phe Thr Thr Pro Val Leu Arg Ala Ser Val Ser Ser Pro Phe
           195                 200                 205
   Pro Glu Gly Ser Leu Val Thr Leu Asn Cys Glu Thr Asn Leu Leu Leu
       210                 215                 220
   Gln Arg Pro Gly Leu Gln Leu His Phe Ser Phe Tyr Val Gly Ser Lys
   225                 230                 235                 240
   Ile Leu Glu Tyr Arg Asn Thr Ser Ser Glu Tyr His Ile Ala Arg Ala
                   245                 250                 255
   Glu Arg Glu Asp Ala Gly Phe Tyr Trp Cys Glu Val Ala Thr Glu Asp
               260                 265                 270
   Ser Ser Val Leu Lys Arg Ser Pro Glu Leu Glu Leu Gln Val Leu Gly
           275                 280                 285
   Pro Gln Ser Ser Ala Pro Gly Ser Ala Ser Gly Ser Gly Gln Ser Phe
       290                 295                 300
   Gly Leu Leu Asp Pro Lys Leu Cys Tyr Leu Leu Asp Gly Ile Leu Phe
   305                 310                 315                 320
   Ile Tyr Gly Val Ile Leu Thr Ala Leu Phe Leu Arg Val Lys Phe Ser
                   325                 330                 335
   Arg Ser Ala Asp Ala Pro Ala Tyr Gln Gln Gly Gln Asn Gln Leu Tyr
               340                 345                 350
   Asn Glu Leu Asn Leu Gly Arg Arg Glu Glu Tyr Asp Val Leu Asp Lys
           355                 360                 365
   Arg Arg Gly Arg Asp Pro Glu Met Gly Gly Lys Pro Gln Arg Arg Lys
       370                 375                 380
   Asn Pro Gln Glu Gly Leu Tyr Asn Glu Leu Gln Lys Asp Lys Met Ala
   385                 390                 395                 400
   Glu Ala Tyr Ser Glu Ile Gly Met Lys Gly Glu Arg Arg Arg Gly Lys
                   405                 410                 415
   Gly His Asp Gly Leu Tyr Gln Gly Leu Ser Thr Ala Thr Lys Asp Thr
               420                 425                 430
   Tyr Asp Ala Leu His Met Gln Ala Leu Pro Pro Arg
           435                 440
<210> 13
   <211> 1848
   <212> DNA
   <213> Artificial Sequence
<220>
   <223> Chimeric Protein: FcgammaRl-IgE
<220>
   <221> CDS
   <222> (1)..(1848)
<400> 13
   cat atg gaa gtg gtt aat gcc acc aag gct gtg atc acc ttg cag cct       48
   His Met Glu Val Val Asn Ala Thr Lys Ala Val Ile Thr Leu Gln Pro
   1               5                   10                  15
   cca tgg gtc agt att ttc cag aag gaa aat gtc act tta tgg tgt gag       96
   Pro Trp Val Ser Ile Phe Gln Lys Glu Asn Val Thr Leu Trp Cys Glu
               20                  25                  30
   ggg cct cac ctg cct gga gac agt tcc aca caa tgg ttt atc aac gga      144
   Gly Pro His Leu Pro Gly Asp Ser Ser Thr Gln Trp Phe Ile Asn Gly
           35                  40                  45
   aca gcc gtt cag atc tcc acg cct agt tat agc atc cca gag gcc agt      192
   Thr Ala Val Gln Ile Ser Thr Pro Ser Tyr Ser Ile Pro Glu Ala Ser
       50                  55                  60
   ttt cag gac agt ggc gaa tac agg tgt cag ata ggt tcc tca atg cca      240
   Phe Gln Asp Ser Gly Glu Tyr Arg Cys Gln Ile Gly Ser Ser Met Pro
   65                  70                  75                  80
   agt gac cct gtg cag ttg caa atc cac aat gat tgg ctg cta ctc cag      288
   Ser Asp Pro Val Gln Leu Gln Ile His Asn Asp Trp Leu Leu Leu Gln
                   85                  90                  95
   gcc tcc cgc aga gtc ctc aca gaa gga gaa ccc ctg gcc ttg agg tgt      336
   Ala Ser Arg Arg Val Leu Thr Glu Gly Glu Pro Leu Ala Leu Arg Cys
               100                 105                 110
   cac gga tgg aag aat aaa ctg gtg tac aat gtg gtt ttc tat aga aat      384
   His Gly Trp Lys Asn Lys Leu Val Tyr Asn Val Val Phe Tyr Arg Asn
           115                 120                 125
   gga aaa tcc ttt cag ttt tct tca gat tcg gag gtc gcc att ctg aaa      432
   Gly Lys Ser Phe Gln Phe Ser Ser Asp Ser Glu Val Ala Ile Leu Lys
       130                 135                 140
   acc aac ctg agt cac agc ggc atc tac cac tgc tca ggc acg gga aga        480
   Thr Asn Leu Ser His Ser Gly Ile Tyr His Cys Ser Gly Thr Gly Arg
   145                 150                 155                 160
   cac cgc tac aca tct gca gga gtg tcc atc acg gtg aaa gag ctg ttt        528
   His Arg Tyr Thr Ser Ala Gly Val Ser Ile Thr Val Lys Glu Leu Phe
                   165                 170                 175
   acc acg cca gtg ctg aga gca tcc gtg tca tct ccc ttc ccg gag ggg        576
   Thr Thr Pro Val Leu Arg Ala Ser Val Ser Ser Pro Phe Pro Glu Gly
               180                 185                 190
   agt ctg gtc acc ctg aac tgt gag acg aat ttg ctc ctg cag aga ccc        624
   Ser Leu Val Thr Leu Asn Cys Glu Thr Asn Leu Leu Leu Gln Arg Pro
           195                 200                 205
   ggc tta cag ctt cac ttc tcc ttc tac gtg ggc agc aag atc ctg gag        672
   Gly Leu Gln Leu His Phe Ser Phe Tyr Val Gly Ser Lys Ile Leu Glu
       210                 215                 220
   tac agg aac aca tcc tca gag tac cat ata gca agg gcg gaa aga gaa        720
   Tyr Arg Asn Thr Ser Ser Glu Tyr His Ile Ala Arg Ala Glu Arg Glu
   225                 230                 235                 240
   gat gct gga ttc tac tgg tgt gag gta gcc acg gag gac agc agt gtc        768
   Asp Ala Gly Phe Tyr Trp Cys Glu Val Ala Thr Glu Asp Ser Ser Val
                   245                 250                 255
   ctt aag cgc agc cct gag ttg gag ctc caa gtg ctt ggt ccc cag tca        816
   Leu Lys Arg Ser Pro Glu Leu Glu Leu Gln Val Leu Gly Pro Gln Ser
               260                 265                 270
   tca gct cct ggt tct gct tct ggt tct ggt gtt cga cca gtt aac atc        864
   Ser Ala Pro Gly Ser Ala Ser Gly Ser Gly Val Arg Pro Val Asn Ile
           275                 280                 285
   acc gag ccc aca ctg gaa ctg ctg cac agc agc tgc gac ccc aac gcc        912
   Thr Glu Pro Thr Leu Glu Leu Leu His Ser Ser Cys Asp Pro Asn Ala
       290                 295                 300
   ttc cac agc acc atc cag ctg tat tgc ttc atc tac ggc cac atc ctg        960
   Phe His Ser Thr Ile Gln Leu Tyr Cys Phe Ile Tyr Gly His Ile Leu
   305                 310                 315                 320
   aac gac gtg tcc gtg tcc tgg ctg atg gac gac aga gag atc acc gac       1008
   Asn Asp Val Ser Val Ser Trp Leu Met Asp Asp Arg Glu Ile Thr Asp
                   325                 330                 335
   acc ctg gcc cag acc gtg ctg atc aaa gag gaa ggc aag ctg gcc tct       1056
   Thr Leu Ala Gln Thr Val Leu Ile Lys Glu Glu Gly Lys Leu Ala Ser
               340                 345                 350
   acc tgc agc aag ctg aat atc aca gag cag cag tgg atg agc gag agc       1104
   Thr Cys Ser Lys Leu Asn Ile Thr Glu Gln Gln Trp Met Ser Glu Ser
           355                 360                 365
   acc ttc acc tgt aaa gtg acc tcc cag ggc gtg gac tac ctg gcc cac       1152
   Thr Phe Thr Cys Lys Val Thr Ser Gln Gly Val Asp Tyr Leu Ala His
       370                 375                 380
   acc aga aga tgc ccc gac cac gaa ccc aga ggc gtg atc acc tac ctg       1200
   Thr Arg Arg Cys Pro Asp His Glu Pro Arg Gly Val Ile Thr Tyr Leu
   385                 390                 395                 400
   atc ccc cct agc ccc ctg gac ctg tac cag aac ggc gct cct aag ctg       1248
   Ile Pro Pro Ser Pro Leu Asp Leu Tyr Gln Asn Gly Ala Pro Lys Leu
               405                 410                 415
   acc tgc ctg gtg gtg gac ctg gaa agc gag aag aac gtg aac gtg aca       1296
   Thr Cys Leu Val Val Asp Leu Glu Ser Glu Lys Asn Val Asn Val Thr
           420                 425                 430
   tgg aac cag gaa aag aaa acc agc gtg tcc gcc agc cag tgg tac acc       1344
   Trp Asn Gln Glu Lys Lys Thr Ser Val Ser Ala Ser Gln Trp Tyr Thr
       435                 440                 445
   aag cac cac aac aac gcc acc acc tcc atc acc agc atc ctg ccc gtg       1392
   Lys His His Asn Asn Ala Thr Thr Ser Ile Thr Ser Ile Leu Pro Val
       450                 455                 460
   gtg gcc aag gac tgg atc gag ggc tac ggc tac cag tgc atc gtg gac       1440
   Val Ala Lys Asp Trp Ile Glu Gly Tyr Gly Tyr Gln Cys Ile Val Asp
   465                 470                 475                 480
   cac ccc gac ttc cct aag ccc atc gtg cgg agc atc acc aag acc cct       1488
   His Pro Asp Phe Pro Lys Pro Ile Val Arg Ser Ile Thr Lys Thr Pro
               485                 490                 495
   ggc cag aga tct gcc ccc gag gtg tac gtg ttc ccc cca cct gag gaa       1536
   Gly Gln Arg Ser Ala Pro Glu Val Tyr Val Phe Pro Pro Pro Glu Glu
           500                 505                 510
   gag tcc gag gac aag aga acc ctg acc tgt ctg atc cag aac ttc ttc       1584
   Glu Ser Glu Asp Lys Arg Thr Leu Thr Cys Leu Ile Gln Asn Phe Phe
       515                 520                 525
   cca gag gac atc agc gtg cag tgg ctg ggc gac ggc aag ctg atc tcc       1632
   Pro Glu Asp Ile Ser Val Gln Trp Leu Gly Asp Gly Lys Leu Ile Ser
       530                 535                 540
   aac agc cag cac agc aca acc acc cct ctg aag tcc aac ggc agc aac       1680
   Asn Ser Gln His Ser Thr Thr Thr Pro Leu Lys Ser Asn Gly Ser Asn
   545                 550                 555                 560
   cag ggc ttc ttc atc ttc agc aga ctg gaa gtg gcc aag acc ctg tgg       1728
   Gln Gly Phe Phe Ile Phe Ser Arg Leu Glu Val Ala Lys Thr Leu Trp
               565                 570                 575
   acc cag aga aag cag ttt aca tgc caa gtg atc cat gag gcc ctg cag       1776
   Thr Gln Arg Lys Gln Phe Thr Cys Gln Val Ile His Glu Ala Leu Gln
           580                 585                 590
   aag ccc aga aag ctg gaa aag acc atc agc acc agc ctg ggc aac acc       1824
   Lys Pro Arg Lys Leu Glu Lys Thr Ile Ser Thr Ser Leu Gly Asn Thr
       595                 600                 605
   tcc ctg cgc cct agt tag ctc gag                                       1848
   Ser Leu Arg Pro Ser     Leu Glu
       610                 615
<210> 14
   <211> 613
   <212> PRT
   <213> Artificial Sequence
<220>
   <223> Synthetic Construct
<400> 14
   His Met Glu Val Val Asn Ala Thr Lys Ala Val Ile Thr Leu Gln Pro
   1               5                   10                  15
   Pro Trp Val Ser Ile Phe Gln Lys Glu Asn Val Thr Leu Trp Cys Glu
               20                  25                  30
   Gly Pro His Leu Pro Gly Asp Ser Ser Thr Gln Trp Phe Ile Asn Gly
           35                  40                  45
   Thr Ala Val Gln Ile Ser Thr Pro Ser Tyr Ser Ile Pro Glu Ala Ser
       50                  55                  60
   Phe Gln Asp Ser Gly Glu Tyr Arg Cys Gln Ile Gly Ser Ser Met Pro
   65                  70                  75                  80
   Ser Asp Pro Val Gln Leu Gln Ile His Asn Asp Trp Leu Leu Leu Gln
                   85                  90                  95
   Ala Ser Arg Arg Val Leu Thr Glu Gly Glu Pro Leu Ala Leu Arg Cys
               100                 105                 110
   His Gly Trp Lys Asn Lys Leu Val Tyr Asn Val Val Phe Tyr Arg Asn
           115                 120                 125
   Gly Lys Ser Phe Gln Phe Ser Ser Asp Ser Glu Val Ala Ile Leu Lys
       130                 135                 140
   Thr Asn Leu Ser His Ser Gly Ile Tyr His Cys Ser Gly Thr Gly Arg
   145                 150                 155                 160
   His Arg Tyr Thr Ser Ala Gly Val Ser Ile Thr Val Lys Glu Leu Phe
                   165                 170                 175
   Thr Thr Pro Val Leu Arg Ala Ser Val Ser Ser Pro Phe Pro Glu Gly
               180                 185                 190
   Ser Leu Val Thr Leu Asn Cys Glu Thr Asn Leu Leu Leu Gln Arg Pro
           195                 200                 205
   Gly Leu Gln Leu His Phe Ser Phe Tyr Val Gly Ser Lys Ile Leu Glu
       210                 215                 220
   Tyr Arg Asn Thr Ser Ser Glu Tyr His Ile Ala Arg Ala Glu Arg Glu
   225                 230                 235                 240
   Asp Ala Gly Phe Tyr Trp Cys Glu Val Ala Thr Glu Asp Ser Ser Val
                   245                 250                 255
   Leu Lys Arg Ser Pro Glu Leu Glu Leu Gln Val Leu Gly Pro Gln Ser
               260                 265                 270
   Ser Ala Pro Gly Ser Ala Ser Gly Ser Gly Val Arg Pro Val Asn Ile
           275                 280                 285
   Thr Glu Pro Thr Leu Glu Leu Leu His Ser Ser Cys Asp Pro Asn Ala
       290                 295                 300
   Phe His Ser Thr Ile Gln Leu Tyr Cys Phe Ile Tyr Gly His Ile Leu
   305                 310                 315                 320
   Asn Asp Val Ser Val Ser Trp Leu Met Asp Asp Arg Glu Ile Thr Asp
                   325                 330                 335
   Thr Leu Ala Gln Thr Val Leu Ile Lys Glu Glu Gly Lys Leu Ala Ser
               340                 345                 350
   Thr Cys Ser Lys Leu Asn Ile Thr Glu Gln Gln Trp Met Ser Glu Ser
           355                 360                 365
   Thr Phe Thr Cys Lys Val Thr Ser Gln Gly Val Asp Tyr Leu Ala His
       370                 375                 380
   Thr Arg Arg Cys Pro Asp His Glu Pro Arg Gly Val Ile Thr Tyr Leu
   385                 390                 395                 400
   Ile Pro Pro Ser Pro Leu Asp Leu Tyr Gln Asn Gly Ala Pro Lys Leu
                   405                 410                 415
   Thr Cys Leu Val Val Asp Leu Glu Ser Glu Lys Asn Val Asn Val Thr
               420                 425                 430
   Trp Asn Gln Glu Lys Lys Thr Ser Val Ser Ala Ser Gln Trp Tyr Thr
           435                 440                 445
   Lys His His Asn Asn Ala Thr Thr Ser Ile Thr Ser Ile Leu Pro Val
       450                 455                 460
   Val Ala Lys Asp Trp Ile Glu Gly Tyr Gly Tyr Gln Cys Ile Val Asp
   465                 470                 475                 480
   His Pro Asp Phe Pro Lys Pro Ile Val Arg Ser Ile Thr Lys Thr Pro
                   485                 490                 495
   Gly Gln Arg Ser Ala Pro Glu Val Tyr Val Phe Pro Pro Pro Glu Glu
               500                 505                 510
   Glu Ser Glu Asp Lys Arg Thr Leu Thr Cys Leu Ile Gln Asn Phe Phe
           515                 520                 525
   Pro Glu Asp Ile Ser Val Gln Trp Leu Gly Asp Gly Lys Leu Ile Ser
       530                 535                 540
   Asn Ser Gln His Ser Thr Thr Thr Pro Leu Lys Ser Asn Gly Ser Asn
   545                 550                 555                 560
   Gln Gly Phe Phe Ile Phe Ser Arg Leu Glu Val Ala Lys Thr Leu Trp
                   565                 570                 575
   Thr Gln Arg Lys Gln Phe Thr Cys Gln Val Ile His Glu Ala Leu Gln
               580                 585                 590
   Lys Pro Arg Lys Leu Glu Lys Thr Ile Ser Thr Ser Leu Gly Asn Thr
           595                 600                 605
   Ser Leu Arg Pro Ser
       610
<210> 15
   <211> 1449
   <212> DNA
   <213> Artificial Sequence
<220>
   <223> Chimeric Protein: BBP-IgE
<220>
   <221> CDS
   <222> (1)..(1449)
<400> 15
   cat atg act tac aag ctg gtg atc aac ggt aaa acc ttg aag ggt gag      48
   His Met Thr Tyr Lys Leu Val Ile Asn Gly Lys Thr Leu Lys Gly Glu
   1               5                   10                  15
   acc acc act gag gca gtc gac gcc gcc act gcc gag aag gtc ttt aaa      96
   Thr Thr Thr Glu Ala Val Asp Ala Ala Thr Ala Glu Lys Val Phe Lys
               20                  25                  30
   cag tat gcc aat gat aac ggc gtg gac ggc gag tgg acc tac gat gac      144
   Gln Tyr Ala Asn Asp Asn Gly Val Asp Gly Glu Trp Thr Tyr Asp Asp
           35                  40                  45
   gcc act aag aca ttc act gtg act gaa aag ccc gag gtg att gac gcg       192
   Ala Thr Lys Thr Phe Thr Val Thr Glu Lys Pro Glu Val Ile Asp Ala
       50                  55                  60
   tcc gaa ttg aca cct gcg gtg acc acc tac aaa ctg gtt atc aac ggc       240
   Ser Glu Leu Thr Pro Ala Val Thr Thr Tyr Lys Leu Val Ile Asn Gly
   65                  70                  75                  80
   aag act ctg aag ggc gag acc acc aca gag gca gtc gat gcc gcc acc       288
   Lys Thr Leu Lys Gly Glu Thr Thr Thr Glu Ala Val Asp Ala Ala Thr
                   85                  90                  95
   gcc gag aag gtc ttc aag caa tat gcc aac gac aac ggg gtg gac ggg       336
   Ala Glu Lys Val Phe Lys Gln Tyr Ala Asn Asp Asn Gly Val Asp Gly
               100                 105                 110
   gag tgg acc tac gat gat gcc acc aag acc ttc acc gtg acc gag aag       384
   Glu Trp Thr Tyr Asp Asp Ala Thr Lys Thr Phe Thr Val Thr Glu Lys
           115                 120                 125
   ccc gaa gtg atc gat gcg agt gaa ctg act ccc gcc gtg aca ggt tct       432
   Pro Glu Val Ile Asp Ala Ser Glu Leu Thr Pro Ala Val Thr Gly Ser
       130                 135                 140
   gct tct ggt tct ggt gtt cga cca gtt aac atc acc gag ccc aca ctg       480
   Ala Ser Gly Ser Gly Val Arg Pro Val Asn Ile Thr Glu Pro Thr Leu
   145                 150                 155                 160
   gaa ctg ctg cac agc agc tgc gac ccc aac gcc ttc cac agc acc atc       528
   Glu Leu Leu His Ser Ser Cys Asp Pro Asn Ala Phe His Ser Thr Ile
                   165                 170                 175
   cag ctg tat tgc ttc atc tac ggc cac atc ctg aac gac gtg tcc gtg       576
   Gln Leu Tyr Cys Phe Ile Tyr Gly His Ile Leu Asn Asp Val Ser Val
               180                 185                 190
   tcc tgg ctg atg gac gac aga gag atc acc gac acc ctg gcc cag acc       624
   Ser Trp Leu Met Asp Asp Arg Glu Ile Thr Asp Thr Leu Ala Gln Thr
           195                 200                 205
   gtg ctg atc aaa gag gaa ggc aag ctg gcc tct acc tgc agc aag ctg       672
   Val Leu Ile Lys Glu Glu Gly Lys Leu Ala Ser Thr Cys Ser Lys Leu
       210                 215                 220
   aat atc aca gag cag cag tgg atg agc gag agc acc ttc acc tgt aaa       720
   Asn Ile Thr Glu Gln Gln Trp Met Ser Glu Ser Thr Phe Thr Cys Lys
   225                 230                 235                 240
   gtg acc tcc cag ggc gtg gac tac ctg gcc cac acc aga aga tgc ccc       768
   Val Thr Ser Gln Gly Val Asp Tyr Leu Ala His Thr Arg Arg Cys Pro
                   245                 250                 255
   gac cac gaa ccc aga ggc gtg atc acc tac ctg atc ccc cct agc ccc       816
   Asp His Glu Pro Arg Gly Val Ile Thr Tyr Leu Ile Pro Pro Ser Pro
               260                 265                 270
   ctg gac ctg tac cag aac ggc gct cct aag ctg acc tgc ctg gtg gtg       864
   Leu Asp Leu Tyr Gln Asn Gly Ala Pro Lys Leu Thr Cys Leu Val Val
           275                 280                 285
   gac ctg gaa agc gag aag aac gtg aac gtg aca tgg aac cag gaa aag       912
   Asp Leu Glu Ser Glu Lys Asn Val Asn Val Thr Trp Asn Gln Glu Lys
       290                 295                 300
   aaa acc agc gtg tcc gcc agc cag tgg tac acc aag cac cac aac aac        960
   Lys Thr Ser Val Ser Ala Ser Gln Trp Tyr Thr Lys His His Asn Asn
   305                 310                 315                 320
   gcc acc acc tcc atc acc agc atc ctg ccc gtg gtg gcc aag gac tgg       1008
   Ala Thr Thr Ser Ile Thr Ser Ile Leu Pro Val Val Ala Lys Asp Trp
                   325                 330                 335
   atc gag ggc tac ggc tac cag tgc atc gtg gac cac ccc gac ttc cct       1056
   Ile Glu Gly Tyr Gly Tyr Gln Cys Ile Val Asp His Pro Asp Phe Pro
               340                 345                 350
   aag ccc atc gtg cgg agc atc acc aag acc cct ggc cag aga tct gcc       1104
   Lys Pro Ile Val Arg Ser Ile Thr Lys Thr Pro Gly Gln Arg Ser Ala
           355                 360                 365
   ccc gag gtg tac gtg ttc ccc cca cct gag gaa gag tcc gag gac aag       1152
   Pro Glu Val Tyr Val Phe Pro Pro Pro Glu Glu Glu Ser Glu Asp Lys
       370                 375                 380
   aga acc ctg acc tgt ctg atc cag aac ttc ttc cca gag gac atc agc       1200
   Arg Thr Leu Thr Cys Leu Ile Gln Asn Phe Phe Pro Glu Asp Ile Ser
   385                 390                 395                 400
   gtg cag tgg ctg ggc gac ggc aag ctg atc tcc aac agc cag cac agc       1248
   Val Gln Trp Leu Gly Asp Gly Lys Leu Ile Ser Asn Ser Gln His Ser
                   405                 410                 415
   aca acc acc cct ctg aag tcc aac ggc agc aac cag ggc ttc ttc atc       1296
   Thr Thr Thr Pro Leu Lys Ser Asn Gly Ser Asn Gln Gly Phe Phe Ile
               420                 425                 430
   ttc agc aga ctg gaa gtg gcc aag acc ctg tgg acc cag aga aag cag       1344
   Phe Ser Arg Leu Glu Val Ala Lys Thr Leu Trp Thr Gln Arg Lys Gln
           435                 440                 445
   ttt aca tgc caa gtg atc cat gag gcc ctg cag aag ccc aga aag ctg       1392
   Phe Thr Cys Gln Val Ile His Glu Ala Leu Gln Lys Pro Arg Lys Leu
       450                 455                 460
   gaa aag acc atc agc acc agc ctg ggc aac acc tcc ctg cgc cct agt       1440
   Glu Lys Thr Ile Ser Thr Ser Leu Gly Asn Thr Ser Leu Arg Pro Ser
   465                 470                 475                 480
   tag ctc gag                                                           1449
       Leu Glu
<210> 16
   <211> 480
   <212> PRT
   <213> Artificial Sequence
<220>
   <223> Synthetic Construct
<400> 16
   His Met Thr Tyr Lys Leu Val Ile Asn Gly Lys Thr Leu Lys Gly Glu
   1               5                   10                  15
   Thr Thr Thr Glu Ala Val Asp Ala Ala Thr Ala Glu Lys Val Phe Lys
   20 25 30
   Gln Tyr Ala Asn Asp Asn Gly Val Asp Gly Glu Trp Thr Tyr Asp Asp
   35 40 45
   Ala Thr Lys Thr Phe Thr Val Thr Glu Lys Pro Glu Val Ile Asp Ala
   50 55 60
   Ser Glu Leu Thr Pro Ala Val Thr Thr Tyr Lys Leu Val Ile Asn Gly
   65 70 75 80
   Lys Thr Leu Lys Gly Glu Thr Thr Thr Glu Ala Val Asp Ala Ala Thr
   85 90 95
   Ala Glu Lys Val Phe Lys Gln Tyr Ala Asn Asp Asn Gly Val Asp Gly
   100 105 110
   Glu Trp Thr Tyr Asp Asp Ala Thr Lys Thr Phe Thr Val Thr Glu Lys
   115 120 125
   Pro Glu Val Ile Asp Ala Ser Glu Leu Thr Pro Ala Val Thr Gly Ser
   130 135 140
   Ala Ser Gly Ser Gly Val Arg Pro Val Asn Ile Thr Glu Pro Thr Leu
   145 150 155 160
   Glu Leu Leu His Ser Ser Cys Asp Pro Asn Ala Phe His Ser Thr Ile
   165 170 175
   Gln Leu Tyr Cys Phe Ile Tyr Gly His Ile Leu Asn Asp Val Ser Val
   180 185 190
   Ser Trp Leu Met Asp Asp Arg Glu Ile Thr Asp Thr Leu Ala Gln Thr
   195 200 205
   Val Leu Ile Lys Glu Glu Gly Lys Leu Ala Ser Thr Cys Ser Lys Leu
   210 215 220
   Asn Ile Thr Glu Gln Gln Trp Met Ser Glu Ser Thr Phe Thr Cys Lys
   225 230 235 240
   Val Thr Ser Gln Gly Val Asp Tyr Leu Ala His Thr Arg Arg Cys Pro
   245 250 255
   Asp His Glu Pro Arg Gly Val Ile Thr Tyr Leu Ile Pro Pro Ser Pro
               260                 265                 270
   Leu Asp Leu Tyr Gln Asn Gly Ala Pro Lys Leu Thr Cys Leu Val Val
           275                 280                 285
   Asp Leu Glu Ser Glu Lys Asn Val Asn Val Thr Trp Asn Gln Glu Lys
       290                 295                 300
   Lys Thr Ser Val Ser Ala Ser Gln Trp Tyr Thr Lys His His Asn Asn
   305                 310                 315                 320
   Ala Thr Thr Ser Ile Thr Ser Ile Leu Pro Val Val Ala Lys Asp Trp
                   325                 330                 335
   Ile Glu Gly Tyr Gly Tyr Gln Cys Ile Val Asp His Pro Asp Phe Pro
               340                 345                 350
   Lys Pro Ile Val Arg Ser Ile Thr Lys Thr Pro Gly Gln Arg Ser Ala
           355                 360                 365
   Pro Glu Val Tyr Val Phe Pro Pro Pro Glu Glu Glu Ser Glu Asp Lys
       370                 375                 380
   Arg Thr Leu Thr Cys Leu Ile Gln Asn Phe Phe Pro Glu Asp Ile Ser
   385                 390                 395                 400
   Val Gln Trp Leu Gly Asp Gly Lys Leu Ile Ser Asn Ser Gln His Ser
                   405                 410                 415
   Thr Thr Thr Pro Leu Lys Ser Asn Gly Ser Asn Gln Gly Phe Phe Ile
               420                 425                 430
   Phe Ser Arg Leu Glu Val Ala Lys Thr Leu Trp Thr Gln Arg Lys Gln
           435                 440                 445
   Phe Thr Cys Gln Val Ile His Glu Ala Leu Gln Lys Pro Arg Lys Leu
       450                 455                 460
   Glu Lys Thr Ile Ser Thr Ser Leu Gly Asn Thr Ser Leu Arg Pro Ser
   465                 470                 475                 480
<210> 17
   <211> 864
   <212> DNA
   <213> Artificial Sequence
<220>
   <223> Chimeric Protein: mSa-CD3Z
<220>
   <221> CDS
   <222> (1)..(864)
<400> 17
   atg aag tgg aaa gcc ctt ttt acc gcc gcc att ctg caa gcg caa ttg          48
   Met Lys Trp Lys Ala Leu Phe Thr Ala Ala Ile Leu Gln Ala Gln Leu
   1               5                   10                  15
   ccg atc act gaa gcc gca tcg gcc gag gcc ggt atc act gga acc tgg          96
   Pro Ile Thr Glu Ala Ala Ser Ala Glu Ala Gly Ile Thr Gly Thr Trp
               20                  25                  30
   tac aac cag cac gga tcc aca ttc acc gtg acc gcc ggt gct gac gga         144
   Tyr Asn Gln His Gly Ser Thr Phe Thr Val Thr Ala Gly Ala Asp Gly
           35                  40                  45
   aat ctg acc gga cag tac gag aat cgg gct cag ggc acc ggt tgt cag         192
   Asn Leu Thr Gly Gln Tyr Glu Asn Arg Ala Gln Gly Thr Gly Cys Gln
       50                  55                  60
   aac tcc cct tac acc ctc act ggg aga tac aac ggc acc aag ctg gaa         240
   Asn Ser Pro Tyr Thr Leu Thr Gly Arg Tyr Asn Gly Thr Lys Leu Glu
   65                  70                  75                  80
   tgg agg gtg gaa tgg aac aac tcc acc gaa aac tgc cat tcc cgc act         288
   Trp Arg Val Glu Trp Asn Asn Ser Thr Glu Asn Cys His Ser Arg Thr
                   85                  90                  95
   gag tgg cgc gga cag tat cag ggg gga gcc gaa gcg cgg atc aac acc         336
   Glu Trp Arg Gly Gln Tyr Gln Gly Gly Ala Glu Ala Arg Ile Asn Thr
               100                 105                 110
   caa tgg aac ctg acc tac gag ggc ggg agc gga ccc gcg act gag cag         384
   Gln Trp Asn Leu Thr Tyr Glu Gly Gly Ser Gly Pro Ala Thr Glu Gln
           115                 120                 125
   ggc cag gat acg ttc act aag gtc aag ggc agc gca tca ggc tcg gga         432
   Gly Gln Asp Thr Phe Thr Lys Val Lys Gly Ser Ala Ser Gly Ser Gly
       130                 135                 140
   cag agc ttt ggc ctg ctg gat ccc aaa ctc tgc tac ctg ctg gat gga         480
   Gln Ser Phe Gly Leu Leu Asp Pro Lys Leu Cys Tyr Leu Leu Asp Gly
   145                 150                 155                 160
   atc ctc ttc atc tat ggt gtc att ctc act gcc ttg ttc ctg aga gtg         528
   Ile Leu Phe Ile Tyr Gly Val Ile Leu Thr Ala Leu Phe Leu Arg Val
                   165                 170                 175
   aag ttc agc agg agc gca gac gcc ccc gcg tac cag cag ggc cag aac         576
   Lys Phe Ser Arg Ser Ala Asp Ala Pro Ala Tyr Gln Gln Gly Gln Asn
               180                 185                 190
   cag ctc tat aac gag ctc aat cta gga cga aga gag gag tac gat gtt         624
   Gln Leu Tyr Asn Glu Leu Asn Leu Gly Arg Arg Glu Glu Tyr Asp Val
           195                 200                 205
   ttg gac aag aga cgt ggc cgg gac cct gag atg ggg gga aag ccg cag         672
   Leu Asp Lys Arg Arg Gly Arg Asp Pro Glu Met Gly Gly Lys Pro Gln
       210                 215                 220
   aga agg aag aac cct cag gaa ggc ctg tac aat gaa ctg cag aaa gat         720
   Arg Arg Lys Asn Pro Gln Glu Gly Leu Tyr Asn Glu Leu Gln Lys Asp
   225                 230                 235                 240
   aag atg gcg gag gcc tac agt gag att ggg atg aaa ggc gag cgc cgg          768
   Lys Met Ala Glu Ala Tyr Ser Glu Ile Gly Met Lys Gly Glu Arg Arg
                   245                 250                 255
   agg ggc aag ggg cac gat ggc ctt tac cag ggt ctc agt aca gcc acc          816
   Arg Gly Lys Gly His Asp Gly Leu Tyr Gln Gly Leu Ser Thr Ala Thr
               260                 265                 270
   aag gac acc tac gac gcc ctt cac atg cag gcc ctg ccc cct cgc taa          864
   Lys Asp Thr Tyr Asp Ala Leu His Met Gln Ala Leu Pro Pro Arg
           275                 280                 285
<210> 18
   <211> 287
   <212> PRT
   <213> Artificial Sequence
<220>
   <223> Synthetic Construct
<400> 18
   Met Lys Trp Lys Ala Leu Phe Thr Ala Ala Ile Leu Gln Ala Gln Leu
   1               5                   10                  15
   Pro Ile Thr Glu Ala Ala Ser Ala Glu Ala Gly Ile Thr Gly Thr Trp
               20                  25                  30
   Tyr Asn Gln His Gly Ser Thr Phe Thr Val Thr Ala Gly Ala Asp Gly
           35                  40                  45
   Asn Leu Thr Gly Gln Tyr Glu Asn Arg Ala Gln Gly Thr Gly Cys Gln
       50                  55                  60
   Asn Ser Pro Tyr Thr Leu Thr Gly Arg Tyr Asn Gly Thr Lys Leu Glu
   65                  70                  75                  80
   Trp Arg Val Glu Trp Asn Asn Ser Thr Glu Asn Cys His Ser Arg Thr
                   85                  90                  95
   Glu Trp Arg Gly Gln Tyr Gln Gly Gly Ala Glu Ala Arg Ile Asn Thr
               100                 105                 110
   Gln Trp Asn Leu Thr Tyr Glu Gly Gly Ser Gly Pro Ala Thr Glu Gln
           115                 120                 125
   Gly Gln Asp Thr Phe Thr Lys Val Lys Gly Ser Ala Ser Gly Ser Gly
       130                 135                 140
   Gln Ser Phe Gly Leu Leu Asp Pro Lys Leu Cys Tyr Leu Leu Asp Gly
   145                 150                 155                 160
   Ile Leu Phe Ile Tyr Gly Val Ile Leu Thr Ala Leu Phe Leu Arg Val
                   165                 170                 175
   Lys Phe Ser Arg Ser Ala Asp Ala Pro Ala Tyr Gln Gln Gly Gln Asn
               180                 185                 190
   Gln Leu Tyr Asn Glu Leu Asn Leu Gly Arg Arg Glu Glu Tyr Asp Val
           195                 200                 205
   Leu Asp Lys Arg Arg Gly Arg Asp Pro Glu Met Gly Gly Lys Pro Gln
       210                 215                 220
   Arg Arg Lys Asn Pro Gln Glu Gly Leu Tyr Asn Glu Leu Gln Lys Asp
   225                 230                 235                 240
   Lys Met Ala Glu Ala Tyr Ser Glu Ile Gly Met Lys Gly Glu Arg Arg
                   245                 250                 255
   Arg Gly Lys Gly His Asp Gly Leu Tyr Gln Gly Leu Ser Thr Ala Thr
               260                 265                 270
   Lys Asp Thr Tyr Asp Ala Leu His Met Gln Ala Leu Pro Pro Arg
           275                 280                 285
<210> 19
   <211> 7
   <212> PRT
   <213> Artificial Sequence
<220>
   <223> Linker
<400> 19
   Gly Ser Ala Ser Gly Ser Gly
   1               5